# EUROPEAN PATENT SPECIFICATION

(11) **EP 2 501 397 B1**
(45) Date of publication and mention of the grant of the patent: **04.10.2017**
(21) Application number: 10832282.7
(22) Date of filing: 19.11.2010
(51) Int. Cl.: A61K 38/16, A61K 39/39, C12N 15/867, C12N 15/86, A61P 31/06, A61K 39/04, A61K 38/00

(54) **METHODS FOR PRODUCING AN IMMUNE RESPONSE TO TUBERCULOSIS**
VERFAHREN ZUM HERVORRUFEN EINER IMMUNANTWORT AUF TUBERKULOSE
PROCÉDÉS DE GÉNÉRATION DE RÉPONSE IMMUNITAIRE À LA TUBERCULOSE

(30) Priority: 20.11.2009 US 263206 P
(43) Date of publication of application: 26.09.2012
(60) Divisional application: 17183578.8
(73) Proprietor: Oregon Health & Science University, Portland, OR 97201 (US); The United States Government as Represented by the Department of Veterans Affairs, Washington, DC 20420 (US)
(72) Inventor: LEWINSOHN, David, M., Portland, OR 97201 (US); LEWINSOHN, Deborah, A., Portland, OR 97201 (US)
(74) Representative: Gowshall, Jonathan Vallance
(86) International application number: PCT/US2010/057479
(87) International publication number: WO 2011/063263

(56) References cited:
- WO-A2-2008/124647
- US-A1- 2002 131 975
- US-A1- 2003 199 012
- US-A1- 2008 138 356
- US-A1- 2009 124 549
- DATABASE UniProt [Online] 10 July 2007 (2007-07-10), "SubName: Full=PPE family protein;", XP002717415, retrieved from EBI accession no. UNIPROT:A5U183 Database accession no. A5U183
- BROSCH ROLAND ET AL: "GENOME PLASTICITY OF BCG AND IMPACT ON VACCINE EFFICACY", PROCEEDINGS OF THE NATIONAL ACADEMY OF SCIENCES - PNAS, NATIONAL ACADEMY OF SCIENCES, US, vol. 104, no. 13, 27 March 2007 (2007-03-27), pages 5596-5601, XP009085785, ISSN: 0027-8424, DOI: 10.1073/PNAS.0700869104 & DATABASE UniProt [Online] 6 February 2007 (2007-02-06), "SubName: Full=PPE family protein;", retrieved from EBI accession no. UNIPROT:A1KHH8 Database accession no. A1KHH8
- SEKI M ET AL: "Whole genome sequence analysis of Mycobacterium bovis bacillus Calmette-Guerin (BCG) Tokyo 172: A comparative study of BCG vaccine substrains", VACCINE, ELSEVIER LTD, GB, vol. 27, no. 11, 10 March 2009 (2009-03-10), pages 1710-1716, XP025995122, ISSN: 0264-410X, DOI: 10.1016/J.VACCINE.2009.01.034 [retrieved on 2009-02-04] & DATABASE UniProt [Online] 26 May 2009 (2009-05-26), "SubName: Full=PPE family protein;", retrieved from EBI accession no. UNIPROT:C1AM31 Database accession no. C1AM31
- GARNIER T ET AL: "THE COMPLETE GENOME SEQUENCE OF MYCOBACTERIUM BOVIS", PROCEEDINGS OF THE NATIONAL ACADEMY OF SCIENCES - PNAS, NATIONAL ACADEMY OF SCIENCES, US, vol. 100, no. 13, 24 June 2003 (2003-06-24), pages 7877-7882, XP001205229, ISSN: 0027-8424, DOI: 10.1073/PNAS.1130426100
- DATABASE PROTEIN [Online] 24 April 2009 XP003032776 Retrieved from NCBI Database accession no. YP_177935

### SEQUENCE LISTING

<110> Oregon Health & Science University The Government of the United States of America as Represented by the Department of Veterans Affairs Lewinsohn, David M Lewinsohn, Deborah A
<120> METHODS FOR PRODUCING AN IMMUNE RESPONSE TO TUBERCULOSIS
<130> 899-81443-02
<150> US 61/263,206
   <151> 2009-11-20
<160> 36
<170> PatentIn version 3.5
<210> 1
   <211> 211
   <212> PRT
   <213> Mycobacterium tuberculosis
<400> 1
   Val Pro His Pro Trp Asp Thr Gly Asp His Glu Arg Asn Trp Gln Gly
   1               5                   10                  15
   Tyr Phe Ile Pro Ala Met Ser Val Leu Arg Asn Arg Val Gly Ala Arg
               20                  25                  30
   Thr His Ala Glu Leu Arg Asp Ala Glu Asn Asp Leu Val Glu Ala Arg
           35                  40                  45
   Val Ile Glu Leu Arg Glu Asp Pro Asn Leu Leu Gly Asp Arg Thr Asp
       50                  55                  60
   Leu Ala Tyr Leu Arg Ala Ile His Arg Gln Leu Phe Gln Asp Ile Tyr
   65                  70                  75                  80
   Val Trp Ala Gly Asp Leu Arg Thr Val Gly Ile Glu Lys Glu Asp Glu
                   85                  90                  95
   Ser Phe Cys Ala Pro Gly Gly Ile Ser Arg Pro Met Glu His Val Ala
               100                 105                 110
   Ala Glu Ile Tyr Gln Leu Asp Arg Leu Arg Ala Val Gly Glu Gly Asp
           115                 120                 125
   Leu Ala Gly Gln Val Ala Tyr Arg Tyr Asp Tyr Val Asn Tyr Ala His
       130                 135                 140
   Pro Phe Arg Glu Gly Asn Gly Arg Ser Thr Arg Glu Phe Phe Asp Leu
   145                 150                 155                 160
   Leu Leu Ser Glu Arg Gly Ser Gly Leu Asp Trp Gly Lys Thr Asp Leu
                   165                 170                 175
   Glu Glu Leu His Gly Ala Cys His Val Ala Arg Ala Asn Ser Asp Leu
               180                 185                 190
   Thr Gly Leu Val Ala Met Phe Lys Gly Ile Leu Asp Ala Glu Pro Thr
           195                 200                 205
   Tyr Asp Phe
       210
<210> 2
   <211> 380
   <212> PRT
   <213> Mycobacterium tuberculosis
<400> 2
   Met Asp Phe Ala Leu Leu Pro Pro Glu Val Asn Ser Ala Arg Met Tyr
   1               5                   10                  15
   Thr Gly Pro Gly Ala Gly Ser Leu Leu Ala Ala Ala Gly Gly Trp Asp
               20                  25                  30
   Ser Leu Ala Ala Glu Leu Ala Thr Thr Ala Glu Ala Tyr Gly Ser Val
           35                  40                  45
   Leu Ser Gly Leu Ala Ala Leu His Trp Arg Gly Pro Ala Ala Glu Ser
       50                  55                  60
   Met Ala Val Thr Ala Ala Pro Tyr Ile Gly Trp Leu Tyr Thr Thr Ala
   65                  70                  75                  80
   Glu Lys Thr Gln Gln Thr Ala Ile Gln Ala Arg Ala Ala Ala Leu Ala
                   85                  90                  95
   Phe Glu Gln Ala Tyr Ala Met Thr Leu Pro Pro Pro Val Val Ala Ala
               100                 105                 110
   Asn Arg Ile Gln Leu Leu Ala Leu Ile Ala Thr Asn Phe Phe Gly Gln
           115                 120                 125
   Asn Thr Ala Ala Ile Ala Ala Thr Glu Ala Gln Tyr Ala Glu Met Trp
       130                 135                 140
   Ala Gln Asp Ala Ala Ala Met Tyr Gly Tyr Ala Thr Ala Ser Ala Ala
   145                 150                 155                 160
   Ala Ala Leu Leu Thr Pro Phe Ser Pro Pro Arg Gln Thr Thr Asn Pro
   165 170 175
   Ala Gly Leu Thr Ala Gln Ala Ala Ala Val Ser Gln Ala Thr Asp Pro
   180 185 190
   Leu Ser Leu Leu Ile Glu Thr Val Thr Gln Ala Leu Gln Ala Leu Thr
   195 200 205
   Ile Pro Ser Phe Ile Pro Glu Asp Phe Thr Phe Leu Asp Ala Ile Phe
   210 215 220
   Ala Gly Tyr Ala Thr Val Gly Val Thr Gln Asp Val Glu Ser Phe Val
   225 230 235 240
   Ala Gly Thr Ile Gly Ala Glu Ser Asn Leu Gly Leu Leu Asn Val Gly
   245 250 255
   Asp Glu Asn Pro Ala Glu Val Thr Pro Gly Asp Phe Gly Ile Gly Glu
   260 265 270
   Leu Val Ser Ala Thr Ser Pro Gly Gly Gly Val Ser Ala Ser Gly Ala
   275 280 285
   Gly Gly Ala Ala Ser Val Gly Asn Thr Val Leu Ala Ser Val Gly Arg
   290 295 300
   Ala Asn Ser Ile Gly Gln Leu Ser Val Pro Pro Ser Trp Ala Ala Pro
   305 310 315 320
   Ser Thr Arg Pro Val Ser Ala Leu Ser Pro Ala Gly Leu Thr Thr Leu
   325 330 335
   Pro Gly Thr Asp Val Ala Glu His Gly Met Pro Gly Val Pro Gly Val
   340 345 350
   Pro Val Ala Ala Gly Arg Ala Ser Gly Val Leu Pro Arg Tyr Gly Val
   355 360 365
   Arg Leu Thr Val Met Ala His Pro Pro Ala Ala Gly
   370 375 380
<210> 3
   <211> 239
   <212> PRT
   <213> Mycobacterium tuberculosis
<400> 3
   Met Thr Glu Pro Arg Pro Val Phe Ala Val Val Ile Ser Ala Gly Leu
   1               5                   10                  15
   Ser Ala Ile Pro Met Val Gly Gly Pro Leu Gln Thr Val Phe Asp Ala
               20                  25                  30
   Ile Glu Glu Arg Thr Arg His Arg Ala Glu Thr Thr Thr Arg Glu Ile
           35                  40                  45
   Cys Glu Ser Val Gly Gly Ala Asp Thr Val Leu Ser Arg Ile Asp Lys
       50                  55                  60
   Asn Pro Glu Leu Glu Pro Leu Leu Ser Gln Ala Ile Glu Ala Ala Thr
   65                  70                  75                  80
   Arg Thr Ser Met Glu Ala Lys Arg Arg Leu Leu Ala Gln Ala Ala Ala
                   85                  90                  95
   Ala Ala Leu Glu Asp Asp Gln Lys Val Glu Pro Ala Ser Leu Ile Val
               100                 105                 110
   Ala Thr Leu Ser Gln Leu Glu Pro Val His Ile His Ala Leu Val Arg
           115                 120                 125
   Leu Ala Lys Ala Ala Lys Ser Ser Pro Asp Gln Asp Glu Ile Gln Arg
       130                 135                 140
   Arg Glu Val Met Arg Ala Ala Ser Lys Val Glu Pro Val Pro Val Leu
   145                 150                 155                 160
   Ala Ala Leu Ile Gln Thr Gly Val Ala Ile Ala Thr Thr Thr Val Trp
                   165                 170                 175
   His Gly Asn Gly Thr Gly Thr Pro Ala Glu Glu Ser Gly His Ile Leu
               180                 185                 190
   Ile His Asp Val Ser Asp Phe Gly His Arg Leu Leu Ala Tyr Leu Arg
           195                 200                 205
   Ala Ala Asp Ala Gly Ala Glu Leu Leu Ile Leu Pro Ser Gly Gly Ser
       210                 215                 220
   Ala Pro Thr Gly Asp His Pro Thr Pro His Pro Ser Thr Ser Arg
   225                 230                 235
<210> 4
   <211> 479
   <212> PRT
   <213> Mycobacterium tuberculosis
<400> 4
   Met Ala Asp Phe Leu Thr Leu Ser Pro Glu Val Asn Ser Ala Arg Met
   1               5                   10                  15
   Tyr Ala Gly Gly Gly Pro Gly Ser Leu Ser Ala Ala Ala Ala Ala Trp
               20                  25                  30
   Asp Glu Leu Ala Ala Glu Leu Trp Leu Ala Ala Ala Ser Phe Glu Ser
           35                  40                  45
   Val Cys Ser Gly Leu Ala Asp Arg Trp Trp Gln Gly Pro Ser Ser Arg
       50                  55                  60
   Met Met Ala Ala Gln Ala Ala Arg His Thr Gly Trp Leu Ala Ala Ala
   65                  70                  75                  80
   Ala Thr Gln Ala Glu Gly Ala Ala Ser Gln Ala Gln Thr Met Ala Leu
                   85                  90                  95
   Ala Tyr Glu Ala Ala Phe Ala Ala Thr Val His Pro Ala Leu Val Ala
               100                 105                 110
   Ala Asn Arg Ala Leu Val Ala Trp Leu Ala Gly Ser Asn Val Phe Gly
           115                 120                 125
   Gln Asn Thr Pro Ala Ile Ala Ala Ala Glu Ala Ile Tyr Glu Gln Met
       130                 135                 140
   Trp Ala Gln Asp Val Val Ala Met Leu Asn Tyr His Ala Val Ala Ser
   145                 150                 155                 160
   Ala Val Gly Ala Arg Leu Arg Pro Trp Gln Gln Leu Leu His Glu Leu
                   165                 170                 175
   Pro Arg Arg Leu Gly Gly Glu His Ser Asp Ser Thr Asn Thr Glu Leu
               180                 185                 190
   Ala Asn Pro Ser Ser Thr Thr Thr Arg Ile Thr Val Pro Gly Ala Ser
           195                 200                 205
   Pro Val His Ala Ala Thr Leu Leu Pro Phe Ile Gly Arg Leu Leu Ala
       210                 215                 220
   Ala Arg Tyr Ala Glu Leu Asn Thr Ala Ile Gly Thr Asn Trp Phe Pro
   225                 230                 235                 240
   Gly Thr Thr Pro Glu Val Val Ser Tyr Pro Ala Thr Ile Gly Val Leu
                   245                 250                 255
   Ser Gly Ser Leu Gly Ala Val Asp Ala Asn Gln Ser Ile Ala Ile Gly
               260                 265                 270
   Gln Gln Met Leu His Asn Glu Ile Leu Ala Ala Thr Ala Ser Gly Gln
   275 280 285
   Pro Val Thr Val Ala Gly Leu Ser Met Gly Ser Met Val Ile Asp Arg
   290 295 300
   Glu Leu Ala Tyr Leu Ala Ile Asp Pro Asn Ala Pro Pro Ser Ser Ala
   305 310 315 320
   Leu Thr Phe Val Glu Leu Ala Gly Pro Glu Arg Gly Leu Ala Gln Thr
   325 330 335
   Tyr Leu Pro Val Gly Thr Thr Ile Pro Ile Ala Gly Tyr Thr Val Gly
   340 345 350
   Asn Ala Pro Glu Ser Gln Tyr Asn Thr Ser Val Val Tyr Ser Gln Tyr
   355 360 365
   Asp Ile Trp Ala Asp Pro Pro Asp Arg Pro Trp Asn Leu Leu Ala Gly
   370 375 380
   Ala Asn Ala Leu Met Gly Ala Ala Tyr Phe His Asp Leu Thr Ala Tyr
   385 390 395 400
   Ala Ala Pro Gln Gln Gly Ile Glu Ile Ala Ala Val Thr Ser Ser Leu
   405 410 415
   Gly Gly Thr Thr Thr Thr Tyr Met Ile Pro Ser Pro Thr Leu Pro Leu
   420 425 430
   Leu Leu Pro Leu Lys Gln Ile Gly Val Pro Asp Trp Ile Val Gly Gly
   435 440 445
   Leu Asn Asn Val Leu Lys Pro Leu Val Asp Ala Gly Tyr Ser Gln Tyr
   450 455 460
   Ala Pro Thr Ala Gly Pro Tyr Phe Ser His Gly Asn Leu Val Trp
   465 470 475
<210> 5
   <211> 394
   <212> PRT
   <213> Mycobacterium tuberculosis
<400> 5
   Met Thr Leu Asp Val Pro Val Asn Gln Gly His Val Pro Pro Gly Ser
   1 5 10 15
   Val Ala Cys Cys Leu Val Gly Val Thr Ala Val Ala Asp Gly Ile Ala
               20                  25                  30
   Gly His Ser Leu Ser Asn Phe Gly Ala Leu Pro Pro Glu Ile Asn Ser
           35                  40                  45
   Gly Arg Met Tyr Ser Gly Pro Gly Ser Gly Pro Leu Met Ala Ala Ala
       50                  55                  60
   Ala Ala Trp Asp Gly Leu Ala Ala Glu Leu Ser Ser Ala Ala Thr Gly
   65                  70                  75                  80
   Tyr Gly Ala Ala Ile Ser Glu Leu Thr Asn Met Arg Trp Trp Ser Gly
                   85                  90                  95
   Pro Ala Ser Asp Ser Met Val Ala Ala Val Leu Pro Phe Val Gly Trp
               100                 105                 110
   Leu Ser Thr Thr Ala Thr Leu Ala Glu Gln Ala Ala Met Gln Ala Arg
           115                 120                 125
   Ala Ala Ala Ala Ala Phe Glu Ala Ala Phe Ala Met Thr Val Pro Pro
       130                 135                 140
   Pro Ala Ile Ala Ala Asn Arg Thr Leu Leu Met Thr Leu Val Asp Thr
   145                 150                 155                 160
   Asn Trp Phe Gly Gln Asn Thr Pro Ala Ile Ala Thr Thr Glu Ser Gln
                   165                 170                 175
   Tyr Ala Glu Met Trp Ala Gln Asp Ala Ala Ala Met Tyr Gly Tyr Ala
               180                 185                 190
   Ser Ala Ala Ala Pro Ala Thr Val Leu Thr Pro Phe Ala Pro Pro Pro
           195                 200                 205
   Gln Thr Thr Asn Ala Thr Gly Leu Val Gly His Ala Thr Ala Val Ala
       210                 215                 220
   Ala Leu Arg Gly Gln His Ser Trp Ala Ala Ala Ile Pro Trp Ser Asp
   225                 230                 235                 240
   Ile Gln Lys Tyr Trp Met Met Phe Leu Gly Ala Leu Ala Thr Ala Glu
                   245                 250                 255
   Gly Phe Ile Tyr Asp Ser Gly Gly Leu Thr Leu Asn Ala Leu Gln Phe
               260                 265                 270
   Val Gly Gly Met Leu Trp Ser Thr Ala Leu Ala Glu Ala Gly Ala Ala
           275                 280                 285
   Glu Ala Ala Ala Gly Ala Gly Gly Ala Ala Gly Trp Ser Ala Trp Ser
       290                 295                 300
   Gln Leu Gly Ala Gly Pro Val Ala Ala Ser Ala Thr Leu Ala Ala Lys
   305                 310                 315                 320
   Ile Gly Pro Met Ser Val Pro Pro Gly Trp Ser Ala Pro Pro Ala Thr
                   325                 330                 335
   Pro Gln Ala Gln Thr Val Ala Arg Ser Ile Pro Gly Ile Arg Ser Ala
               340                 345                 350
   Ala Glu Ala Ala Glu Thr Ser Val Leu Leu Arg Gly Ala Pro Thr Pro
           355                 360                 365
   Gly Arg Ser Arg Ala Ala His Met Gly Arg Arg Tyr Gly Arg Arg Leu
       370                 375                 380
   Thr Val Met Ala Asp Arg Pro Asn Val Gly
   385                 390
<210> 6
   <211> 391
   <212> PRT
   <213> Mycobacterium tuberculosis
<400> 6
   Met Asp Phe Gly Ala Leu Pro Pro Glu Ile Asn Ser Ala Arg Met Tyr
   1               5                   10                  15
   Ala Gly Ala Gly Ala Gly Pro Met Met Ala Ala Gly Ala Ala Trp Asn
               20                  25                  30
   Gly Leu Ala Ala Glu Leu Gly Thr Thr Ala Ala Ser Tyr Glu Ser Val
           35                  40                  45
   Ile Thr Arg Leu Thr Thr Glu Ser Trp Met Gly Pro Ala Ser Met Ala
       50                  55                  60
   Met Val Ala Ala Ala Gln Pro Tyr Leu Ala Trp Leu Thr Tyr Thr Ala
   65                  70                  75                  80
   Glu Ala Ala Ala His Ala Gly Ser Gln Ala Met Ala Ser Ala Ala Ala
                   85                  90                  95
   Tyr Glu Ala Ala Tyr Ala Met Thr Val Pro Pro Glu Val Val Ala Ala
               100                 105                 110
   Asn Arg Ala Leu Leu Ala Ala Leu Val Ala Thr Asn Val Leu Gly Ile
           115                 120                 125
   Asn Thr Pro Ala Ile Met Ala Thr Glu Ala Leu Tyr Ala Glu Met Trp
       130                 135                 140
   Ala Gln Asp Ala Leu Ala Met Tyr Gly Tyr Ala Ala Ala Ser Gly Ala
   145                 150                 155                 160
   Ala Gly Met Leu Gln Pro Leu Ser Pro Pro Ser Gln Thr Thr Asn Pro
                   165                 170                 175
   Gly Gly Leu Ala Ala Gln Ser Ala Ala Val Gly Ser Ala Ala Ala Thr
               180                 185                 190
   Ala Ala Val Asn Gln Val Ser Val Ala Asp Leu Ile Ser Ser Leu Pro
           195                 200                 205
   Asn Ala Val Ser Gly Leu Ala Ser Pro Val Thr Ser Val Leu Asp Ser
       210                 215                 220
   Thr Gly Leu Ser Gly Ile Ile Ala Asp Ile Asp Ala Leu Leu Ala Thr
   225                 230                 235                 240
   Pro Phe Val Ala Asn Ile Ile Asn Ser Ala Val Asn Thr Ala Ala Trp
                   245                 250                 255
   Tyr Val Asn Ala Ala Ile Pro Thr Ala Ile Phe Leu Ala Asn Ala Leu
               260                 265                 270
   Asn Ser Gly Ala Pro Val Ala Ile Ala Glu Gly Ala Ile Glu Ala Ala
           275                 280                 285
   Glu Gly Ala Ala Ser Ala Ala Ala Ala Gly Leu Ala Asp Ser Val Thr
       290                 295                 300
   Pro Ala Gly Leu Gly Ala Ser Leu Gly Glu Ala Thr Leu Val Gly Arg
   305                 310                 315                 320
   Leu Ser Val Pro Ala Ala Trp Ser Thr Ala Ala Pro Ala Thr Thr Ala
                   325                 330                 335
   Gly Ala Thr Ala Leu Glu Gly Ser Gly Trp Thr Val Ala Ala Glu Glu
               340                 345                 350
   Ala Gly Pro Val Thr Gly Met Met Pro Gly Met Ala Ser Ala Ala Lys
           355                 360                 365
   Gly Thr Gly Ala Tyr Ala Gly Pro Arg Tyr Gly Phe Lys Pro Thr Val
       370                 375                 380
   Met Pro Lys Gln Val Val Val
   385                 390
<210> 7
   <211> 552
   <212> PRT
   <213> Mycobacterium tuberculosis
<400> 7
   Met Ala His Phe Ser Val Leu Pro Pro Glu Ile Asn Ser Leu Arg Met
   1               5                   10                  15
   Tyr Leu Gly Ala Gly Ser Ala Pro Met Leu Gln Ala Ala Ala Ala Trp
               20                  25                  30
   Asp Gly Leu Ala Ala Glu Leu Gly Thr Ala Ala Ser Ser Phe Ser Ser
           35                  40                  45
   Val Thr Thr Gly Leu Thr Gly Gln Ala Trp Gln Gly Pro Ala Ser Ala
       50                  55                  60
   Ala Met Ala Ala Ala Ala Ala Pro Tyr Ala Gly Phe Leu Thr Thr Ala
   65                  70                  75                  80
   Ser Ala Gln Ala Gln Leu Ala Ala Gly Gln Ala Lys Ala Val Ala Ser
                   85                  90                  95
   Val Phe Glu Ala Ala Lys Ala Ala Ile Val Pro Pro Ala Ala Val Ala
               100                 105                 110
   Ala Asn Arg Glu Ala Phe Leu Ala Leu Ile Arg Ser Asn Trp Leu Gly
           115                 120                 125
   Leu Asn Ala Pro Trp Ile Ala Ala Val Glu Ser Leu Tyr Glu Glu Tyr
       130                 135                 140
   Trp Ala Ala Asp Val Ala Ala Met Thr Gly Tyr His Ala Gly Ala Ser
   145                 150                 155                 160
   Gln Ala Ala Ala Gln Leu Pro Leu Pro Ala Gly Leu Gln Gln Phe Leu
                   165                 170                 175
   Asn Thr Leu Pro Asn Leu Gly Ile Gly Asn Gln Gly Asn Ala Asn Leu
               180                 185                 190
   Gly Gly Gly Asn Thr Gly Ser Gly Asn Ile Gly Asn Gly Asn Lys Gly
           195                 200                 205
   Ser Ser Asn Leu Gly Gly Gly Asn Ile Gly Asn Asn Asn Ile Gly Ser
       210                 215                 220
   Gly Asn Arg Gly Ser Asp Asn Phe Gly Ala Gly Asn Val Gly Thr Gly
   225                 230                 235                 240
   Asn Ile Gly Phe Gly Asn Gln Gly Pro Ile Asp Val Asn Leu Leu Ala
                   245                 250                 255
   Thr Pro Gly Gln Asn Asn Val Gly Leu Gly Asn Ile Gly Asn Asn Asn
               260                 265                 270
   Met Gly Phe Gly Asn Thr Gly Asp Ala Asn Thr Gly Gly Gly Asn Thr
           275                 280                 285
   Gly Asn Gly Asn Ile Gly Gly Gly Asn Thr Gly Asn Asn Asn Phe Gly
       290                 295                 300
   Phe Gly Asn Thr Gly Asn Asn Asn Ile Gly Ile Gly Leu Thr Gly Asn
   305                 310                 315                 320
   Asn Gln Met Gly Ile Asn Leu Ala Gly Leu Leu Asn Ser Gly Ser Gly
                   325                 330                 335
   Asn Ile Gly Ile Gly Asn Ser Gly Thr Asn Asn Ile Gly Leu Phe Asn
               340                 345                 350
   Ser Gly Ser Gly Asn Ile Gly Val Phe Asn Thr Gly Ala Asn Thr Leu
           355                 360                 365
   Val Pro Gly Asp Leu Asn Asn Leu Gly Val Gly Asn Ser Gly Asn Ala
       370                 375                 380
   Asn Ile Gly Phe Gly Asn Ala Gly Val Leu Asn Thr Gly Phe Gly Asn
   385                 390                 395                 400
   Ala Ser Ile Leu Asn Thr Gly Leu Gly Asn Ala Gly Glu Leu Asn Thr
                   405                 410                 415
   Gly Phe Gly Asn Ala Gly Phe Val Asn Thr Gly Phe Asp Asn Ser Gly
               420                 425                 430
   Asn Val Asn Thr Gly Asn Gly Asn Ser Gly Asn Ile Asn Thr Gly Ser
           435                 440                 445
   Trp Asn Ala Gly Asn Val Asn Thr Gly Phe Gly Ile Ile Thr Asp Ser
       450                 455                 460
   Gly Leu Thr Asn Ser Gly Phe Gly Asn Thr Gly Thr Asp Val Ser Gly
   465 470 475 480
   Phe Phe Asn Thr Pro Thr Gly Pro Leu Ala Val Asp Val Ser Gly Phe
   485 490 495
   Phe Asn Thr Ala Ser Gly Gly Thr Val Ile Asn Gly Gln Thr Ser Gly
   500 505 510
   Ile Gly Asn Ile Gly Val Pro Gly Thr Leu Phe Gly Ser Val Arg Ser
   515 520 525
   Gly Leu Asn Thr Gly Leu Phe Asn Met Gly Thr Ala Ile Ser Gly Leu
   530 535 540
   Phe Asn Leu Arg Gln Leu Leu Gly
   545 550
<210> 8
   <211> 561
   <212> PRT
   <213> Mycobacterium tuberculosis
<400> 8
   Met Glu Tyr Leu Ile Ala Ala Gln Asp Val Leu Val Ala Ala Ala Ala
   1               5                   10                  15
   Asp Leu Glu Gly Ile Gly Ser Ala Leu Ala Ala Ala Asn Arg Ala Ala
               20                  25                  30
   Glu Ala Pro Thr Thr Gly Leu Leu Ala Ala Gly Ala Asp Glu Val Ser
           35                  40                  45
   Ala Ala Ile Ala Ser Leu Phe Ser Gly Asn Ala Gln Ala Tyr Gln Ala
       50                  55                  60
   Leu Ser Ala Gln Ala Ala Ala Phe His Gln Gln Phe Val Arg Ala Leu
   65                  70                  75                  80
   Ser Ser Ala Ala Gly Ser Tyr Ala Ala Ala Glu Ala Ala Asn Ala Ser
                   85                  90                  95
   Pro Met Gln Ala Val Leu Asp Val Val Asn Gly Pro Thr Gln Leu Leu
               100                 105                 110
   Leu Gly Arg Pro Leu Ile Gly Asp Gly Ala Asn Gly Gly Pro Gly Gln
           115                 120                 125
   Asn Gly Gly Asp Gly Gly Leu Leu Tyr Gly Asn Gly Gly Asn Gly Gly
   130 135 140
   Ser Ser Ser Thr Pro Gly Gln Pro Gly Gly Arg Gly Gly Ala Ala Gly
   145 150 155 160
   Leu Ile Gly Asn Gly Gly Ala Gly Gly Ala Gly Gly Pro Gly Ala Asn
   165 170 175
   Gly Gly Ala Gly Gly Asn Gly Gly Trp Leu Tyr Gly Asn Gly Gly Leu
   180 185 190
   Gly Gly Asn Gly Gly Ala Ala Thr Gln Ile Gly Gly Asn Gly Gly Asn
   195 200 205
   Gly Gly His Gly Gly Asn Ala Gly Leu Trp Gly Asn Gly Gly Ala Gly
   210 215 220
   Gly Ala Gly Ala Ala Gly Ala Ala Gly Ala Asn Gly Gln Asn Pro Val
   225 230 235 240
   Ser His Gln Val Thr His Ala Thr Asp Gly Ala Asp Gly Thr Thr Gly
   245 250 255
   Pro Asp Gly Asn Gly Thr Asp Ala Gly Ser Gly Ser Asn Ala Val Asn
   260 265 270
   Pro Gly Val Gly Gly Gly Ala Gly Gly Ile Gly Gly Asp Gly Thr Asn
   275 280 285
   Leu Gly Gln Thr Asp Val Ser Gly Gly Ala Gly Gly Asp Gly Gly Asp
   290 295 300
   Gly Ala Asn Phe Ala Ser Gly Gly Ala Gly Gly Asn Gly Gly Ala Ala
   305 310 315 320
   Gln Ser Gly Phe Gly Asp Ala Val Gly Gly Asn Gly Gly Ala Gly Gly
   325 330 335
   Asn Gly Gly Ala Gly Gly Gly Gly Gly Leu Gly Gly Ala Gly Gly Ser
   340 345 350
   Ala Asn Val Ala Asn Ala Gly Asn Ser Ile Gly Gly Asn Gly Gly Ala
   355 360 365
   Gly Gly Asn Gly Gly Ile Gly Ala Pro Gly Gly Ala Gly Gly Ala Gly
   370 375 380
   Gly Asn Ala Asn Gln Asp Asn Pro Pro Gly Gly Asn Ser Thr Gly Gly
   385 390 395 400
   Asn Gly Gly Ala Gly Gly Asp Gly Gly Val Gly Ala Ser Ala Asp Val
                   405                 410                 415
   Gly Gly Ala Gly Gly Phe Gly Gly Ser Gly Gly Arg Gly Gly Leu Leu
               420                 425                 430
   Leu Gly Thr Gly Gly Ala Gly Gly Asp Gly Gly Val Gly Gly Asp Gly
           435                 440                 445
   Gly Ile Gly Ala Gln Gly Gly Ser Gly Gly Asn Gly Gly Asn Gly Gly
       450                 455                 460
   Ile Gly Ala Asp Gly Met Ala Asn Gln Asp Gly Asp Gly Gly Asp Gly
   465                 470                 475                 480
   Gly Asn Gly Gly Asp Gly Gly Ala Gly Gly Ala Gly Gly Val Gly Gly
                   485                 490                 495
   Asn Gly Gly Thr Gly Gly Ala Gly Gly Leu Phe Gly Gln Ser Gly Ser
               500                 505                 510
   Pro Gly Ser Gly Ala Ala Gly Gly Leu Gly Gly Ala Gly Gly Asn Gly
           515                 520                 525
   Gly Ala Gly Gly Gly Gly Gly Thr Gly Phe Asn Pro Gly Ala Pro Gly
       530                 535                 540
   Asp Pro Gly Thr Gln Gly Ala Thr Gly Ala Asn Gly Gln His Gly Leu
   545                 550                 555                 560
   Asn
<210> 9
   <211> 1538
   <212> PRT
   <213> Mycobacterium tuberculosis
<400> 9
   Met Val Met Ser Leu Met Val Ala Pro Glu Leu Val Ala Ala Ala Ala
   1               5                   10                  15
   Ala Asp Leu Thr Gly Ile Gly Gln Ala Ile Ser Ala Ala Asn Ala Ala
               20                  25                  30
   Ala Ala Gly Pro Thr Thr Gln Val Leu Ala Ala Ala Gly Asp Glu Val
           35                  40                  45
   Ser Ala Ala Ile Ala Ala Leu Phe Gly Thr His Ala Gln Glu Tyr Gln
       50                  55                  60
   Ala Leu Ser Ala Arg Val Ala Thr Phe His Glu Gln Phe Val Arg Ser
   65                  70                  75                  80
   Leu Thr Ala Ala Gly Ser Ala Tyr Ala Thr Ala Glu Ala Ala Asn Ala
                   85                  90                  95
   Ser Pro Leu Gln Ala Leu Glu Gln Gln Val Leu Gly Ala Ile Asn Ala
               100                 105                 110
   Pro Thr Gln Leu Trp Leu Gly Arg Pro Leu Ile Gly Asp Gly Val His
           115                 120                 125
   Gly Ala Pro Gly Thr Gly Gln Pro Gly Gly Ala Gly Gly Leu Leu Trp
       130                 135                 140
   Gly Asn Gly Gly Asn Gly Gly Ser Gly Ala Ala Gly Gln Val Gly Gly
   145                 150                 155                 160
   Pro Gly Gly Ala Ala Gly Leu Phe Gly Asn Gly Gly Ser Gly Gly Ser
                   165                 170                 175
   Gly Gly Ala Gly Ala Ala Gly Gly Val Gly Gly Ser Gly Gly Trp Leu
               180                 185                 190
   Asn Gly Asn Gly Gly Ala Gly Gly Ala Gly Gly Thr Gly Ala Asn Gly
           195                 200                 205
   Gly Ala Gly Gly Asn Ala Trp Leu Phe Gly Ala Gly Gly Ser Gly Gly
       210                 215                 220
   Ala Gly Thr Asn Gly Gly Val Gly Gly Ser Gly Gly Phe Val Tyr Gly
   225                 230                 235                 240
   Asn Gly Gly Ala Gly Gly Ile Gly Gly Ile Gly Gly Ile Gly Gly Asn
                   245                 250                 255
   Gly Gly Asp Ala Gly Leu Phe Gly Asn Gly Gly Ala Gly Gly Ala Gly
               260                 265                 270
   Ala Ala Gly Leu Pro Gly Ala Ala Gly Leu Asn Gly Gly Asp Gly Ser
           275                 280                 285
   Asp Gly Gly Asn Gly Gly Thr Gly Gly Asn Gly Gly Arg Gly Gly Leu
       290                 295                 300
   Leu Val Gly Asn Gly Gly Ala Gly Gly Ala Gly Gly Val Gly Gly Asp
   305                 310                 315                 320
   Gly Gly Lys Gly Gly Ala Gly Asp Pro Ser Phe Ala Val Asn Asn Gly
                   325                 330                 335
   Ala Gly Gly Asn Gly Gly His Gly Gly Asn Pro Gly Val Gly Gly Ala
               340                 345                 350
   Gly Gly Ala Gly Gly Leu Leu Ala Gly Ala His Gly Ala Ala Gly Ala
           355                 360                 365
   Thr Pro Thr Ser Gly Gly Asn Gly Gly Asp Gly Gly Ile Gly Ala Thr
       370                 375                 380
   Ala Asn Ser Pro Leu Gln Ala Gly Gly Ala Gly Gly Asn Gly Gly His
   385                 390                 395                 400
   Gly Gly Leu Val Gly Asn Gly Gly Thr Gly Gly Ala Gly Gly Ala Gly
                   405                 410                 415
   His Ala Gly Ser Thr Gly Ala Thr Gly Thr Ala Leu Gln Pro Thr Gly
               420                 425                 430
   Gly Asn Gly Thr Asn Gly Gly Ala Gly Gly His Gly Gly Asn Gly Gly
           435                 440                 445
   Asn Gly Gly Ala Gln His Gly Asp Gly Gly Val Gly Gly Lys Gly Gly
       450                 455                 460
   Ala Gly Gly Ser Gly Gly Ala Gly Gly Asn Gly Phe Asp Ala Ala Thr
   465                 470                 475                 480
   Leu Gly Ser Pro Gly Ala Asp Gly Gly Met Gly Gly Asn Gly Gly Lys
                   485                 490                 495
   Gly Gly Asp Gly Gly Lys Ala Gly Asp Gly Gly Ala Gly Ala Ala Gly
               500                 505                 510
   Asp Val Thr Leu Ala Val Asn Gln Gly Ala Gly Gly Asp Gly Gly Asn
           515                 520                 525
   Gly Gly Glu Val Gly Val Gly Gly Lys Gly Gly Ala Gly Gly Val Ser
       530                 535                 540
   Ala Asn Pro Ala Leu Asn Gly Ser Ala Gly Ala Asn Gly Thr Ala Pro
   545                 550                 555                 560
   Thr Ser Gly Gly Asn Gly Gly Asn Gly Gly Ala Gly Ala Thr Pro Thr
                   565                 570                 575
   Val Ala Gly Glu Asn Gly Gly Ala Gly Gly Asn Gly Gly His Gly Gly
   580 585 590
   Ser Val Gly Asn Gly Gly Ala Gly Gly Ala Gly Gly Asn Gly Val Ala
   595 600 605
   Gly Thr Gly Leu Ala Leu Asn Gly Gly Asn Gly Gly Asn Gly Gly Ile
   610 615 620
   Gly Gly Asn Gly Gly Ser Ala Ala Gly Thr Gly Gly Asp Gly Gly Lys
   625 630 635 640
   Gly Gly Asn Gly Gly Ala Gly Ala Asn Gly Gln Asp Phe Ser Ala Ser
   645 650 655
   Ala Asn Gly Ala Asn Gly Gly Gln Gly Gly Asn Gly Gly Asn Gly Gly
   660 665 670
   Ile Gly Gly Lys Gly Gly Asp Ala Phe Ala Thr Phe Ala Lys Ala Gly
   675 680 685
   Asn Gly Gly Ala Gly Gly Asn Gly Gly Asn Val Gly Val Ala Gly Gln
   690 695 700
   Gly Gly Ala Gly Gly Lys Gly Ala Ile Pro Ala Met Lys Gly Ala Thr
   705 710 715 720
   Gly Ala Asp Gly Thr Ala Pro Thr Ser Gly Gly Asp Gly Gly Asn Gly
   725 730 735
   Gly Asn Gly Ala Ser Pro Thr Val Ala Gly Gly Asn Gly Gly Asp Gly
   740 745 750
   Gly Lys Gly Gly Ser Gly Gly Asn Val Gly Asn Gly Gly Asn Gly Gly
   755 760 765
   Ala Gly Gly Asn Gly Ala Ala Gly Gln Ala Gly Thr Pro Gly Pro Thr
   770 775 780
   Ser Gly Asp Ser Gly Thr Ser Gly Thr Asp Gly Gly Ala Gly Gly Asn
   785 790 795 800
   Gly Gly Ala Gly Gly Ala Gly Gly Thr Leu Ala Gly His Gly Gly Asn
   805 810 815
   Gly Gly Lys Gly Gly Asn Gly Gly Gln Gly Gly Ile Gly Gly Ala Gly
   820 825 830
   Glu Arg Gly Ala Asp Gly Ala Gly Pro Asn Ala Asn Gly Ala Asn Gly
           835                 840                 845
   Glu Asn Gly Gly Ser Gly Gly Asn Gly Gly Asp Gly Gly Ala Gly Gly
       850                 855                 860
   Asn Gly Gly Ala Gly Gly Lys Ala Gln Ala Ala Gly Tyr Thr Asp Gly
   865                 870                 875                 880
   Ala Thr Gly Thr Gly Gly Asp Gly Gly Asn Gly Gly Asp Gly Gly Lys
                   885                 890                 895
   Ala Gly Asp Gly Gly Ala Gly Glu Asn Gly Leu Asn Ser Gly Ala Met
               900                 905                 910
   Leu Pro Gly Gly Gly Thr Val Gly Asn Pro Gly Thr Gly Gly Asn Gly
           915                 920                 925
   Gly Asn Gly Gly Asn Ala Gly Val Gly Gly Thr Gly Gly Lys Ala Gly
       930                 935                 940
   Thr Gly Ser Leu Thr Gly Leu Asp Gly Thr Asp Gly Ile Thr Pro Asn
   945                 950                 955                 960
   Gly Gly Asn Gly Gly Asn Gly Gly Asn Gly Gly Lys Gly Gly Thr Ala
                   965                 970                 975
   Gly Asn Gly Ser Gly Ala Ala Gly Gly Asn Gly Gly Asn Gly Gly Ser
               980                 985                 990
   Gly Leu Asn Gly Gly Asp Ala Gly  Asn Gly Gly Asn Gly  Gly Gly Ala
           995                 1000                 1005
   Leu Asn  Gln Ala Gly Phe Phe  Gly Thr Gly Gly Lys  Gly Gly Asn
       1010                 1015                 1020
   Gly Gly  Asn Gly Gly Ala Gly  Met Ile Asn Gly Gly  Leu Gly Gly
       1025                 1030                 1035
   Phe Gly  Gly Ala Gly Gly Gly  Gly Ala Val Asp Val  Ala Ala Thr
       1040                 1045                 1050
   Thr Gly  Gly Ala Gly Gly Asn  Gly Gly Ala Gly Gly  Phe Ala Ser
       1055                 1060                 1065
   Thr Gly  Leu Gly Gly Pro Gly  Gly Ala Gly Gly Pro  Gly Gly Ala
       1070                 1075                 1080
   Gly Asp Phe Ala Ser Gly Val Gly Gly Val Gly Gly Ala Gly Gly
   1085 1090 1095
   Asp Gly Gly Ala Gly Gly Val Gly Gly Phe Gly Gly Gln Gly Gly
   1100 1105 1110
   Ile Gly Gly Glu Gly Arg Thr Gly Gly Asn Gly Gly Ser Gly Gly
   1115 1120 1125
   Asp Gly Gly Gly Gly Ile Ser Leu Gly Gly Asn Gly Gly Leu Gly
   1130 1135 1140
   Gly Asn Gly Gly Val Ser Glu Thr Gly Phe Gly Gly Ala Gly Gly
   1145 1150 1155
   Asn Gly Gly Tyr Gly Gly Pro Gly Gly Pro Glu Gly Asn Gly Gly
   1160 1165 1170
   Leu Gly Gly Asn Gly Gly Ala Gly Gly Asn Gly Gly Val Ser Thr
   1175 1180 1185
   Thr Gly Gly Asp Gly Gly Ala Gly Gly Lys Gly Gly Asn Gly Gly
   1190 1195 1200
   Asp Gly Gly Asn Val Gly Leu Gly Gly Asp Ala Gly Ser Gly Gly
   1205 1210 1215
   Ala Gly Gly Asn Gly Gly Ile Gly Thr Asp Ala Gly Gly Ala Gly
   1220 1225 1230
   Gly Ala Gly Gly Ala Gly Gly Asn Gly Gly Ser Ser Lys Ser Thr
   1235 1240 1245
   Thr Thr Gly Asn Ala Gly Ser Gly Gly Ala Gly Gly Asn Gly Gly
   1250 1255 1260
   Thr Gly Leu Asn Gly Ala Gly Gly Ala Gly Gly Ala Gly Gly Asn
   1265 1270 1275
   Ala Gly Val Ala Gly Val Ser Phe Gly Asn Ala Val Gly Gly Asp
   1280 1285 1290
   Gly Gly Asn Gly Gly Asn Gly Gly His Gly Gly Asp Gly Thr Thr
   1295 1300 1305
   Gly Gly Ala Gly Gly Lys Gly Gly Asn Gly Ser Ser Gly Ala Ala
   1310 1315 1320
   Ser Gly Ser Gly Val Val Asn Val Thr Ala Gly His Gly Gly Asn
   1325 1330 1335
   Gly Gly Asn Gly Gly Asn Gly Gly Asn Gly Ser Ala Gly Ala Gly
   1340 1345 1350
   Gly Gln Gly Gly Ala Gly Gly Ser Ala Gly Asn Gly Gly His Gly
   1355 1360 1365
   Gly Gly Ala Thr Gly Gly Asp Gly Gly Asn Gly Gly Asn Gly Gly
   1370 1375 1380
   Asn Ser Gly Asn Ser Thr Gly Val Ala Gly Leu Ala Gly Gly Ala
   1385 1390 1395
   Ala Gly Ala Gly Gly Asn Gly Gly Gly Thr Ser Ser Ala Ala Gly
   1400 1405 1410
   His Gly Gly Ser Gly Gly Ser Gly Gly Ser Gly Thr Thr Gly Gly
   1415 1420 1425
   Ala Gly Ala Ala Gly Gly Asn Gly Gly Ala Gly Ala Gly Gly Gly
   1430 1435 1440
   Ser Leu Ser Thr Gly Gln Ser Gly Gly Pro Arg Arg Gln Arg Trp
   1445 1450 1455
   Cys Arg Trp Gln Arg Arg Arg Trp Leu Gly Arg Gln Arg Arg Arg
   1460 1465 1470
   Arg Trp Cys Arg Trp Gln Arg Arg Cys Arg Arg Gln Arg Trp Arg
   1475 1480 1485
   Trp Arg Cys Arg Gln Arg Arg Leu Arg Arg Gln Trp Arg Gln Gly
   1490 1495 1500
   Arg Arg Arg Cys Arg Pro Trp Leu His Arg Arg Arg Gly Arg Gln
   1505 1510 1515
   Gly Arg Arg Trp Arg Gln Arg Arg Phe Gln Gln Arg Gln Arg Ser
   1520 1525 1530
   Arg Trp Gln Arg Arg
   1535
<210> 10
   <211> 423
   <212> PRT
   <213> Mycobacterium tuberculosis
<400> 10
   Val Ile Gln Thr Cys Glu Val Glu Leu Arg Trp Arg Ala Ser Gln Leu
   1               5                   10                  15
   Thr Leu Ala Ile Ala Thr Cys Ala Gly Val Ala Leu Ala Ala Ala Val
               20                  25                  30
   Val Ala Gly Arg Trp Gln Leu Ile Ala Phe Ala Ala Pro Leu Leu Gly
           35                  40                  45
   Val Leu Cys Ser Ile Ser Trp Gln Arg Pro Val Pro Val Ile Gln Val
       50                  55                  60
   His Gly Asp Pro Asp Ser Gln Arg Cys Phe Glu Asn Glu His Val Arg
   65                  70                  75                  80
   Val Thr Val Trp Val Thr Thr Glu Ser Val Asp Ala Ala Val Glu Leu
                   85                  90                  95
   Thr Val Ser Ala Leu Ala Gly Met Gln Phe Glu Ala Leu Glu Ser Val
               100                 105                 110
   Ser Arg Arg Thr Thr Thr Val Ser Ala Val Ala Gln Arg Trp Gly Arg
           115                 120                 125
   Tyr Pro Ile Arg Ala Arg Val Ala Val Val Ala Arg Gly Gly Leu Leu
       130                 135                 140
   Met Gly Ala Gly Thr Val Asp Ala Ala Glu Ile Val Val Phe Pro Leu
   145                 150                 155                 160
   Thr Pro Pro Gln Ser Thr Pro Leu Pro Gln Thr Glu Leu Leu Asp Arg
                   165                 170                 175
   Leu Gly Ala His Leu Thr Arg His Val Gly Pro Gly Val Glu Tyr Ala
               180                 185                 190
   Asp Ile Arg Pro Tyr Val Pro Gly Asp Gln Leu Arg Ala Val Asn Trp
           195                 200                 205
   Val Val Ser Ala Arg Arg Gly Arg Leu His Val Thr Arg Arg Leu Thr
       210                 215                 220
   Asp Arg Ala Ala Asp Val Val Val Leu Ile Asp Met Tyr Arg Gln Pro
   225                 230                 235                 240
   Ala Gly Pro Ala Thr Glu Ala Thr Glu Arg Val Val Arg Gly Ala Ala
                   245                 250                 255
   Gln Val Val Gln Thr Ala Leu Arg Asn Gly Asp Arg Ala Gly Ile Val
   260 265 270
   Ala Leu Gly Gly Asn Arg Pro Arg Trp Leu Gly Ala Asp Ile Gly Gln
   275 280 285
   Arg Gln Phe Tyr Arg Val Leu Asp Thr Val Leu Gly Ala Gly Glu Gly
   290 295 300
   Phe Glu Asn Thr Thr Gly Thr Leu Ala Pro Arg Ala Ala Val Pro Ala
   305 310 315 320
   Gly Ala Val Val Ile Ala Phe Ser Thr Leu Leu Asp Thr Glu Phe Ala
   325 330 335
   Leu Ala Leu Ile Asp Leu Arg Lys Arg Gly His Val Val Val Ala Val
   340 345 350
   Asp Val Leu Asp Ser Cys Pro Leu Gln Asp Gln Leu Asp Pro Leu Val
   355 360 365
   Val Arg Met Trp Ala Leu Gln Arg Ser Ala Met Tyr Arg Asp Met Ala
   370 375 380
   Thr Ile Gly Val Asp Val Leu Ser Trp Pro Ala Asp His Ser Leu Gln
   385 390 395 400
   Gln Ser Met Gly Ala Leu Pro Asn Arg Arg Arg Arg Gly Arg Gly Arg
   405 410 415
   Ala Ser Arg Ala Arg Leu Pro
   420
<210> 11
   <211> 340
   <212> PRT
   <213> Mycobacterium tuberculosis
<400> 11
   Val Asn Arg Arg Ile Leu Thr Leu Met Val Ala Leu Val Pro Ile Val
   1               5                   10                  15
   Val Phe Gly Val Leu Leu Ala Val Val Thr Val Pro Phe Val Ala Leu
               20                  25                  30
   Gly Pro Gly Pro Thr Phe Asp Thr Leu Gly Glu Ile Asp Gly Lys Gln
           35                  40                  45
   Val Val Gln Ile Val Gly Thr Gln Thr Tyr Pro Thr Ser Gly His Leu
       50                  55                  60
   Asn Met Thr Thr Val Ser Gln Arg Asp Gly Leu Thr Leu Gly Glu Ala
   65                  70                  75                  80
   Leu Ala Leu Trp Leu Ser Gly Gln Glu Gln Leu Met Pro Arg Asp Leu
                   85                  90                  95
   Val Tyr Pro Pro Gly Lys Ser Arg Glu Glu Ile Glu Asn Asp Asn Ala
               100                 105                 110
   Ala Asp Phe Lys Arg Ser Glu Ala Ala Ala Glu Tyr Ala Ala Leu Gly
           115                 120                 125
   Tyr Leu Lys Tyr Pro Lys Ala Val Thr Val Ala Ser Val Met Asp Pro
       130                 135                 140
   Gly Pro Ser Val Asp Lys Leu Gln Ala Gly Asp Ala Ile Asp Ala Val
   145                 150                 155                 160
   Asp Gly Thr Pro Val Gly Asn Leu Asp Gln Phe Thr Ala Leu Leu Lys
                   165                 170                 175
   Asn Thr Lys Pro Gly Gln Glu Val Thr Ile Asp Phe Arg Arg Lys Asn
               180                 185                 190
   Glu Pro Pro Gly Ile Ala Gln Ile Thr Leu Gly Lys Asn Lys Asp Arg
           195                 200                 205
   Asp Gln Gly Val Leu Gly Ile Glu Val Val Asp Ala Pro Trp Ala Pro
       210                 215                 220
   Phe Ala Val Asp Phe His Leu Ala Asn Val Gly Gly Pro Ser Ala Gly
   225                 230                 235                 240
   Leu Met Phe Ser Leu Ala Val Val Asp Lys Leu Thr Ser Gly His Leu
                   245                 250                 255
   Val Gly Ser Thr Phe Val Ala Gly Thr Gly Thr Ile Ala Val Asp Gly
               260                 265                 270
   Lys Val Gly Gln Ile Gly Gly Ile Thr His Lys Met Ala Ala Ala Arg
           275                 280                 285
   Ala Ala Gly Ala Thr Val Phe Leu Val Pro Ala Lys Asn Cys Tyr Glu
       290                 295                 300
   Ala Ser Ser Asp Ser Pro Pro Gly Leu Lys Leu Val Lys Val Glu Thr
   305                 310                 315                 320
   Leu Ser Gln Ala Val Asp Ala Leu His Ala Met Thr Ser Gly Ser Pro
                   325                 330                 335
   Thr Pro Ser Cys
               340
<210> 12
   <211> 923
   <212> PRT
   <213> Mycobacterium tuberculosis
<400> 12
   Met Ser Phe Val Val Thr Ala Pro Pro Val Leu Ala Ser Ala Ala Ser
   1 5 10 15
   Asp Leu Gly Gly Ile Ala Ser Met Ile Ser Glu Ala Asn Ala Met Ala
   20 25 30
   Ala Val Arg Thr Thr Ala Leu Ala Pro Ala Ala Ala Asp Glu Val Ser
   35 40 45
   Ala Ala Ile Ala Ala Leu Phe Ser Ser Tyr Ala Arg Asp Tyr Gln Thr
   50 55 60
   Leu Ser Val Gln Val Thr Ala Phe His Val Gln Phe Ala Gln Thr Leu
   65 70 75 80
   Thr Asn Ala Gly Gln Leu Tyr Ala Val Val Asp Val Gly Asn Gly Val
   85 90 95
   Leu Leu Lys Thr Glu Gln Gln Val Leu Gly Val Ile Asn Ala Pro Thr
   100 105 110
   Gln Thr Leu Val Gly Arg Pro Leu Ile Gly Asp Gly Thr His Gly Ala
   115 120 125
   Pro Gly Thr Gly Gln Asn Gly Gly Ala Gly Gly Ile Leu Trp Gly Asn
   130 135 140
   Gly Gly Asn Gly Gly Ser Gly Ala Pro Gly Gln Pro Gly Gly Arg Gly
   145 150 155 160
   Gly Asp Ala Gly Leu Phe Gly His Gly Gly His Gly Gly Val Gly Gly
   165 170 175
   Pro Gly Ile Ala Gly Ala Ala Gly Thr Ala Gly Leu Pro Gly Gly Asn
   180 185 190
   Gly Ala Asn Gly Gly Ser Gly Gly Ile Gly Gly Ala Gly Gly Ala Gly
           195                 200                 205
   Gly Asn Gly Gly Leu Leu Phe Gly Asn Gly Gly Ala Gly Gly Gln Gly
       210                 215                 220
   Gly Ser Gly Gly Leu Gly Gly Ser Gly Gly Thr Gly Gly Ala Gly Met
   225                 230                 235                 240
   Ala Ala Gly Pro Ala Gly Gly Thr Gly Gly Ile Gly Gly Ile Gly Gly
                   245                 250                 255
   Ile Gly Gly Ala Gly Gly Val Gly Gly His Gly Ser Ala Leu Phe Gly
               260                 265                 270
   His Gly Gly Ile Asn Gly Asp Gly Gly Thr Gly Gly Met Gly Gly Gln
           275                 280                 285
   Gly Gly Ala Gly Gly Asn Gly Trp Ala Ala Glu Gly Ile Thr Val Gly
       290                 295                 300
   Ile Gly Glu Gln Gly Gly Gln Gly Gly Asp Gly Gly Ala Gly Gly Ala
   305                 310                 315                 320
   Gly Gly Ile Gly Gly Ser Ala Gly Gly Ile Gly Gly Ser Gln Gly Ala
                   325                 330                 335
   Gly Gly His Gly Gly Asp Gly Gly Gln Gly Gly Ala Gly Gly Ser Gly
               340                 345                 350
   Gly Val Gly Gly Gly Gly Ala Gly Ala Gly Gly Asp Gly Gly Ala Gly
           355                 360                 365
   Gly Ile Gly Gly Thr Gly Gly Asn Gly Ser Ile Gly Gly Ala Ala Gly
       370                 375                 380
   Asn Gly Gly Asn Gly Gly Arg Gly Gly Ala Gly Gly Met Ala Thr Ala
   385                 390                 395                 400
   Gly Ser Asp Gly Gly Asn Gly Gly Gly Gly Gly Asn Gly Gly Val Gly
                   405                 410                 415
   Val Gly Ser Ala Gly Gly Ala Gly Gly Thr Gly Gly Asp Gly Gly Ala
               420                 425                 430
   Ala Gly Ala Gly Gly Ala Pro Gly His Gly Tyr Phe Gln Gln Pro Ala
           435                 440                 445
   Pro Gln Gly Leu Pro Ile Gly Thr Gly Gly Thr Gly Gly Glu Gly Gly
       450                 455                 460
   Ala Gly Gly Ala Gly Gly Asp Gly Gly Gln Gly Asp Ile Gly Phe Asp
   465 470 475 480
   Gly Gly Arg Gly Gly Asp Gly Gly Pro Gly Gly Gly Gly Gly Ala Gly
   485 490 495
   Gly Asp Gly Ser Gly Thr Phe Asn Ala Gln Ala Asn Asn Gly Gly Asp
   500 505 510
   Gly Gly Ala Gly Gly Val Gly Gly Ala Gly Gly Thr Gly Gly Thr Gly
   515 520 525
   Gly Val Gly Ala Asp Gly Gly Arg Gly Gly Asp Ser Gly Arg Gly Gly
   530 535 540
   Asp Gly Gly Asn Ala Gly His Gly Gly Ala Ala Gln Phe Ser Gly Arg
   545 550 555 560
   Gly Ala Tyr Gly Gly Glu Gly Gly Ser Gly Gly Ala Gly Gly Asn Ala
   565 570 575
   Gly Gly Ala Gly Thr Gly Gly Thr Ala Gly Ser Gly Gly Ala Gly Gly
   580 585 590
   Phe Gly Gly Asn Gly Ala Asp Gly Gly Asn Gly Gly Asn Gly Gly Asn
   595 600 605
   Gly Gly Phe Gly Gly Ile Asn Gly Thr Phe Gly Thr Asn Gly Ala Gly
   610 615 620
   Gly Thr Gly Gly Leu Gly Thr Leu Leu Gly Gly His Asn Gly Asn Ile
   625 630 635 640
   Gly Leu Asn Gly Ala Thr Gly Gly Ile Gly Ser Thr Thr Leu Thr Asn
   645 650 655
   Ala Thr Val Pro Leu Gln Leu Val Asn Thr Thr Glu Pro Val Val Phe
   660 665 670
   Ile Ser Leu Asn Gly Gly Gln Met Val Pro Val Leu Leu Asp Thr Gly
   675 680 685
   Ser Thr Gly Leu Val Met Asp Ser Gln Phe Leu Thr Gln Asn Phe Gly
   690 695 700
   Pro Val Ile Gly Thr Gly Thr Ala Gly Tyr Ala Gly Gly Leu Thr Tyr
   705 710 715 720
   Asn Tyr Asn Thr Tyr Ser Thr Thr Val Asp Phe Gly Asn Gly Leu Leu
   725 730 735
   Thr Leu Pro Thr Ser Val Asn Val Val Thr Ser Ser Ser Pro Gly Thr
   740 745 750
   Leu Gly Asn Phe Leu Ser Arg Ser Gly Ala Val Gly Val Leu Gly Ile
   755 760 765
   Gly Pro Asn Asn Gly Phe Pro Gly Thr Ser Ser Ile Val Thr Ala Met
   770 775 780
   Pro Gly Leu Leu Asn Asn Gly Val Leu Ile Asp Glu Ser Ala Gly Ile
   785 790 795 800
   Leu Gln Phe Gly Pro Asn Thr Leu Thr Gly Gly Ile Thr Ile Ser Gly
   805 810 815
   Ala Pro Ile Ser Thr Val Ala Val Gln Ile Asp Asn Gly Pro Leu Gln
   820 825 830
   Gln Ala Pro Val Met Phe Asp Ser Gly Gly Ile Asn Gly Thr Ile Pro
   835 840 845
   Ser Ala Leu Ala Ser Leu Pro Ser Gly Gly Phe Val Pro Ala Gly Thr
   850 855 860
   Thr Ile Ser Val Tyr Thr Ser Asp Gly Gln Thr Leu Leu Tyr Ser Tyr
   865 870 875 880
   Thr Thr Thr Ala Thr Asn Thr Pro Phe Val Thr Ser Gly Gly Val Met
   885 890 895
   Asn Thr Gly His Val Pro Phe Ala Gln Gln Pro Ile Tyr Val Ser Tyr
   900 905 910
   Ser Pro Thr Ala Ile Gly Thr Thr Thr Phe Asn
   915 920
<210> 13
   <211> 332
   <212> PRT
   <213> Mycobacterium tuberculosis
<400> 13
   Met Thr His Asp His Ala His Ser Arg Gly Val Pro Ala Met Ile Lys
   1               5                   10                  15
   Glu Ile Phe Ala Pro His Ser His Asp Ala Ala Asp Ser Val Asp Asp
               20                  25                  30
   Thr Leu Glu Ser Thr Ala Ala Gly Ile Arg Thr Val Lys Ile Ser Leu
           35                  40                  45
   Leu Val Leu Gly Leu Thr Ala Leu Ile Gln Ile Val Ile Val Val Met
       50                  55                  60
   Ser Gly Ser Val Ala Leu Ala Ala Asp Thr Ile His Asn Phe Ala Asp
   65                  70                  75                  80
   Ala Leu Thr Ala Val Pro Leu Trp Ile Ala Phe Ala Leu Gly Ala Lys
                   85                  90                  95
   Pro Ala Thr Arg Arg Tyr Thr Tyr Gly Phe Gly Arg Val Glu Asp Leu
               100                 105                 110
   Ala Gly Ser Phe Val Val Ala Met Ile Thr Met Ser Ala Ile Ile Ala
           115                 120                 125
   Gly Tyr Glu Ala Ile Ala Arg Leu Ile His Pro Gln Gln Ile Glu His
       130                 135                 140
   Val Gly Trp Val Ala Leu Ala Gly Leu Val Gly Phe Ile Gly Asn Glu
   145                 150                 155                 160
   Trp Val Ala Leu Tyr Arg Ile Arg Val Gly His Arg Ile Gly Ser Ala
                   165                 170                 175
   Ala Leu Ile Ala Asp Gly Leu His Ala Arg Thr Asp Gly Phe Thr Ser
               180                 185                 190
   Leu Ala Val Leu Cys Ser Ala Gly Gly Val Ala Leu Gly Phe Pro Leu
           195                 200                 205
   Ala Asp Pro Ile Val Gly Leu Leu Ile Thr Ala Ala Ile Leu Ala Val
       210                 215                 220
   Leu Arg Thr Ala Ala Arg Asp Val Phe Arg Arg Leu Leu Asp Gly Val
   225                 230                 235                 240
   Asp Pro Ala Met Val Asp Ala Ala Glu Gln Ala Leu Ala Ala Arg Pro
                   245                 250                 255
   Gly Val Gln Ala Val Arg Ser Val Arg Met Arg Trp Ile Gly His Arg
               260                 265                 270
   Leu His Ala Asp Ala Glu Leu Asp Val Asp Pro Ala Leu Asp Leu Ala
           275                 280                 285
   Gln Ala His Arg Ile Ala His Asp Ala Glu His Glu Leu Thr His Thr
   290 295 300
   Val Pro Lys Leu Thr Thr Ala Leu Ile His Ala Tyr Pro Ala Glu His
   305 310 315 320
   Gly Ser Ser Ile Pro Asp Arg Gly Arg Thr Val Glu
   325 330
<210> 14
   <211> 615
   <212> PRT
   <213> Mycobacterium tuberculosis
<400> 14
   Val Val Asn Phe Ser Val Leu Pro Pro Glu Ile Asn Ser Gly Arg Met
   1 5 10 15
   Phe Phe Gly Ala Gly Ser Gly Pro Met Leu Ala Ala Ala Ala Ala Trp
   20 25 30
   Asp Gly Leu Ala Ala Glu Leu Gly Leu Ala Ala Glu Ser Phe Gly Leu
   35 40 45
   Val Thr Ser Gly Leu Ala Gly Gly Ser Gly Gln Ala Trp Gln Gly Ala
   50 55 60
   Ala Ala Ala Ala Met Val Val Ala Ala Ala Pro Tyr Ala Gly Trp Leu
   65 70 75 80
   Ala Ala Ala Ala Ala Arg Ala Gly Gly Ala Ala Val Gln Ala Lys Ala
   85 90 95
   Val Ala Gly Ala Phe Glu Ala Ala Arg Ala Ala Met Val Asp Pro Val
   100 105 110
   Val Val Ala Ala Asn Arg Ser Ala Phe Val Gln Leu Val Leu Ser Asn
   115 120 125
   Val Phe Gly Gln Asn Ala Pro Ala Ile Ala Ala Ala Glu Ala Thr Tyr
   130 135 140
   Glu Gln Met Trp Ala Ala Asp Val Ala Ala Met Val Gly Tyr His Gly
   145 150 155 160
   Gly Ala Ser Ala Ala Ala Ala Ala Leu Ala Pro Trp Gln Gln Ala Val
   165 170 175
   Pro Gly Leu Ser Gly Leu Leu Gly Gly Ala Ala Asn Ala Pro Ala Ala
   180 185 190
   Ala Ala Gln Gly Ala Ala Gln Gly Leu Ala Glu Leu Thr Leu Asn Leu
   195 200 205
   Gly Val Gly Asn Ile Gly Ser Leu Asn Leu Gly Ser Gly Asn Ile Gly
   210 215 220
   Gly Thr Asn Val Gly Ser Gly Asn Val Gly Gly Thr Asn Leu Gly Ser
   225 230 235 240
   Gly Asn Tyr Gly Ser Leu Asn Trp Gly Ser Gly Asn Thr Gly Thr Gly
   245 250 255
   Asn Ala Gly Ser Gly Asn Thr Gly Asp Tyr Asn Pro Gly Ser Gly Asn
   260 265 270
   Phe Gly Ser Gly Asn Phe Gly Ser Gly Asn Ile Gly Ser Leu Asn Val
   275 280 285
   Gly Ser Gly Asn Phe Gly Thr Leu Asn Leu Ala Asn Gly Asn Asn Gly
   290 295 300
   Asp Val Asn Phe Gly Gly Gly Asn Thr Gly Asp Phe Asn Phe Gly Gly
   305 310 315 320
   Gly Asn Asn Gly Thr Leu Asn Phe Gly Phe Gly Asn Thr Gly Ser Gly
   325 330 335
   Asn Phe Gly Phe Gly Asn Thr Gly Asn Asn Asn Ile Gly Ile Gly Leu
   340 345 350
   Thr Gly Asp Gly Gln Ile Gly Ile Gly Gly Leu Asn Ser Gly Thr Gly
   355 360 365
   Asn Ile Gly Phe Gly Asn Ser Gly Asn Asn Asn Ile Gly Phe Phe Asn
   370 375 380
   Ser Gly Asp Gly Asn Ile Gly Phe Phe Asn Ser Gly Asp Gly Asn Thr
   385 390 395 400
   Gly Phe Gly Asn Ala Gly Asn Ile Asn Thr Gly Phe Trp Asn Ala Gly
   405 410 415
   Asn Leu Asn Thr Gly Phe Gly Ser Ala Gly Asn Gly Asn Val Gly Ile
   420 425 430
   Phe Asp Gly Gly Asn Ser Asn Ser Gly Ser Phe Asn Val Gly Phe Gln
                   435                 440                 445
   Asn Thr Gly Phe Gly Asn Ser Gly Ala Gly Asn Thr Gly Phe Phe Asn
       450                 455                 460
   Ala Gly Asp Ser Asn Thr Gly Phe Ala Asn Ala Gly Asn Val Asn Thr
   465                 470                 475                 480
   Gly Phe Phe Asn Gly Gly Asp Ile Asn Thr Gly Gly Phe Asn Gly Gly
                   485                 490                 495
   Asn Val Asn Thr Gly Phe Gly Ser Ala Leu Thr Gln Ala Gly Ala Asn
               500                 505                 510
   Ser Gly Phe Gly Asn Leu Gly Thr Gly Asn Ser Gly Trp Gly Asn Ser
           515                 520                 525
   Asp Pro Ser Gly Thr Gly Asn Ser Gly Phe Phe Asn Thr Gly Asn Gly
       530                 535                 540
   Asn Ser Gly Phe Ser Asn Ala Gly Pro Ala Met Leu Pro Gly Phe Asn
   545                 550                 555                 560
   Ser Gly Phe Ala Asn Ile Gly Ser Phe Asn Ala Gly Ile Ala Asn Ser
                   565                 570                 575
   Gly Asn Asn Leu Ala Gly Ile Ser Asn Ser Gly Asp Asp Ser Ser Gly
               580                 585                 590
   Ala Val Asn Ser Gly Ser Gln Asn Ser Gly Ala Phe Asn Ala Gly Val
           595                 600                 605
   Gly Leu Ser Gly Phe Phe Arg
       610                 615
<210> 15
   <211> 590
   <212> PRT
   <213> Mycobacterium tuberculosis
<400> 15
   Met Asn Tyr Ser Val Leu Pro Pro Glu Ile Asn Ser Leu Arg Met Phe
   1               5                   10                  15
   Thr Gly Ala Gly Ser Ala Pro Met Leu Ala Ala Ser Val Ala Trp Asp
               20                  25                  30
   Arg Leu Ala Ala Glu Leu Ala Val Ala Ala Ser Ser Phe Gly Ser Val
           35                  40                  45
   Thr Ser Gly Leu Ala Gly Gln Ser Trp Gln Gly Ala Ala Ala Ala Ala
   50 55 60
   Met Ala Ala Ala Ala Ala Pro Tyr Ala Gly Trp Leu Ala Ala Ala Ala
   65 70 75 80
   Ala Arg Ala Ala Gly Ala Ser Ala Gln Ala Lys Ala Val Ala Ser Ala
   85 90 95
   Phe Glu Ala Ala Arg Ala Ala Thr Val His Pro Met Leu Val Ala Ala
   100 105 110
   Asn Arg Asn Ala Phe Val Gln Leu Val Leu Ser Asn Leu Phe Gly Gln
   115 120 125
   Asn Ala Pro Ala Ile Ala Ala Ala Glu Ala Met Tyr Glu Gln Met Trp
   130 135 140
   Ala Ala Asp Val Ala Ala Met Val Gly Tyr His Gly Gly Ala Ser Ala
   145 150 155 160
   Ala Ala Ala Gln Leu Ser Ser Trp Ser Ile Gly Leu Gln Gln Ala Leu
   165 170 175
   Pro Ala Ala Pro Ser Ala Leu Ala Ala Ala Ile Gly Leu Gly Asn Ile
   180 185 190
   Gly Val Gly Asn Leu Gly Gly Gly Asn Thr Gly Asp Tyr Asn Leu Gly
   195 200 205
   Ser Gly Asn Ser Gly Asn Ala Asn Val Gly Ser Gly Asn Ser Gly Asn
   210 215 220
   Ala Asn Val Gly Ser Gly Asn Asp Gly Ala Thr Asn Leu Gly Ser Gly
   225 230 235 240
   Asn Ile Gly Asn Thr Asn Leu Gly Ser Gly Asn Val Gly Asn Val Asn
   245 250 255
   Leu Gly Ser Gly Asn Arg Gly Phe Gly Asn Leu Gly Asn Gly Asn Phe
   260 265 270
   Gly Ser Gly Asn Leu Gly Ser Gly Asn Thr Gly Ser Thr Asn Phe Gly
   275 280 285
   Gly Gly Asn Leu Gly Ser Phe Asn Leu Gly Ser Gly Asn Ile Gly Ser
   290 295 300
   Ser Asn Ile Gly Phe Gly Asn Asn Gly Asp Asn Asn Leu Gly Leu Gly
   305 310 315 320
   Asn Asn Gly Asn Asn Asn Ile Gly Phe Gly Leu Thr Gly Asp Asn Leu
   325 330 335
   Val Gly Ile Gly Ala Leu Asn Ser Gly Ile Gly Asn Leu Gly Phe Gly
   340 345 350
   Asn Ser Gly Asn Asn Asn Ile Gly Phe Phe Asn Ser Gly Asn Asn Asn
   355 360 365
   Val Gly Phe Phe Asn Ser Gly Asn Asn Asn Phe Gly Phe Gly Asn Ala
   370 375 380
   Gly Asp Ile Asn Thr Gly Phe Gly Asn Ala Gly Asp Thr Asn Thr Gly
   385 390 395 400
   Phe Gly Asn Ala Gly Phe Phe Asn Met Gly Ile Gly Asn Ala Gly Asn
   405 410 415
   Glu Asp Met Gly Val Gly Asn Gly Gly Ser Phe Asn Val Gly Val Gly
   420 425 430
   Asn Ala Gly Asn Gln Ser Val Gly Phe Gly Asn Ala Gly Thr Leu Asn
   435 440 445
   Val Gly Phe Ala Asn Ala Gly Ser Ile Asn Thr Gly Phe Ala Asn Ser
   450 455 460
   Gly Ser Ile Asn Thr Gly Gly Phe Asp Ser Gly Asp Arg Asn Thr Gly
   465 470 475 480
   Phe Gly Ser Ser Val Asp Gln Ser Val Ser Ser Ser Gly Phe Gly Asn
   485 490 495
   Thr Gly Met Asn Ser Ser Gly Phe Phe Asn Thr Gly Asn Val Ser Ala
   500 505 510
   Gly Tyr Gly Asn Asn Gly Asp Val Gln Ser Gly Ile Asn Asn Thr Asn
   515 520 525
   Ser Gly Gly Phe Asn Val Gly Phe Tyr Asn Ser Gly Ala Gly Thr Val
   530 535 540
   Gly Ile Ala Asn Ser Gly Leu Gln Thr Thr Gly Ile Ala Asn Ser Gly
   545 550 555 560
   Thr Leu Asn Thr Gly Val Ala Asn Thr Gly Asp His Ser Ser Gly Gly
   565 570 575
   Phe Asn Gln Gly Ser Asp Gln Ser Gly Phe Phe Gly Gln Pro
   580 585 590
<210> 16
   <211> 308
   <212> PRT
   <213> Mycobacterium tuberculosis
<400> 16
   Met Ser Phe Val Phe Ala Ala Pro Glu Ala Leu Ala Ala Ala Ala Ala
   1               5                   10                  15
   Asp Met Ala Gly Ile Gly Ser Thr Leu Asn Ala Ala Asn Val Val Ala
               20                  25                  30
   Ala Val Pro Thr Thr Gly Val Leu Ala Ala Ala Ala Asp Glu Val Ser
           35                  40                  45
   Thr Gln Val Ala Ala Leu Leu Ser Ala His Ala Gln Gly Tyr Gln Gln
       50                  55                  60
   Leu Ser Arg Gln Met Met Thr Ala Phe His Asp Gln Phe Val Gln Ala
   65                  70                  75                  80
   Leu Arg Ala Ser Ala Asp Ala Tyr Ala Thr Ala Glu Ala Ser Ala Ala
                   85                  90                  95
   Gln Thr Met Val Asn Ala Val Asn Ala Pro Ala Arg Ala Leu Leu Gly
               100                 105                 110
   His Pro Leu Ile Ser Ala Asp Ala Ser Thr Gly Gly Gly Ser Asn Ala
           115                 120                 125
   Leu Ser Arg Val Gln Ser Met Phe Leu Gly Thr Gly Gly Ser Ser Ala
       130                 135                 140
   Leu Gly Gly Ser Ala Ala Ala Asn Ala Ala Ala Ser Gly Ala Leu Gln
   145                 150                 155                 160
   Leu Gln Pro Thr Gly Gly Ala Ser Gly Leu Ser Ala Val Gly Ala Leu
                   165                 170                 175
   Leu Pro Arg Ala Gly Ala Ala Ala Ala Ala Ala Leu Pro Ala Leu Ala
               180                 185                 190
   Ala Glu Ser Ile Gly Asn Ala Ile Lys Asn Leu Tyr Asn Ala Val Glu
           195                 200                 205
   Pro Trp Val Gln Tyr Gly Phe Asn Leu Thr Ala Trp Ala Val Gly Trp
       210                 215                 220
   Leu Pro Tyr Ile Gly Ile Leu Ala Pro Gln Ile Asn Phe Phe Tyr Tyr
   225                 230                 235                 240
   Leu Gly Glu Pro Ile Val Gln Ala Val Leu Phe Asn Ala Ile Asp Phe
                   245                 250                 255
   Val Asp Gly Thr Val Thr Phe Ser Gln Ala Leu Thr Asn Ile Glu Thr
               260                 265                 270
   Ala Thr Ala Ala Ser Ile Asn Gln Phe Ile Asn Thr Glu Ile Asn Trp
           275                 280                 285
   Ile Arg Gly Phe Leu Pro Pro Leu Pro Pro Ile Ser Pro Pro Gly Phe
       290                 295                 300
   Pro Ser Leu Pro
   305
<210> 17
   <211> 132
   <212> PRT
   <213> Mycobacterium tuberculosis
<400> 17
   Met Asp Tyr Ala Phe Leu Pro Pro Glu Ile Asn Ser Ala Arg Met Tyr
   1               5                   10                  15
   Ser Gly Pro Gly Pro Asn Ser Met Leu Val Ala Ala Ala Ser Trp Asp
               20                  25                  30
   Ala Leu Ala Ala Glu Leu Ala Ser Ala Ala Glu Asn Tyr Gly Ser Val
           35                  40                  45
   Ile Ala Arg Leu Thr Gly Met His Trp Trp Gly Pro Ala Ser Thr Ser
       50                  55                  60
   Met Leu Ala Met Ser Ala Pro Tyr Val Glu Trp Leu Glu Arg Thr Ala
   65                  70                  75                  80
   Ala Gln Thr Lys Gln Thr Ala Thr Gln Ala Arg Ala Ala Ala Ala Ala
                   85                  90                  95
   Phe Glu Gln Ala His Ala Met Thr Val Pro Pro Ala Leu Val Thr Gly
               100                 105                 110
   Ile Arg Gly Ala Ile Val Val Glu Thr Ala Ser Ala Ser Asn Thr Ala
   115 120 125
   Gly Thr Pro Pro
   130
<210> 18
   <211> 905
   <212> PRT
   <213> Mycobacterium tuberculosis
<400> 18
   Leu Ser Ala Ser Val Ser Ala Thr Thr Ala His His Gly Leu Pro Ala
   1               5                   10                  15
   His Glu Val Val Leu Leu Leu Glu Ser Asp Pro Tyr His Gly Leu Ser
               20                  25                  30
   Asp Gly Glu Ala Ala Gln Arg Leu Glu Arg Phe Gly Pro Asn Thr Leu
           35                  40                  45
   Ala Val Val Thr Arg Ala Ser Leu Leu Ala Arg Ile Leu Arg Gln Phe
       50                  55                  60
   His His Pro Leu Ile Tyr Val Leu Leu Val Ala Gly Thr Ile Thr Ala
   65                  70                  75                  80
   Gly Leu Lys Glu Phe Val Asp Ala Ala Val Ile Phe Gly Val Val Val
                   85                  90                  95
   Ile Asn Ala Ile Val Gly Phe Ile Gln Glu Ser Lys Ala Glu Ala Ala
               100                 105                 110
   Leu Gln Gly Leu Arg Ser Met Val His Thr His Ala Lys Val Val Arg
           115                 120                 125
   Glu Gly His Glu His Thr Met Pro Ser Glu Glu Leu Val Pro Gly Asp
       130                 135                 140
   Leu Val Leu Leu Ala Ala Gly Asp Lys Val Pro Ala Asp Leu Arg Leu
   145                 150                 155                 160
   Val Arg Gln Thr Gly Leu Ser Val Asn Glu Ser Ala Leu Thr Gly Glu
                   165                 170                 175
   Ser Thr Pro Val His Lys Asp Glu Val Ala Leu Pro Glu Gly Thr Pro
               180                 185                 190
   Val Ala Asp Arg Arg Asn Ile Ala Tyr Ser Gly Thr Leu Val Thr Ala
           195                 200                 205
   Gly His Gly Ala Gly Ile Val Val Ala Thr Gly Ala Glu Thr Glu Leu
       210                 215                 220
   Gly Glu Ile His Arg Leu Val Gly Ala Ala Glu Val Val Ala Thr Pro
   225                 230                 235                 240
   Leu Thr Ala Lys Leu Ala Trp Phe Ser Lys Phe Leu Thr Ile Ala Ile
                   245                 250                 255
   Leu Gly Leu Ala Ala Leu Thr Phe Gly Val Gly Leu Leu Arg Arg Gln
               260                 265                 270
   Asp Ala Val Glu Thr Phe Thr Ala Ala Ile Ala Leu Ala Val Gly Ala
           275                 280                 285
   Ile Pro Glu Gly Leu Pro Thr Ala Val Thr Ile Thr Leu Ala Ile Gly
       290                 295                 300
   Met Ala Arg Met Ala Lys Arg Arg Ala Val Ile Arg Arg Leu Pro Ala
   305                 310                 315                 320
   Val Glu Thr Leu Gly Ser Thr Thr Val Ile Cys Ala Asp Lys Thr Gly
                   325                 330                 335
   Thr Leu Thr Glu Asn Gln Met Thr Val Gln Ser Ile Trp Thr Pro His
               340                 345                 350
   Gly Glu Ile Arg Ala Thr Gly Thr Gly Tyr Ala Pro Asp Val Leu Leu
           355                 360                 365
   Cys Asp Thr Asp Asp Ala Pro Val Pro Val Asn Ala Asn Ala Ala Leu
       370                 375                 380
   Arg Trp Ser Leu Leu Ala Gly Ala Cys Ser Asn Asp Ala Ala Leu Val
   385                 390                 395                 400
   Arg Asp Gly Thr Arg Trp Gln Ile Val Gly Asp Pro Thr Glu Gly Ala
                   405                 410                 415
   Met Leu Val Val Ala Ala Lys Ala Gly Phe Asn Pro Glu Arg Leu Ala
               420                 425                 430
   Thr Thr Leu Pro Gln Val Ala Ala Ile Pro Phe Ser Ser Glu Arg Gln
           435                 440                 445
   Tyr Met Ala Thr Leu His Arg Asp Gly Thr Asp His Val Val Leu Ala
       450                 455                 460
   Lys Gly Ala Val Glu Arg Met Leu Asp Leu Cys Gly Thr Glu Met Gly
   465 470 475 480
   Ala Asp Gly Ala Leu Arg Pro Leu Asp Arg Ala Thr Val Leu Arg Ala
   485 490 495
   Thr Glu Met Leu Thr Ser Arg Gly Leu Arg Val Leu Ala Thr Gly Met
   500 505 510
   Gly Ala Gly Ala Gly Thr Pro Asp Asp Phe Asp Glu Asn Val Ile Pro
   515 520 525
   Gly Ser Leu Ala Leu Thr Gly Leu Gln Ala Met Ser Asp Pro Pro Arg
   530 535 540
   Ala Ala Ala Ala Ser Ala Val Ala Ala Cys His Ser Ala Gly Ile Ala
   545 550 555 560
   Val Lys Met Ile Thr Gly Asp His Ala Gly Thr Ala Thr Ala Ile Ala
   565 570 575
   Thr Glu Val Gly Leu Leu Asp Asn Thr Glu Pro Ala Ala Gly Ser Val
   580 585 590
   Leu Thr Gly Ala Glu Leu Ala Ala Leu Ser Ala Asp Gln Tyr Pro Glu
   595 600 605
   Ala Val Asp Thr Ala Ser Val Phe Ala Arg Val Ser Pro Glu Gln Lys
   610 615 620
   Leu Arg Leu Val Gln Ala Leu Gln Ala Arg Gly His Val Val Ala Met
   625 630 635 640
   Thr Gly Asp Gly Val Asn Asp Ala Pro Ala Leu Arg Gln Ala Asn Ile
   645 650 655
   Gly Val Ala Met Gly Arg Gly Gly Thr Glu Val Ala Lys Asp Ala Ala
   660 665 670
   Asp Met Val Leu Thr Asp Asp Asp Phe Ala Thr Ile Glu Ala Ala Val
   675 680 685
   Glu Glu Gly Arg Gly Val Phe Asp Asn Leu Thr Lys Phe Ile Thr Trp
   690 695 700
   Thr Leu Pro Thr Asn Leu Gly Glu Gly Leu Val Ile Leu Ala Ala Ile
   705 710 715 720
   Ala Val Gly Val Ala Leu Pro Ile Leu Pro Thr Gln Ile Leu Trp Ile
                   725                 730                 735
   Asn Met Thr Thr Ala Ile Ala Leu Gly Leu Met Leu Ala Phe Glu Pro
               740                 745                 750
   Lys Glu Ala Gly Ile Met Thr Arg Pro Pro Arg Asp Pro Asp Gln Pro
           755                 760                 765
   Leu Leu Thr Gly Trp Leu Val Arg Arg Thr Leu Leu Val Ser Thr Leu
       770                 775                 780
   Leu Val Ala Ser Ala Trp Trp Leu Phe Ala Trp Glu Leu Asp Asn Gly
   785                 790                 795                 800
   Ala Gly Leu His Glu Ala Arg Thr Ala Ala Leu Asn Leu Phe Val Val
                   805                 810                 815
   Val Glu Ala Phe Tyr Leu Phe Ser Cys Arg Ser Leu Thr Arg Ser Ala
               820                 825                 830
   Trp Arg Leu Gly Met Phe Ala Asn Arg Trp Ile Ile Leu Gly Val Ser
           835                 840                 845
   Ala Gln Ala Ile Ala Gln Phe Ala Ile Thr Tyr Leu Pro Ala Met Asn
       850                 855                 860
   Met Val Phe Asp Thr Ala Pro Ile Asp Ile Gly Val Trp Val Arg Ile
   865                 870                 875                 880
   Phe Ala Val Ala Thr Ala Ile Thr Ile Val Val Ala Thr Asp Thr Leu
                   885                 890                 895
   Leu Pro Arg Ile Arg Ala Gln Pro Pro
               900                 905
<210> 19
   <211> 636
   <212> DNA
   <213> Mycobacterium tuberculosis
<400> 19
   gtgccgcatc catgggacac cggcgatcac gaacggaatt ggcagggcta cttcatcccc        60
   gctatgtccg tcttgaggaa ccgggtcggc gctcgaacgc atgccgaact gcgtgatgcc       120
   gagaacgacc tcgttgaggc ccgggtgatc gaactccgcg aggatcccaa tctgctgggc       180
   gaccgcacag atctcgcata cctgcgggcg attcaccgcc agctgttcca ggacatttac       240
   gtctgggcgg gagatctgcg gacagtcggc atcgagaagg aggacgagtc tttctgcgcg       300
   ccgggcggca tcagtcggcc catggagcat gtggctgcgg agatctacca gctcgaccgg       360
   ctcagagcgg tcggcgaagg tgatctcgct ggccaggtcg cataccggta cgactacgtg       420
   aactatgccc acccgttccg cgagggcaac ggccgctcga cccgcgagtt cttcgatctc       480
   ctgttgtccg aacgcggttc tggcctcgac tgggggaaga ccgacctgga agagttgcac       540
   ggcgcttgtc acgtggcgcg cgccaactct gatctcacgg gcctggtcgc gatgttcaag       600
   gggatcctcg acgccgagcc cacttacgac ttctga                                 636
<210> 20
   <211> 1143
   <212> DNA
   <213> Mycobacterium tuberculosis
<400> 20
   atggatttcg cactgttacc accggaagtc aactccgccc ggatgtacac cggccctggg 60
   gcaggatcgc tgttggctgc cgcgggcggc tgggattcgc tggccgccga gttggccacc 120
   acagccgagg catatggatc ggtgctgtcc ggactggccg ccttgcattg gcgtggaccg 180
   gcagcggaat cgatggcggt gacggccgct ccctatatcg gttggctgta cacgaccgcc 240
   gaaaagacac agcaaacagc gatccaagcc agggcggcag cgctggcctt cgagcaagca 300
   tacgcaatga ccctgccgcc accggtggta gcggccaacc ggatacagct gctagcactg 360
   atcgcgacga acttcttcgg ccagaacact gcggcgatcg cggccaccga ggcacagtac 420
   gccgagatgt gggcccagga cgccgccgcg atgtacggtt acgccaccgc ctcagcggct 480
   gcggccctgc tgacaccgtt ctccccgccg cggcagacca ccaacccggc cggcctgacc 540
   gctcaggccg ccgcggtcag ccaggccacc gacccactgt cgctgctgat tgagacggtg 600
   acccaagcgc tgcaagcgct gacgattccg agcttcatcc ctgaggactt caccttcctt 660
   gacgccatat tcgctggata tgccacggta ggtgtgacgc aggatgtcga gtcctttgtt 720
   gccgggacca tcggggccga gagcaaccta ggccttttga acgtcggcga cgagaatccc 780
   gcggaggtga caccgggcga ctttgggatc ggcgagttgg tttccgcgac cagtcccggc 840
   ggtggggtgt ctgcgtcggg tgccggcggt gcggcgagcg tcggcaacac ggtgctcgcg 900
   agtgtcggcc gggcaaactc gattgggcaa ctatcggtcc caccgagctg ggccgcgccc 960
   tcgacgcgcc ctgtctcggc attgtcgccc gccggcctga ccacactccc ggggaccgac 1020
   gtggccgagc acgggatgcc aggtgtaccg ggggtgccag tggcagcagg gcgagcctcc 1080
   ggcgtcctac ctcgatacgg ggttcggctc acggtgatgg cccacccacc cgcggcaggg 1140
   taa 1143
<210> 21
   <211> 720
   <212> DNA
   <213> Mycobacterium tuberculosis
<400> 21
   atgactgagc ccagacctgt cttcgctgtc gtgatcagcg ccggcctatc cgccatcccg 60
   atggtcggcg gcccgctaca aaccgtgttc gacgccatcg aggaacgcac ccggcaccgc     120
   gccgagacaa ccacgcgcga gatatgcgag agcgtcggcg gcgcggacac cgtgttgagc     180
   cgcattgaca aaaatcccga actcgagccg cttctcagcc aggcgatcga ggccgccact     240
   cgcaccagta tggaggccaa gcgccggctc ctcgcgcaag ctgccgccgc cgcgctcgag     300
   gatgaccaga aggtcgagcc ggcatcactc atcgtggcca cgctttccca acttgagccc     360
   gtgcatatcc atgcactcgt tcggctggcc aaagccgcca agtcctcacc ggaccaggac     420
   gagatccagc gacgcgaggt gatgagggcg gcgagtaagg tcgagcccgt gccggtgcta     480
   gcggccctca ttcaaaccgg cgtcgcgatc gcgacaacaa ccgtttggca cggcaacggc     540
   accgggactc cggcagaaga aagcggccac atccttatcc acgacgtcag cgacttcggc     600
   caccgcctgc tggcctatct cagggccgcc gacgcaggtg ccgagctcct catcctcccc     660
   tctggagggt ctgcgccaac cggcgaccac ccgacgccgc acccgtccac gtcgagatga     720
<210> 22
   <211> 1440
   <212> DNA
   <213> Mycobacterium tuberculosis
<400> 22
   atggcggatt tcttgacgtt gtcaccagag gtgaattcgg cccggatgta cgcgggtggg      60
   gggcccgggt cgctatcggc ggccgcggcg gcctgggatg agttggccgc cgaactgtgg     120
   ttggcggcgg cctcgttcga gtcggtgtgc tccggcctgg cggaccgttg gtggcaaggg     180
   ccgtcgtctc ggatgatggc ggcgcaggcc gcccgccata cggggtggct ggccgcggcg     240
   gccacccagg cagagggagc agccagccag gctcagacga tggcgctggc ctatgaagcg     300
   gcgttcgccg caaccgtaca cccggcgctg gtcgcggcga accgcgccct cgtggcctgg     360
   ttggcggggt cgaatgtgtt cgggcagaac accccggcga ttgcggccgc cgaggccatc     420
   tacgagcaga tgtgggctca ggatgttgtc gcgatgttga actaccatgc ggtggcctcg     480
   gcggtcgggg cgcggttgcg gccgtggcag cagttgctgc atgagctgcc caggcggttg     540
   ggcggcgaac actccgacag cacaaacacg gaactcgcta acccgagttc aacgacgaca     600
   cgcattaccg tccccggcgc atctccggtg catgcagcga cgttactgcc gttcatcgga     660
   aggctactgg cggcgcgtta tgccgagctg aacaccgcga tcggcacgaa ctggtttccg     720
   ggcaccacgc cagaagtggt gagctatccg gccaccatcg gggtccttag cggctctctt     780
   ggcgccgtcg atgccaacca gtccatcgct atcggtcagc agatgttgca caacgagatc     840
   ctggccgcca cggcctccgg tcagccggtg acggtggccg gactgtcgat gggcagcatg     900
   gtcatcgacc gcgaacttgc ctatctggcc atcgacccca acgcgccacc ctcgagcgcg     960
   ctcacattcg tcgagctcgc cggcccggaa cgcggtcttg cccagaccta cctgcccgtt    1020
   ggcaccacca ttccaatcgc ggggtacacc gtggggaatg cgcccgagag ccagtacaac    1080
   accagcgtgg tttatagcca gtacgatatc tgggccgatc cgcccgaccg tccgtggaac    1140
   ctgttggccg gcgccaacgc actgatgggc gcggcttact ttcacgatct gaccgcctac    1200
   gccgcaccac aacaggggat agagatcgcc gctgtcacga gttcactggg cggaaccacg    1260
   acaacgtaca tgattccgtc gcccacgctg ccgttgctgt tgccactgaa gcagatcggt    1320
   gtcccagact ggatcgtcgg cgggctgaac aacgtgctga agccgctcgt cgacgcgggc    1380
   tactcacagt acgcccccac cgccggccct tatttcagcc acggcaacct ggtgtggtag    1440
<210> 23
   <211> 1185
   <212> DNA
   <213> Mycobacterium tuberculosis
<400> 23
   atgaccctcg atgtcccggt caaccagggg catgtccccc cgggcagcgt cgcctgctgc      60
   cttgttgggg tcaccgccgt tgctgacggc atcgccgggc attccctgtc caactttggg     120
   gcgttacctc ccgagatcaa ttcgggtcgt atgtatagcg gtccgggatc cgggccactg     180
   atggctgccg cggcggcctg ggacgggctg gccgcagagt tgtcgtcggc agcgactggc     240
   tacggtgcgg cgatctcgga gctgacaaac atgcggtggt ggtcggggcc ggcatcggat     300
   tcgatggtgg ccgccgtcct gccctttgtc ggctggctga gtaccaccgc gacgctagcc     360
   gaacaggccg cgatgcaggc tagggcggcc gcagcggcct ttgaagccgc cttcgccatg     420
   acggtgcccc cgccggcgat cgcggccaac cggaccttgt tgatgacgct cgtcgatacc     480
   aactggttcg ggcaaaacac gccggcgatc gccaccaccg agtcccaata cgccgagatg     540
   tgggcccaag acgccgccgc gatgtacggc tatgccagcg ccgcggcacc cgccacggtt     600
   ttgactccgt tcgcaccacc gccgcaaacc accaacgcga ccggcctcgt cggccacgca     660
   acagcggtgg ccgcgctgcg ggggcagcac agctgggccg cggcgattcc atggagcgac     720
   atacagaaat actggatgat gttcctgggc gccctcgcca ctgccgaagg gttcatttac     780
   gacagcggtg ggttaacgct gaatgctctg cagttcgtcg gcgggatgtt gtggagcacc     840
   gcattggcag aagccggtgc ggccgaggca gcggccggcg cgggtggagc cgctggatgg     900
   tcggcgtggt cgcagctggg agctggaccg gtggcggcga gcgcgactct ggccgccaag     960
   atcggaccga tgtcggtgcc gccgggctgg tccgcaccgc ccgccacgcc ccaggcgcaa    1020
   accgtcgcgc gatcgattcc cggtattcgc agcgccgccg aggcggctga aacatcggtc    1080
   ctactccggg gggcaccgac tccgggcagg agtcgcgccg cccatatggg acgccgatat    1140
   ggaagacgac tcaccgtgat ggctgaccgg ccgaacgtcg gatag                     1185
<210> 24
   <211> 1176
   <212> DNA
   <213> Mycobacterium tuberculosis
<400> 24
   atggatttcg gagctttacc ccctgagatc aactccgcac gcatgtacgc cggcgcgggt      60
   gcaggaccga tgatggccgc cggggccgca tggaacggcc tggccgccga gttgggtacg     120
   acggccgcgt cgtatgagtc ggtgatcacc cggctgacca ccgagtcgtg gatgggtccg     180
   gcctcgatgg cgatggtcgc cgcagcccag ccctatctgg cttggttgac ctacaccgcc     240
   gaagccgctg cgcatgccgg ctcgcaggcc atggcgtcgg cggccgccta cgaggcggcc     300
   tatgcgatga cagtgccgcc ggaggtggtc gcggccaacc gggcgctgct ggcggccctg     360
   gtcgcgacga acgtcctggg gatcaacaca ccggcaatca tggcgaccga agccctctat     420
   gccgagatgt gggctcagga cgctctggct atgtacggct acgcggccgc ttcgggagcc     480
   gccgggatgc tgcaaccgtt aagcccgccg tcgcagacca ccaacccggg cgggctggcc     540
   gcccagtccg ccgcggtcgg ctcggctgcc gccaccgccg ccgtcaacca ggtgagcgta     600
   gcggacctga tcagtagcct gcccaacgcg gtgagtgggc tcgcctcccc agtcacatcg     660
   gttctcgact cgacggggct gagcggaatc attgccgaca tcgacgccct gctcgcgacc     720
   ccgttcgtgg caaacatcat caacagcgca gtcaacaccg ccgcttggta tgtcaacgcc     780
   gccatcccca ccgcgatatt cctagcaaat gccctgaaca gtggggcgcc ggtagcgatc     840
   gccgaaggcg ccatcgaggc tgccgagggt gccgccagtg cggccgccgc ggggttggcg     900
   gactcggtga cgccagcggg gctcggcgca agtttaggcg aggccaccct ggtcggccgg     960
   ctgtcagtgc cggcggcctg gtctacggcc gcaccggcga caaccgccgg cgccacagcg    1020
   ctcgaaggca gcggctggac cgtcgccgcc gaagaagccg gcccagttac cgggatgatg    1080
   ccgggaatgg cctcggccgc caagggcacc ggtgcctatg ccgggccgcg gtacggattc    1140
   aagcccactg tcatgcccaa acaggtcgtc gtgtga                              1176
<210> 25
   <211> 1659
   <212> DNA
   <213> Mycobacterium tuberculosis
<400> 25
   atggctcatt tttcggtgtt gccgccggag atcaactcgt tgcggatgta cctgggtgcc 60
   ggttcggcgc cgatgcttca ggcggcggcg gcctgggacg ggctggccgc ggagttggga 120
   accgccgcgt cgtcgttctc ctcggtgacc acggggttaa ccgggcaggc gtggcagggc 180
   ccggcgtcgg cggcgatggc cgccgcggcg gcgccgtatg cgggcttttt gaccacagcc 240
   tcggctcaag cccagctggc tgccgggcag gctaaggcgg tggccagcgt gttcgaggcc 300
   gccaaggccg cgatcgtgcc tccggccgcg gtggcggcca accgtgaggc gttcttggcg 360
   ttgattcggt cgaattggct ggggctcaac gcgccgtgga tcgccgccgt tgaaagcctt 420
   tacgaggaat actgggccgc tgatgtggcg gcgatgaccg gctatcacgc cggggcctcg 480
   caggccgccg cgcagttgcc gttgccggcc ggcctgcaac agttcctcaa caccctgccc 540
   aatctgggca tcggcaacca gggcaacgcc aacctcggcg gcggcaacac cggcagcggc 600
   aacatcggca acggaaacaa aggcagctcc aacctcggcg gcggcaacat cggcaataac 660
   aacatcggca gcggcaaccg aggcagcgac aacttcggcg ccggcaacgt cggcaccgga 720
   aacatcggct tcggcaacca gggccccata gacgttaacc tcttggcgac gccgggccag 780
   aacaacgtgg gcctgggcaa catcggcaac aacaacatgg gcttcggcaa caccggcgac 840
   gccaacaccg gcggcggcaa caccggcaac ggcaacatcg gtggcggcaa caccggcaac 900
   aacaacttcg gcttcggcaa caccggcaac aacaacatcg gaatcgggct caccggcaac 960
   aatcagatgg gcatcaacct ggccgggctg ctgaactccg gcagcggcaa tatcggcatc 1020
   ggcaactccg gcaccaacaa catcggcttg ttcaactccg gcagcggcaa catcggcgtc 1080
   ttcaacaccg gagccaatac cctggtgcct ggcgacctca acaacctggg cgtcgggaat 1140
   tccggcaacg ccaacatcgg cttcgggaac gcgggcgttc tcaacaccgg cttcgggaac 1200
   gcgagcatcc tcaacaccgg cttggggaac gcgggtgaat taaacaccgg cttcggaaac 1260
   gcgggcttcg tcaacacggg gtttgacaac tccggcaacg tcaacaccgg caatgggaac 1320
   tcgggcaaca tcaacaccgg ctcgtggaat gcgggcaatg tgaacaccgg tttcgggatc 1380
   attaccgaca gcggcctgac caactcgggc ttcggcaaca ccggcaccga cgtctcgggc 1440
   ttcttcaaca cccccaccgg ccccttagcc gtcgacgtct ccgggttctt caacacggcc 1500
   agcgggggca ctgtcatcaa cggccagacc tcgggcattg gcaacatcgg cgtcccgggc 1560
   accctctttg gctccgtccg gagcggcttg aacacgggcc tgtttaacat gggcaccgcc 1620
   atatcggggt tgttcaacct gcgccagctg ttggggtag 1659
<210> 26
   <211> 1689
   <212> DNA
   <213> Mycobacterium tuberculosis
<400> 26
   atggagtatc tgattgcagc gcaggacgtg ttggtggccg cggccgctga tttggagggc      60
   atcggctcgg cgctggccgc agctaacagg gcggccgagg ccccgaccac ggggctgctg     120
   gccgcgggtg ccgacgaggt atcagcggcc atcgcgtcgc tgttttccgg gaacgcccag     180
   gcctatcaag cgctgagcgc acaggcggcg gcatttcatc agcagtttgt gcgggcactg     240
   agttcggcgg ccggctcgta tgcggcggcc gaggccgcca atgcctcccc gatgcaagcg     300
   gtgctggatg tggtcaatgg gcccacccag ctgttgctgg ggcgcccgct gatcggcgat     360
   ggcgccaacg gcgggccggg acaaaacggg ggcgacggtg gcttgttgta cggcaacggc     420
   ggcaacggcg gctcgagtag caccccaggc cagcccggcg gtcgcggcgg cgcggccggg     480
   ttgatcggca acggtggcgc cgggggagcc ggcgggcccg gcgcgaacgg cggtgccggc     540
   ggcaacggcg ggtggctata cggcaacggt gggctcggcg gcaacggtgg ggcggccacc     600
   cagatcgggg gcaatggcgg caacggaggc cacggcggca acgccgggct atggggcaac     660
   gggggggcgg gtggagccgg agcggcagga gcggccggcg ccaacgggca aaacccggtg     720
   tcccatcagg tcacgcacgc gaccgatggc gccgacggca ctaccggacc cgatggcaac     780
   gggaccgacg ccggctcggg cagcaacgca gtcaaccccg gcgtgggcgg tggtgcaggc     840
   ggcataggcg gggatggaac caaccttggg cagaccgacg tgtccggggg tgccggcggc     900
   gacggcggcg acggcgccaa cttcgcctcc ggaggtgccg gcggtaacgg tggcgccgct     960
   caaagcggct ttggtgacgc tgtcggcggc aatggcggcg ccggcggaaa cggcggagcc    1020
   ggcggcggcg gggggctggg cggagcgggt ggcagcgcca atgttgcaaa tgctggcaac    1080
   agcatagggg gcaacggtgg cgccggcggg aacggcggta tcggcgctcc cggtggtgcc    1140
   ggcggcgccg gaggaaatgc caatcaagat aatcctcctg ggggcaactc caccgggggc    1200
   aatggtggtg ccggcggcga cggcggcgtc ggtgcctcgg ctgacgttgg tggcgccggc    1260
   ggctttgggg gcagcggggg tcgcggcggg ctactgctcg gcacgggcgg cgccggcggc    1320
   gacggcggcg tcgggggcga cgggggcatc ggcgctcaag gcggcagcgg cggcaacggc    1380
   ggcaacggcg ggatcggcgc cgacggcatg gccaaccagg acggcgacgg cggtgacggc    1440
   ggcaacggcg gcgacggcgg ggccggcggg gccggtggcg tcggcggaaa cggcgggacc    1500
   ggcggtgcgg gcggactgtt cggacagtcg ggcagccccg gctccggcgc ggccgggggc    1560
   ctcggcggcg cgggcggcaa cggcggcgcg ggcggcggcg gcgggaccgg gttcaacccc    1620
   ggcgcccccg gcgatcccgg tactcaaggc gctaccggcg ccaacggtca gcacggcctg    1680
   aacggctga                                                          1689
<210> 27
   <211> 4617
   <212> DNA
   <213> Mycobacterium tuberculosis
<400> 27
<210> 28
   <211> 1272
   <212> DNA
   <213> Mycobacterium tuberculosis
<400> 28
   gtgatccaaa cgtgtgaagt cgagttgcgc tggcgtgcat cacaactgac gctggcgatt 60
   gccacctgtg ccggagttgc gctagccgca gcggtcgtcg ctggtcgttg gcagctgatt 120
   gcgttcgcgg cgccgctgct cggcgtgttg tgctcgatca gctggcagcg tccggtcccg 180
   gtgatccagg tgcacggtga cccggattcg cagcgatgtt tcgagaacga acatgtgcga 240
   gtgaccgtgt gggtcacaac ggaatccgtg gacgccgcgg tcgaactcac ggtatcggcg 300
   ttggcgggaa tgcagttcga agctctggaa tccgtgtcac gccggacgac aacggtttcc 360
   gcggtggcgc aacgctgggg gcgctatcct atccgggccc gggtcgccgt cgtcgcacgc 420
   ggtgggttgt tgatgggagc cggaaccgtc gacgccgccg aaatcgtcgt gtttccgctg 480
   acaccgccgc agtcgacgcc actgccgcag accgaattgc tcgaccgcct gggagctcat 540
   ctcacccggc acgtcgggcc gggtgtcgaa tacgccgaca ttcgcccata tgtcccgggc 600
   gaccagctac gtgccgtgaa ctgggtggta agcgcgcgcc gtggccgact gcacgtgaca 660
   aggcggttga ccgaccgggc cgctgacgtg gtggtgttga tcgacatgta tcgacagccg 720
   gcgggtccgg cgaccgaggc caccgaacga gtcgtgcggg gtgctgctca ggtggtgcaa 780
   accgcgctgc gaaacggtga ccgtgctggg atcgttgcgc tgggcggcaa tcggccgcga 840
   tggctgggcg ccgacatcgg gcagcgccag ttctatcggg tgctcgacac cgtgctcggc 900
   gccggggaag ggttcgaaaa caccaccggg acgctggctc cgcgcgcagc tgttcccgca 960
   ggagcggttg tcattgcgtt ttccacgctg ctggataccg agttcgcgct ggcgttgatc 1020
   gacctgcgta aacgcggcca cgtcgtggtt gctgtcgacg ttcttgatag ctgtccgctc 1080
   caggaccaac tggatcccct ggtggtccgg atgtgggcgc tgcagcgctc cgcgatgtat 1140
   cgcgacatgg ccaccatcgg ggtcgacgtg ctgtcctggc cggcggatca ctcgcttcag 1200
   cagtcgatgg gtgcgttgcc caatcgccgt cgtcgcggac ggggcagagc tagccgggcg 1260
   aggctgccat ga 1272
<210> 29
   <211> 1023
   <212> DNA
   <213> Mycobacterium tuberculosis
<400> 29
   gtgaataggc ggatattgac cttgatggtc gcgctggtgc cgatcgtggt attcggcgtg     60
   ttgctcgccg tggtaaccgt gccgtttgtg gcgctgggac ccggcccaac gttcgacacg    120
   ctcggcgaga tagacggcaa gcaggtggtc cagatcgtgg gcacccagac ctacccgacg    180
   tcaggtcacc tcaacatgac gacggtctcc cagcgcgacg gtctaaccct gggtgaagcc    240
   ctggccctgt ggctttcggg tcaagaacag ttgatgccac gcgacctcgt ctaccctccg    300
   ggcaagtcgc gggaagagat cgaaaatgac aacgccgctg atttcaagcg ctccgaggcc    360
   gccgctgagt acgccgctct ggggtacctg aagtatccga aagcagtcac cgtcgcctcg    420
   gtcatggatc cagggccatc ggtggacaag ctgcaggccg gtgacgccat cgacgccgtc    480
   gacggcactc cggtgggcaa cctcgaccag ttcaccgcgc tgttgaagaa cacgaaaccg    540
   ggccaggagg tgacgatcga cttccgccgc aagaacgagc cgcccggcat cgcgcagatc    600
   acgctgggca agaataagga tcgcgaccaa ggcgtcctgg gcatagaggt ggtggacgcg    660
   ccgtgggcgc cgtttgccgt ggacttccac ctcgccaacg tcggcggccc ttcggccgga    720
   ctgatgttca gtctggccgt cgtcgacaag ctcaccagtg gccacctggt tgggtcgacg    780
   ttcgtcgcag gcaccggcac gatcgccgtc gatggcaagg tgggccagat cggtggcatc    840
   acccacaaga tggccgctgc tcgagcggcc ggcgcgacgg tgtttctggt gcccgcgaag    900
   aactgctacg aggcaagttc cgacagcccg cccggtttga agttggtgaa ggtcgagacg    960
   cttagccagg cggtggacgc gctgcacgcg atgacgtcgg gctcgccgac gccgagctgc   1020
   tag                                                                 1023
<210> 30
   <211> 2772
   <212> DNA
   <213> Mycobacterium tuberculosis
<400> 30
   atgtccttcg tggtcacagc accgccggtg ctcgcgtcgg cggcgtcgga tctgggcggt      60
   atcgcgtcca tgatcagcga ggccaacgcg atggcagcgg tccgaacgac ggcgttggcg     120
   cccgccgccg ccgacgaggt ttcggcggcg atcgcggcgc tgttttccag ctacgcgcgg     180
   gactatcaaa cgctgagcgt ccaggtgacg gccttccacg tgcagttcgc gcagacattg     240
   accaatgcgg ggcagctgta tgcggtcgtc gacgtcggca atggcgtgct gttgaagacc     300
   gagcagcagg tgctgggtgt gatcaatgcg cccacccaga cgttggtggg tcgtccgctg     360
   atcggcgatg gcacccacgg ggcgccgggg accgggcaga acggtggggc gggcggaatc     420
   ttgtggggca acggcggtaa cggcgggtcc ggggctcccg gacagccggg cggccggggc     480
   ggtgatgccg gcctgttcgg ccacggcggt catggcggtg tcggggggcc gggcatcgcc     540
   ggtgccgctg gcaccgcggg cctgcccggg ggcaacggcg ccaacggcgg aagcggcggc     600
   atcggcggcg ccggcggcgc cggcggcaac ggcgggctgc tattcggcaa cggtggtgcc     660
   ggcggccagg gtggctccgg cggacttggg ggctccggcg ggacggcggg cgcgggcatg      720
   gctgccggtc ccgccggcgg caccggcggc atcgggggca tcggcggcat cggcggcgcg 780
   ggcggggtcg gcggccacgg ctcggcgttg ttcggccacg ggggaatcaa cggcgatggc 840
   ggtaccggcg gcatgggtgg ccagggcggt gctggcggca acggctgggc cgctgagggc 900
   atcacggtcg gcattggtga gcaaggcggc cagggcggcg acgggggagc cggcggcgcc 960
   ggcgggatcg gtggttcggc gggtgggatc ggcggcagcc agggtgcggg tgggcacggc 1020
   ggcgacggcg gccagggcgg cgccggcggt agtggcggcg ttggcggcgg cggcgcaggc 1080
   gccggcggcg acggcggcgc gggcggcatc ggcggcactg gcggtaacgg cagcatcggc 1140
   ggggccgccg gcaatggcgg taacggcggc cgcggcggcg ccggtggcat ggccaccgcg 1200
   ggaagtgatg gcggcaatgg cggcggcggc ggcaacggcg gcgtcggtgt tggcagcgcc 1260
   ggaggggccg gcggcaccgg cggtgacggc ggggcggccg gggcgggcgg cgcgccgggc 1320
   cacggctact tccaacagcc cgcgccccaa gggctgccca tcggaaccgg cgggaccggc 1380
   ggcgaaggcg gtgccggcgg cgccggtgga gacggcgggc agggcgacat cggcttcgat 1440
   ggcggccggg gtggcgacgg cggcccgggc ggtggcggcg gcgccggcgg tgacggcagc 1500
   ggcaccttca atgcccaagc caacaacggc ggcgacggtg gtgccggcgg tgttggggga 1560
   gccggcggca ccggcggcac gggtggggtc ggggccgacg ggggtcgcgg gggggactcg 1620
   ggccgcggcg gcgacggcgg caacgccggc cacggcggcg ccgcccaatt ctccggtcgc 1680
   ggcgcctacg gcggtgaagg tggcagcggc ggcgccggcg gcaacgccgg tggcgccggc 1740
   accggtggca ccgcgggctc cggcggtgcc ggaggtttcg gcggcaacgg tgccgatggc 1800
   ggcaatggcg gcaacggtgg caacggcggc ttcggcggaa ttaacggcac gttcggcacc 1860
   aacggtgccg gcggcaccgg cgggctcggc accctgctcg gcggccacaa cggcaacatc 1920
   ggcctcaacg gggccaccgg cggcatcggc agcaccacgt tgaccaacgc gaccgtaccg 1980
   ctgcagctgg tgaataccac cgagccggtg gtattcatct ccttaaacgg cggccaaatg 2040
   gtgcccgtgc tgctcgacac cggatccacc ggtctggtca tggacagcca attcctgacg 2100
   cagaacttcg gccccgtcat cgggacgggc accgccggtt acgccggcgg gctgacctac 2160
   aactacaaca cctactcaac gacggtggat ttcggcaatg gccttctcac cctgccgacc 2220
   agcgttaacg tcgtcacctc gtcatcaccg ggaaccctgg gcaacttctt gtcgagatcc 2280
   ggtgcggtgg gcgtcttggg aatcgggccc aacaacgggt tcccgggcac cagctccatc 2340
   gttaccgcga tgcccggcct gctcaacaac ggtgtgctca tcgacgaatc ggcgggcatc 2400
   ctgcagttcg gtcccaacac attaaccggc ggtatcacga tttctggagc accgatttcc 2460
   accgtggctg ttcagatcga caacgggccg ctgcaacaag ctccggtgat gttcgactcc 2520
   ggcggcatca acggaaccat cccgtcagcc ctcgccagcc tgccgtccgg gggattcgtg 2580
   ccggcgggaa cgaccatttc ggtctacacc agcgacggcc agacgctgtt gtactcctac 2640
   accaccaccg cgacaaacac cccatttgtc acctccggcg gcgtgatgaa caccgggcac 2700
   gtccccttcg cgcagcaacc gatatacgtc tcctacagcc ccaccgccat cgggacgacc 2760
   acctttaact ga 2772
<210> 31
   <211> 999
   <212> DNA
   <213> Mycobacterium tuberculosis
<400> 31
   atgacccacg accacgctca ttcacgaggt gtgccggcga tgatcaagga gatcttcgcg 60
   ccgcactccc acgacgccgc cgacagcgtc gacgacaccc tggaatccac tgcggcaggg 120
   atccgtacgg tcaagatcag cttgttggtt ctcgggttga ccgcgctcat ccagattgtg 180
   atcgtggtga tgtcggggtc ggttgcgctg gccgccgaca ccatccacaa cttcgctgat 240
   gcgttgaccg cggtgccgtt gtggatcgcg ttcgcgttgg gcgccaagcc cgccactcgc 300
   cgatatacct acggattcgg tcgcgtcgag gacctggccg ggtcgttcgt ggtcgcgatg 360
   atcacgatgt cggccatcat cgccggttac gaagccatcg cccgcctgat ccacccgcag 420
   cagatcgagc atgtcggctg ggtcgccctg gccgggctgg tcggattcat cggcaacgag 480
   tgggttgccc tctaccgcat cagggttggg caccgcatcg gctcggccgc cctgatcgcc 540
   gacggactac acgctcgaac cgacggattc acctcgctgg ccgtgctgtg ctcggccggc 600
   ggtgtcgcac ttgggttccc actggccgac cccatcgtcg gcctgctcat cacggcggcg 660
   attctggccg tgctacgaac tgccgcgcga gatgtgttcc gccgcctgct cgacggcgtc 720
   gacccagcga tggtcgatgc cgccgaacaa gccctggcgg cccggcccgg cgtgcaggcg 780
   gtacgcagcg tgcggatgcg ctggatcgga caccgcttgc acgccgatgc cgaactcgac 840
   gtcgaccccg ccctggacct cgcgcaagct caccgcatcg cccacgacgc cgaacacgaa 900
   ctcacccaca ccgttcccaa gctgaccacc gccctcatcc acgcctatcc ggctgaacat 960
   ggctcgtcga tcccagatcg tggccgcacc gtagagtga 999
<210> 32
   <211> 1848
   <212> DNA
   <213> Mycobacterium tuberculosis
<400> 32
   gtggtgaatt tttcggtgtt gccgccggag attaattcgg ggcggatgtt ttttggtgcg 60
   gggtcggggc cgatgttggc ggcggcggcg gcctgggatg ggttggcggc tgagttgggg 120
   ttggcggcgg agtcgtttgg gttggtgacc tcgggtctgg cgggtgggtc gggtcaggcg 180
   tggcagggtg cggcggcggc ggcgatggtg gtggcggcgg cgccgtatgc ggggtggttg 240
   gctgctgcgg cggcgcgggc tgggggggct gcggttcagg ctaaggcggt ggccggcgcg 300
   tttgaggcgg cgcgggcggc catggtggat ccggtggtgg tggcggctaa tcgcagtgcg 360
   tttgtgcagt tggtgctgtc gaatgtgttt gggcagaatg cgccggcgat tgccgctgct 420
   gaggccacct atgagcagat gtgggctgcc gatgtggcgg cgatggtggg ttatcacggt 480
   ggggcatcgg cggcggcggc ggcgttggcg ccatggcagc aggcggtgcc gggcttgtcg 540
   ggcttgctag gcggtgcggc taacgcaccg gcggccgctg cacaaggcgc tgcacaaggc 600
   ctcgccgagc tgaccttgaa tttgggtgtc ggcaacatcg gcagcctcaa cctgggcagc 660
   ggcaacatcg gcggtaccaa cgtgggcagt ggcaatgtcg gcggcaccaa cctgggcagc 720
   gggaactacg gcagcctgaa ctggggcagc ggaaacaccg gtaccggcaa tgccggcagc 780
   ggaaacacgg gtgactacaa ccctggcagc ggaaacttcg gcagcggaaa cttcggcagc 840
   ggaaatatcg gcagcctcaa tgtgggcagc ggaaacttcg gcacgctcaa cctcgccaac 900
   ggaaataacg gtgatgtcaa tttcggcggc gggaacaccg gcgacttcaa ctttggcggc 960
   gggaataatg gcaccctcaa ctttgggttc ggaaacaccg gcagcgggaa tttcggtttc 1020
   ggaaacacgg gcaacaacaa tatcggtatc gggctcaccg gtgatggtca gatcggcatc 1080
   ggcggactga actcaggcac tggaaacatc ggcttcggaa actccggcaa caacaacatc 1140
   ggcttcttca actcgggtga tggaaacatc ggcttcttca actcgggtga cggcaacacg 1200
   ggtttcggga acgccggaaa tatcaacacc ggtttctgga acgcaggcaa tttaaacacg 1260
   ggcttcggga gtgccggcaa cggaaacgtc ggtatcttcg acggcgggaa ctcaaactcg 1320
   ggcagcttca acgtgggctt tcagaacacc ggcttcggaa attcgggtgc tggaaacacc 1380
   ggcttcttca atgcgggtga ctcgaacacc ggtttcgcga acgcaggtaa cgtcaacacc 1440
   ggtttcttta acggtggaga tatcaacacc ggtggtttca atggcggcaa cgtcaacacc 1500
   ggttttggca gcgcgctcac ccaagcaggt gccaactcgg gcttcgggaa cctcggtacc 1560
   ggcaactcgg gttgggggaa cagtgacccc tcgggcaccg gcaactccgg gttcttcaac 1620
   acaggcaacg gtaattcggg cttctccaac gccggcccag ccatgcttcc tggcttcaac 1680
   tccgggtttg caaacattgg ctctttcaat gcaggaattg caaactcggg taacaacctc 1740
   gccggtatct ccaactcggg tgacgacagt tcgggtgcgg taaattcggg tagccagaac 1800
   tccggtgctt tcaatgcggg tgtaggactt tcgggattct tcaggtag 1848
<210> 33
   <211> 1773
   <212> DNA
   <213> Mycobacterium tuberculosis
<400> 33
   atgaattatt cggtgttgcc gccggagatt aattcgttgc ggatgtttac cggtgcgggg      60
   tctgcgccga tgcttgcggc atcggtggct tgggatcgtt tggccgcgga gttggcggtg     120
   gcggcgtcct cgtttgggtc ggtgacttcg gggttggcgg gtcagtcctg gcagggtgcg     180
   gcggcggcgg cgatggccgc ggcggcggcg ccgtatgcgg ggtggttggc tgctgcggcg     240
   gcgcgggccg ctggcgcgtc ggctcaggcc aaggcggtgg ccagtgcgtt tgaggcggcg      300
   cgggcggcga cggtgcatcc gatgctggtg gcggccaacc gtaatgcgtt tgtgcagttg      360
   gtgttgtcga atctgtttgg gcagaatgcg ccggcgatcg cggccgctga ggcgatgtat      420
   gaacagatgt gggccgccga tgtggccgcg atggtgggct atcacggcgg ggcatcggcg      480
   gccgcggcgc agctgtcgtc gtggtcaatt ggtctgcagc aggcgttgcc agctgcgcca      540
   tcggcgctgg ccgccgcgat cggcctcggc aacatcggcg tcgggaacct gggcggcggg      600
   aacaccggtg actacaatct gggcagcgga aattccggca acgccaacgt aggtagcgga      660
   aactccggca acgccaatgt gggcagcgga aatgacggtg ccacgaattt gggcagcgga      720
   aatatcggca acaccaatct cggcagcgga aacgttggca atgtcaatct gggcagcgga      780
   aaccgaggct ttggaaacct cggcaacgga aactttggca gtgggaacct gggcagtgga      840
   aacaccggaa gtaccaactt cggcggcgga aatctcggtt ccttcaactt gggcagtgga      900
   aacatcggct cctccaacat cggtttcgga aacaacggcg acaataacct cggcctcggg      960
   aacaatggca acaacaacat cggttttggg ctcaccggcg acaacttggt gggcattggc     1020
   gcgctgaact cgggcatcgg gaatctaggt ttcgggaact cgggtaacaa caacatcggt     1080
   ttcttcaact ctggcaacaa caacgtgggc ttcttcaatt cgggcaacaa caacttcggc     1140
   tttggaaacg cgggcgacat caacacgggc ttcggaaacg ccggcgacac caacacgggc     1200
   ttcggaaacg ccggcttctt caatatgggc atcgggaacg cgggcaacga agacatgggc     1260
   gtcgggaacg gcggttcctt taacgtgggc gttggcaatg cgggcaacca aagtgtgggc     1320
   tttggcaacg cgggcaccct aaacgtgggc ttcgcaaacg cgggcagtat caatacggga     1380
   ttcgcgaact cgggcagcat caatacgggc ggtttcgact cgggcgaccg gaacaccggg     1440
   tttggaagct cggtcgacca atccgtttcg agctcgggct tcggcaacac cggcatgaat     1500
   tcctcaggct tctttaacac gggcaatgtt tcggctggct atgggaacaa cggtgacgtt     1560
   cagtcgggca tcaataacac caactccggc ggcttcaacg tcggcttcta taactcgggt     1620
   gccggcaccg tgggcatcgc aaactctggc ctgcagacca caggcattgc gaactcgggc     1680
   accctcaaca cgggtgtggc gaacacgggt gaccacagct cgggggcctt caatcagggc     1740
   agtgaccagt cgggcttctt cggtcagccc taa                                  1773
<210> 34
   <211> 927
   <212> DNA
   <213> Mycobacterium tuberculosis
<400> 34
   atgtcgtttg tgttcgcggc gccagaggca ctggcggcgg ccgctgcgga catggccggt 60
   atcggttcga ctcttaacgc cgccaatgtg gttgcggcgg ttcccaccac cggagtcctg 120
   gccgcagccg cggacgaggt ctcgactcag gtcgccgcgc tgctttccgc gcatgctcag 180
   gggtatcagc agctcagccg gcagatgatg acagccttcc acgaccagtt cgtgcaggcg 240
   ctgagagcaa gtgcagacgc gtatgcaacc gccgaggcca gcgccgcgca gaccatggtg 300
   aacgccgtga atgcgcccgc aagagcgttg ctggggcatc cactgattag cgccgacgcc 360
   tcgacgggtg ggggctcgaa cgcgctgagc cgggtccaaa gcatgttcct cggcactggc 420
   ggctccagtg cacttggcgg tagcgccgct gcaaatgccg ctgccagcgg tgcactgcag 480
   ctccaaccca ccggtggggc cagcggtttg tccgccgtcg gcgccctgct gccgcgcgcc 540
   ggagcggccg ccgccgcggc gctgccggct ctggccgccg agtcgatcgg caacgcaatc 600
   aagaatctct acaacgccgt cgaaccgtgg gtgcagtacg gcttcaacct caccgcatgg 660
   gcggtgggat ggctgcccta catcggcata ctggcaccgc agatcaactt cttctattac 720
   ctcggcgagc ccatcgtgca ggcagtcctg ttcaatgcga tcgacttcgt ggacgggaca 780
   gtcactttca gccaggcact aaccaatatc gaaacggcca ccgcggcatc gatcaaccaa 840
   ttcatcaaca ccgagatcaa ctggatacgc ggcttcctgc cgccgttgcc gccaatcagc 900
   ccgccgggat tcccgtcttt gccctaa 927
<210> 35
   <211> 399
   <212> DNA
   <213> Mycobacterium tuberculosis
<400> 35
   atggactacg cgttcttacc accggagatc aactccgcgc gtatgtacag cggtcccgga 60
   ccgaattcaa tgttggttgc cgcggccagc tgggatgcgc tggccgcgga gttagcatcc 120
   gcagcagaga actacggctc ggtgattgcg cgtctgaccg gtatgcactg gtggggcccg 180
   gcgtccacgt cgatgctggc catgtcggct ccatacgtgg aatggctgga gcggaccgcc 240
   gcgcagacca agcagaccgc tacccaagcc agagcggcgg cggcggcatt cgagcaggct 300
   catgcgatga cggtgccccc agcgttggtc acaggcatcc ggggtgccat cgtcgtcgaa 360
   acggccagtg ccagcaacac cgctggcact ccaccttga 399
<210> 36
   <211> 2718
   <212> DNA
   <213> Mycobacterium tuberculosis
<400> 36
   ttgtcggcgt cagtgtctgc cacgacggct catcatggct tgccagcaca tgaagtggtg 60
   ctgctgctgg agagcgatcc atatcacggg ctgtccgacg gcgaggccgc ccaacgacta 120
   gaacgcttcg ggcccaacac cttggcggtg gtaacgcgcg ctagcttgct ggcccgcatc 180
   ctgcggcagt ttcatcaccc gctgatctac gttctgctcg ttgccgggac gatcaccgcc 240
   ggtcttaagg aattcgttga cgccgcagtg atcttcggtg tggtggtgat caatgcgatc 300
   gtgggtttca ttcaagaatc caaggcagag gccgcactgc agggcctgcg ctccatggtg 360
   cacacccacg ccaaggtggt gcgcgagggt cacgagcaca caatgccatc cgaagagctg 420
   gttcccggtg accttgtgct gttagcggcc ggtgacaagg ttcccgccga tttgcggctg 480
   gtgcgacaga ccggattgag cgtgaacgag tcagcactta ccggcgagtc gacgccggtt 540
   cacaaggacg aggtggcgtt gccggagggc acaccggtcg ctgatcgtcg caatatcgcg 600
   tattccggca cattggtaac cgcgggccat ggcgccggga tcgtcgtcgc gaccggcgcc 660
   gaaaccgaac tcggtgagat tcatcggctc gttggggccg ccgaggttgt cgccacaccg 720
   ctgaccgcga agctggcgtg gttcagcaag tttctgacca tcgccatcct gggtctggca 780
   gcgctcacgt tcggcgtggg tttgctgcgc cggcaagatg ccgtcgaaac gttcaccgct 840
   gcgatcgcgc tggcggtcgg ggcaattccc gaaggtctgc ccaccgccgt gaccatcacc 900
   ttggccatcg gcatggcccg gatggccaag cgccgcgcgg tcattcgacg tctacccgcg 960
   gtggaaacgc tgggcagcac cacggtcatc tgcgccgaca agaccggaac gctgaccgag 1020
   aatcagatga cggtccagtc gatctggaca ccccacggtg agatccgggc gaccggaacg 1080
   ggctatgcac ccgacgtcct cctgtgcgac accgacgacg cgccggttcc ggtgaatgcc 1140
   aatgcggccc ttcgctggtc gctgctggcc ggtgcctgca gcaacgacgc cgcactggtt 1200
   cgcgacggca cacgctggca gatcgtcggc gatcccaccg agggcgcgat gctcgtcgtg 1260
   gccgccaagg ccggcttcaa cccggagcgg ctggcgacaa ctctgccgca agtggcagcc 1320
   ataccgttca gttccgagcg gcaatacatg gccaccctgc atcgcgacgg gacggatcat 1380
   gtggtgctgg ccaagggtgc tgtggagcgc atgctcgacc tgtgcggcac cgagatgggc 1440
   gccgacggcg cattgcggcc gctggaccgc gccaccgtgt tgcgtgccac cgaaatgttg 1500
   acttcccggg ggttgcgggt gctggcaacc gggatgggtg ccggcgccgg cactcccgac 1560
   gacttcgacg aaaacgtgat accaggttcg ctggcgctga ccggcctgca agcgatgagc 1620
   gatccaccac gagcggccgc ggcatcggcg gtggcggcct gccacagtgc cggcattgcg 1680
   gtaaaaatga ttaccggtga ccacgcgggc accgccacgg cgatcgcaac cgaggtgggg 1740
   ttgctcgaca acactgaacc ggcggcaggc tcggtcctga cgggtgccga gctggccgcg 1800
   ctgagcgcag accagtaccc ggaggccgtg gatacagcca gcgtgtttgc cagggtctct 1860
   cccgagcaga agctgcggtt ggtgcaagca ttgcaggcca gggggcacgt cgtcgcgatg 1920
   accggcgacg gcgtcaacga cgccccggcc ttgcgtcagg ccaacattgg cgtcgcgatg 1980
   ggccgcggtg gcaccgaggt cgccaaggat gccgccgaca tggtgttgac cgacgacgac 2040
   ttcgccacca tcgaagccgc ggtcgaggaa ggccgcggcg tattcgacaa tctgaccaag 2100
   ttcatcacct ggacgctgcc caccaacctc ggtgagggcc tagtgatctt ggccgccatc 2160
   gctgttggcg tcgccttgcc gattctgccc acccaaattc tgtggatcaa catgaccaca 2220
   gcgatcgcgc tcggactcat gctcgcgttc gagcccaagg aggccggaat catgacccgg 2280
   ccaccgcgcg accccgacca accgctgctg accggctggc ttgtcaggcg gactcttctg     2340
   gtttccacct tgctcgtcgc cagcgcgtgg tggctgtttg catgggagct cgacaatggc     2400
   gcgggcctgc atgaggcgcg cacggcggcg ctgaacctgt tcgtcgtcgt cgaggcgttc     2460
   tatctgttca gctgccggtc gctgacccga tcggcctggc ggctcggcat gttcgccaac     2520
   cgctggatca tcctcggcgt cagtgcgcag gccatcgcgc aattcgcgat cacatatcta     2580
   cccgcgatga atatggtgtt cgacaccgcg ccaatcgata tcggggtgtg ggtgcgcata     2640
   ttcgctgtcg cgaccgcaat cacgattgtg gtggccaccg acacgctgct gccgagaata     2700
   cgggcgcaac cgccatga                                                   2718

## Description

### CROSS REFERENCE TO RELATED APPLICATION

This claims the benefit of U.S. Provisional Application No. 61/263,206, filed November 20, 2009.

### FIELD

This application relates to the field of immunology, more specifically to methods for the production of an immune response to tuberculosis antigens in a subject.

### ACKNOWLEDGEMENT OF GOVERNMENT SUPPORT

This invention was made with government support under Grant No. HSSN266200400081C awarded by the National Institutes of Health. The government has certain rights in the invention.

### BACKGROUND

Mycobacterium is a genus of aerobic intracellular bacterial organisms that, upon infection of a host, survive within endosomal compartments of monocytes and macrophages. Human mycobacterial diseases include tuberculosis (caused *by M. tuberculosis*)*,* leprosy (caused by *M. leprae*)*,* Bairnsdale ulcers (caused *by M. ulcerans*)*,* and various infections caused by *M. marinum, M. kansasii, M. scrofulaceum, M. szulgai, M. xenopi, M. fortuitum, M. chelonae, M. haemophilum and M. intracellular* (see Wolinsky, Chapter 37 in Microbiology: Including Immunology and Molecular Genetics, 3rd Ed., Harper & Row, Philadelphia, 1980).

One third of the world's population harbors *M. tuberculosis.* and is at risk for developing tuberculosis (TB). In immunocompromised patients, tuberculosis is increasing at a nearly logarithmic rate, and multidrug resistant strains are appearing. In addition, mycobacterial strains which were previously considered to be nonpathogenic strains (e.g., *M. avium*) have now become major killers of immunosuppressed AIDS patients. Moreover, current mycobacterial vaccines are either inadequate (such as the BCG vaccine for *M. tuberculosis*) or unavailable (such as for *M. leprae)* (Kaufmann, Microbiol. Sci. 4:324-328, 1987; U.S. Congress, Office of Technology Assessment, The Continuing Challenge of Tuberculosis, pp. 62-67, OTA-H-574, U.S. Government Printing Office, Washington, D.C., 1993).

Inhibiting the spread of tuberculosis requires effective vaccination and accurate, early diagnosis of the disease. Currently, vaccination with live bacteria is the most efficient method for inducing protective immunity. The most common mycobacterium employed for this purpose is Bacillus Calmette-Guerin (BCG), an avirulent strain of *Mycobacterium bovis.* However, the safety and efficacy of BCG is a source of controversy and some countries, such as the United States, do not vaccinate the general public.

*Mycobacterium tuberculosis* (Mtb)-specific CD4⁺ and CD⁸⁺ T cells are critical for the effective control of Mtb infection. In the mouse model, passive transfer of CD4⁺ T cells to sublethally irradiated animals renders them less susceptible to Mtb infection (Orme, J. Immunol. 140:3589-3593, 1988). Mice in which the gene(s) for CD4 or for MHC Class II molecules are disrupted, as well as wild-type mice depleted of CD4⁺ T cells, demonstrate increased susceptibility to Mtb infection (Flory et al., J. Leukoc. Biol. 51:225-229, 1992). In humans, human immunodeficiency virus-infected individuals are exquisitely susceptible to developing TB after exposure to Mtb, supporting an essential role for CD4⁺ T cells (Hirsch et al., J. Infect. Dis. 180:2069-2073, 1999). CD8⁺ T cells are also important for effective T cell immunity (see Lazarevic and Flynn, Am. J. Respir. Crit. Care Med. 166:1116-1121, 2002). Inhumans, Mtb-specific CD8⁺ T cells have been identified in Mtb-infected individuals and include CD8⁺ T cells that are both classically HLA-Ia restricted (see, for example, Lewinsohn et al., J. Immunol. 165:925-930, 2000) and nonclassically restricted by the HLA-Ib molecule HLA-E (Lewinsohn et al., J. Exp. Med. 187:1633-1640, 1998). However, there are no vaccines or therapeutic strategies that effectively induce an immune response, such as a CD8 response, to Mtb.

It is also known that:
UNIPROT database accession no. A5U183 discloses the amino acid sequence of a known PPE protein from Mycobacterium tuberculosis strain ATCC 25177/H37Ra.
Brosch Roland et al. 2007 (PNAS US, vol. 104, no. 13, pp. 5596-5601) and Seki M et. al. 2009 (Vaccine, vol 27, no.11, pp. 1710-1716) disclose strains of the bacillus calmette-guerin (BCG) vaccine.
WO 2008/124647 A2 discloses the use of Mtb antigens and fragments to treat Mtb infections.

### SUMMARY

Accordingly, there is a need in the art for agents that can produce an immune response to Mtb that can be used for treatment and/or protection from an Mtb infection.

Methods for producing an immune response to *Mycobacterium tuberculosis* (Mtb) are disclosed herein. Methods for treating an Mtb infection, inhibiting an Mtb infection, or preventing an Mtb infection in a subject, are also disclosed herein. The Mtb infection can be latent or active.

In several embodiments, the methods include administering to the subject a therapeutically effective amount of a polypeptide, or a polynucleotide encoding the polypeptide, wherein the polypeptide comprises at least one of the amino acid sequences set forth as SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, or SEQ ID NO: 18. In additional embodiments, the methods include administering to the subject a therapeutically effective amount of a polypeptide comprising at least nine to twenty consecutive amino acids of at least one of the amino acid sequences set forth as SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, or SEQ ID NO: 18, wherein the nine to twenty consecutive amino acids specifically bind major histocompatibility complex (MHC) class I. In some examples, the polypeptide includes a conservative variant of the polypeptide (for example, one or more conservative amino acid substitutions). In several examples, the immune response is a protective immune response. In additional embodiments, methods are disclosed for inhibiting or preventing an infection with Mtb, or treating an infection with Mtb.

Isolated polypeptides are described herein that include nine to twenty consecutive amino acids of at least one of the amino acid sequences set forth as SEQ ID NOs: 1-18, wherein the nine to twenty consecutive amino acids specifically bind major histocompatibility complex (MHC) class I, wherein the isolated polypeptide does not include any of the full length amino acid sequences set forth as SEQ ID NOs: 1-18. Nucleic acids encoding these polypeptides, vectors including these nucleic acids, host cells including these nucleic acids, and immunogenic compositions including these polypeptides, nucleic acids and/or host cells are also disclosed. Pharmaceutical compositions including a therapeutically effective amount of these polypeptides, nucleic acids, and/or host cells are also described.

The foregoing and other features will become more apparent from the following detailed description, which proceeds with reference to the accompanying figures.

### BRIEF DESCRIPTION OF THE DRAWINGS

**FIG. 1A** is a pair of bar graphs showing percent positive samples in the ELISPOT assay for the indicated antigens in individuals with latent or active TB.
**FIG. 1B** is a pair of graphs showing spot forming units (SFU) for each antigen in the ELISPOT assay. The antigens are listed in Table 2.
**FIG. 2A** is a bar graph showing percent positive samples for five selected antigens by CD8 ELISPOT assay in individuals with latent or active TB.
**FIG. 2B** is a graph showing SFU/250,000 CD4/CD56 depleted PBMC by ELISPOT assay. For each antigen, "L" indicates individuals with latent TB infection and "A" indicates individuals with active TB infection.
**FIG. 3** is a graph showing SFU for each antigen in the ELISPOT assay.
**FIG. 4** is a graph showing SFU for peptides covering amino acids 137-151 of Rv3136.

### SEQUENCE LISTING

The nucleic acid and amino acid sequences listed herein or in the accompanying sequence listing are shown using standard letter abbreviations for nucleotide bases, and one letter code for amino acids. Only one strand of each nucleic acid sequence is shown, but the complementary strand is understood as included by any reference to the displayed strand.

The Sequence Listing is submitted as an ASCII text file in the form of the file named Sequence_Listing.txt, which was created on October 29, 2010, and is 117 kilobytes.
SEQ ID NOs: 1-18 are the amino acid sequences of Mtb polypeptides.
SEQ ID NOs: 19-36 are the nucleic acid sequences of polynucleotides encoding the Mtb polypeptides.

### DETAILED DESCRIPTION

Methods for producing an immune response to *Mycobacterium tuberculosis.* (Mtb) are disclosed herein. In several examples, the immune response is a protective immune response. In additional embodiments, methods are disclosed for inhibiting an infection with Mtb, or treating an infection with Mtb. Pharmaceutical compositions for the inhibition, prevention and/or treatment of tuberculosis are also disclosed.

### I. Abbreviations

- **APC:**: antigen presenting cell
- **BCG:**: Bacillus Calmette-Guerin
- **DC:**: dendritic cell
- **HLA:**: human leukocyte antigen
- **IFN-γ:**: interferon-γ
- **MHC:**: major histocompatibility complex
- **Mtb:**: *Mycobacterium tuberculosis*
- **TB:**: tuberculosis

### II. Terms

Unless otherwise noted, technical terms are used according to conventional usage. Definitions of common terms in molecular biology may be found in Benjamin Lewin, Genes V, published by Oxford University Press, 1994 (ISBN 0-19-854287-9); Kendrew et al. (eds.), The Encyclopedic of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

In order to facilitate review of the various embodiments of this disclosure, the following explanations of specific terms are provided:
**Adjuvant:** A vehicle used to enhance antigenicity. Adjuvants include a suspension of minerals (alum, aluminum hydroxide, or phosphate) on which antigen is adsorbed; or water-in-oil emulsion in which antigen solution is emulsified in mineral oil (Freund incomplete adjuvant), sometimes with the inclusion of killed mycobacteria (Freund's complete adjuvant) to further enhance antigenicity (inhibits degradation of antigen and/or causes influx of macrophages). Immunostimulatory oligonucleotides (such as those including a CpG motif) can also be used as adjuvants (for example see U.S. Patent No. 6,194,388; U.S. Patent No. 6,207,646; U.S. Patent No. 6,214,806; U.S. Patent No. 6,218,371; U.S. Patent No. 6,239,116; U.S. Patent No. 6,339,068; U.S. Patent No. 6,406,705; and U.S. Patent No. 6,429,199). Adjuvants include biological molecules (a "biological adjuvant"), such as costimulatory molecules. Exemplary adjuvants include IL-2, RANTES, GM-CSF, TNF-α, IFN-γ, G-CSF, LFA-3, CD72, B7-1, B7-2, OX-40L and 4-1 BBL
**Amplification:** Use of a technique that increases the number of copies of a nucleic acid molecule (*e.g.,* a DNA or RNA molecule) in a specimen. An example of amplification is the polymerase chain reaction, in which a biological sample collected from a subject is contacted with a pair of oligonucleotide primers, under conditions that allow for the hybridization of the primers to a nucleic acid template in the sample. The primers are extended under suitable conditions, dissociated from the template, and then re-annealed, extended, and dissociated to amplify the number of copies of the nucleic acid. The product of amplification can be characterized by electrophoresis, restriction endonuclease cleavage patterns, oligonucleotide hybridization or ligation, and/or nucleic acid sequencing using standard techniques. Other examples of amplification include strand displacement amplification, as disclosed in U.S. Patent No. 5,744,311; transcription-free isothermal amplification, as disclosed in U.S. Patent No. 6,033,881; repair chain reaction amplification, as disclosed in WO 90/01069; ligase chain reaction amplification, as disclosed in EP-A-320 308; gap filling ligase chain reaction amplification, as disclosed in U.S. Patent No. 5,427,930; and NASBA™ RNA transcription-free amplification, as disclosed in U.S. Patent No. 6,025,134.
**Animal:** Living multi-cellular vertebrate organisms, a category that includes, for example, mammals and birds. The term mammal includes both human and non-human mammals. Similarly, the term "subject" includes both human and veterinary subjects.
**Antibody:** Immunoglobulin molecules and immunologically active portions of immunoglobulin molecules, i.e., molecules that contain an antigen binding site that specifically binds (immunoreacts with) an antigen, such as an Mtb polypeptide.

A naturally occurring antibody (e.g., IgG, IgM, IgD) includes four polypeptide chains, two heavy (H) chains and two light (L) chains interconnected by disulfide bonds. However, it has been shown that the antigen-binding function of an antibody can be performed by fragments of a naturally occurring antibody. Thus, these antigen-binding fragments are also intended to be designated by the term "antibody." Specific, non-limiting examples of binding fragments encompassed within the term antibody include (i) a Fab fragment consisting of the V_{L}, V_{H}, C_{L} and C_{H1} domains; (ii) an F_{d} fragment consisting of the V_{H} and C_{H1} domains; (iii) an Fv fragment consisting of the V_{L} and V_{H} domains of a single arm of an antibody, (iv) a dAb fragment (Ward et al., Nature 341:544-546, 1989) which consists of a V_{H} domain; (v) an isolated complementarity determining region (CDR); and (vi) a F(ab')₂ fragment, a bivalent fragment comprising two Fab fragments linked by a disulfide bridge at the hinge region.

Immunoglobulins and certain variants thereof are known and many have been prepared in recombinant cell culture *(e.g.,* see U.S. Patent Nos. 4,745,055 and 4,444,487; WO 88/03565; EP 256,654; EP 120,694; EP 125,023; Falkner et al., Nature 298:286, 1982; Morrison, J. Immunol. 123:793, 1979; Morrison et al., Ann. Rev. Immunol. 2:239, 1984).

**Antigen:** A compound, composition, or substance that can stimulate the production of antibodies or a T cell response in an animal, including compositions that are injected or absorbed into an animal. An antigen reacts with the products of specific humoral or cellular immunity, including those induced by heterologous immunogens. The term "antigen" includes all related antigenic epitopes. "Epitope" or "antigenic determinant" refers to a site on an antigen to which B and/or T cells respond. In one embodiment, T cells respond to the epitope, when the epitope is presented in conjunction with an MHC molecule. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5, about 9, or about 8-10 amino acids in a unique spatial conformation. Methods of determining spatial conformation of epitopes include, for example, x-ray crystallography and 2-dimensional nuclear magnetic resonance.

An antigen can be a tissue-specific antigen, or a disease-specific antigen. These terms are not exclusive, as a tissue-specific antigen can also be a disease-specific antigen. A tissue-specific antigen is expressed in a limited number of tissues, such as a single tissue. A tissue-specific antigen may be expressed by more than one tissue, such as, but not limited to, an antigen that is expressed in more than one reproductive tissue, such as in both prostate and uterine tissue. A disease-specific antigen is expressed coincidentally with a disease process. Specific non-limiting examples of a disease-specific antigen are an antigen whose expression correlates with, or is predictive of, tuberculosis. A disease-specific antigen can be an antigen recognized by T cells or B cells.

**Antigen presenting cell (APC):** A cell that can present an antigen to T cell, such that the T cells are activated. Dendritic cells (DCs) are the principle APCs involved in primary immune responses. Their major function is to obtain antigen in tissues, migrate to lymphoid organs and present the antigen in order to activate T cells.

When an appropriate maturational cue is received, DCs are signaled to undergo rapid morphological and physiological changes that facilitate the initiation and development of immune responses. Among these are the up-regulation of molecules involved in antigen presentation; production of pro-inflammatory cytokines, including IL-12, key to the generation of Th1 responses; and secretion of chemokines that help to drive differentiation, expansion, and migration of surrounding naive Th cells. Collectively, these up-regulated molecules facilitate the ability of DCs to coordinate the activation and effector function of other surrounding lymphocytes that ultimately provide protection for the host.

**cDNA (complementary DNA):** A piece of DNA lacking internal, noncoding segments (introns) and regulatory sequences that determine transcription. cDNA is synthesized in the laboratory by reverse transcription from messenger RNA extracted from cells.

**CD4:** Cluster of differentiation factor 4, a T cell surface protein that mediates interaction with the MHC Class II molecule. CD4 also serves as the primary receptor site for HIV on T cells during HIV infection. Cells that express CD4 are often helper T cells.

**CD8:** Cluster of differentiation factor 8, a T cell surface protein that mediates interaction with the MHC Class I molecule. Cells that express CD8 are often cytotoxic T cells. "CD8⁺ T cell mediated immunity" is an immune response implemented by presentation of antigens to CD8⁺ T cells.

**Conservative variants:** A substitution of an amino acid residue for another amino acid residue having similar biochemical properties. "Conservative" amino acid substitutions are those substitutions that do not substantially affect or decrease an activity or antigenicity of the *Mycobacterium* polypeptide. A peptide can include one or more amino acid substitutions, for example 1-10 conservative substitutions, 2-5 conservative substitutions, 4-9 conservative substitutions, such as 1, 2, 5 or 10 conservative substitutions. Specific, non-limiting examples of a conservative substitution include the following examples (Table 1).

**Table 1. Exemplary conservative amino acid substitutions**

| **Original Amino Acid** | **Conservative Substitutions** |
|---|---|
| Ala | Ser |
| Arg | Lys |
| Asn | Gln, His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

The term conservative variation also includes the use of a substituted amino acid in place of an unsubstituted parent amino acid, provided that antibodies raised to the substituted polypeptide also immunoreact with the unsubstituted polypeptide, or that an immune response can be generated against the substituted polypeptide that is similar to the immune response against the unsubstituted polypeptide, such as a *Mycobacterium* antigen. Thus, in one embodiment, non-conservative substitutions are those that reduce an activity or antigenicity.

**Consists Essentially Of/Consists Of:** With regard to a polypeptide, a polypeptide consists essentially of a specified amino acid sequence if it does not include any additional amino acid residues. However, the polypeptide can include additional non-peptide components, such as labels (for example, fluorescent, radioactive, or solid particle labels), sugars or lipids. A polypeptide that consists of a specified amino acid sequence does not include any additional amino acid residues, nor does it include additional non-peptide components, such as lipids, sugars or labels.

**Contacting:** The process of incubating one agent in the presence of another. Thus, when a cell is contacted with an agent, the cell is incubated with the agent for a sufficient period of time for the agent and the cell to interact.

**Costimulatory molecule:** Although engagement of the T cell receptor with peptide-MHC delivers one signal to the T cell, this signal alone can be insufficient to activate the T cell. Costimulatory molecules are molecules that, when bound to their ligand, deliver a second signal required for the T cell to become activated. The most well-known costimulatory molecule on the T cell is CD28, which binds to either B7-1 (also called CD80) or B7-2 (also known as CD86). An additional costimulatory molecule is B7-3. Accessory molecules that also provide a second signal for the activation of T cells include intracellular adhesion molecule (ICAM-1 and ICAM-2), leukocyte function associated antigen (LFA-1, LFA-2 and LFA-3). Integrins and tumor necrosis factor (TNF) superfamily members can also serve as costimulatory molecules.

**Cytokine:** Proteins made by cells that affect the behavior of other cells, such as lymphocytes. In one embodiment, a cytokine is a chemokine, a molecule that affects cellular trafficking. Specific, non-limiting examples of cytokines include the interleukins (IL-2, IL-4, IL-6, IL-10, IL-21, etc.), and interferon (IFN)-γ.

**Degenerate variant:** A polynucleotide encoding an epitope of an Mtb polypeptide that includes a sequence that is degenerate as a result of the genetic code. There are 20 natural amino acids, most of which are specified by more than one codon. Therefore, all degenerate nucleotide sequences are included in this disclosure as long as the amino acid sequence of the Mtb polypeptide encoded by the nucleotide sequence is unchanged.

**Dendritic cell (DC):** Dendritic cells are the principle APCs involved in primary immune responses. DCs include plasmacytoid dendritic cells and myeloid dendritic cells. Their major function is to obtain antigen in tissues, migrate to lymphoid organs and present the antigen in order to activate T cells. Immature DCs originate in the bone marrow and reside in the periphery as immature cells.

**Displaying:** The process of localizing a peptide:antigen complex, or a peptide, on the outer surface of a cell where the peptide:antigen complex or peptide is accessible to a second cell, molecules displayed by a second cell, or soluble factors. A peptide, or a peptide:antigen complex, is "displayed" by a cell when it is present on the outer surface of the cell and is accessible to a second cell, to molecules displayed by the second cell, or to soluble factors.

**Epitope:** An antigenic determinant. These are particular chemical groups or peptide sequences on a molecule that are antigenic, e.g., that elicit a specific immune response. An antibody specifically binds a particular antigenic epitope on a polypeptide, such a *Mycobacterium* polypeptide.

**Expression Control Sequences:** Nucleic acid sequences that regulate the expression of a heterologous nucleic acid sequence to which it is operatively linked. Expression control sequences are operatively linked to a nucleic acid sequence when the expression control sequences control and regulate the transcription and, as appropriate, translation of the nucleic acid sequence. Thus expression control sequences can include appropriate promoters, enhancers, transcription terminators, a start codon (*e.g.,* ATG) in front of a protein-encoding gene, splicing signal for introns, maintenance of the correct reading frame of that gene to permit proper translation of mRNA, and stop codons. The term "control sequences" is intended to include, at a minimum, components whose presence can influence expression, and can also include additional components whose presence is advantageous, for example, leader sequences and fusion partner sequences. Expression control sequences can include a promoter.

A promoter is a minimal sequence sufficient to direct transcription. Also included are those promoter elements which are sufficient to render promoter-dependent gene expression controllable for cell-type specific, tissue-specific, or inducible by external signals or agents; such elements may be located in the 5' or 3' regions of the gene. Both constitutive and inducible promoters, are included (see *e.g.,* Bitter et al., Meth. Enzymol. 153:516-544, 1987). For example, when cloning in bacterial systems, inducible promoters such as pL of bacteriophage lambda , plac, ptrp, ptac (ptrp-lac hybrid promoter) and the like may be used. In one embodiment, when cloning in mammalian cell systems, promoters derived from the genome of mammalian cells (*e.g.,* metallothionein promoter) or from mammalian viruses (*e.g.,* the retrovirus long terminal repeat; the adenovirus late promoter; the vaccinia virus 7.5K promoter) can be used. Promoters produced by recombinant DNA or synthetic techniques may also be used to provide for transcription of the nucleic acid sequences. In one embodiment, the promoter is a cytomegalovirus promoter.

**Functionally Equivalent:** Sequence alterations, such as in an epitope of an antigen, which yield the same results as described herein. Such sequence alterations can include, but are not limited to, conservative substitutions, deletions, mutations, frameshifts, and insertions.

**Heterologous:** Originating from separate genetic sources or species. A polypeptide that is heterologous to an Mtb polypeptide originates from a nucleic acid that does not encode the Mtb polypeptide or another Mtb polypeptide. In one specific, non-limiting example, a polypeptide comprising nine consecutive amino acids from an Mtb polypeptide, or at most 20 consecutive amino acids from the Mtb polypeptide, and a heterologous amino acid sequence includes a β-galactosidase, a maltose binding protein, and albumin, hepatitis B surface antigen, or an immunoglobulin amino acid sequence. Generally, an antibody that specifically binds to a protein of interest will not specifically bind to a heterologous protein.

**Host cells:** Cells in which a vector can be propagated and its DNA expressed. The cell may be prokaryotic or eukaryotic. The cell can be mammalian, such as a human cell. The term also includes any progeny of the subject host cell. It is understood that all progeny may not be identical to the parental cell since there may be mutations that occur during replication. However, such progeny are included when the term "host cell" is used.

**Human Leukocyte Antigen (HLA):** A genetic designation of the human major histocompatibility complex (MHC). Individual loci are designated by uppercase letters, as in HLA-E, and alleles are designated by numbers, as in HLA-A*0201. The three main MHC class I genes are called HLA-A, HLA-B, and HLA-C. However, there are many genes that encode β2 microglobulin-associated cell surface molecules that are linked to the MHC class I genes. The expression of these genes is variable, both in the tissue distribution and the amount expressed on cells; these genes have been termed the MHC class IB genes.

**Immune response:** A response of a cell of the immune system, such as a B cell, natural killer cell, or a T cell, to a stimulus. In one embodiment, the response is specific for a particular antigen (an "antigen-specific response"). In one embodiment, an immune response is a T cell response, such as a Th1, Th2, or Th3 response. In another embodiment, an immune response is a response of a suppressor T cell.

**Immunogenic composition:** A composition comprising an effective amount of an immunogenic Mtb polypeptide or a nucleic acid encoding the immunogenic Mtb polypeptide that induces a measurable T response against Mtb, such as a CD8⁺ T cell response, or induces a measurable B cell response (such as production of antibodies that specifically bind an Mtb polypeptide). For *in vitro* use, the immunogenic composition can consist of the isolated nucleic acid, vector including the nucleic acid/or immunogenic peptide. For *in vivo* use, the immunogenic composition will typically comprise the nucleic acid, vector including the nucleic acid, and/or immunogenic polypeptide in pharmaceutically acceptable carriers and/or other agents. An immunogenic composition can optionally include an adjuvant, a costimulatory molecule, or a nucleic acid encoding a costimulatory molecule. An Mtb polypeptide, or nucleic acid encoding the polypeptide, can be readily tested for its ability to induce a CD8⁺ T cell response.

**Immunogenic peptide:** A peptide which comprises an allele-specific motif or other sequence such that the peptide will bind an MHC molecule and induce a T cell response, such as a CD8⁺ or CD4⁺ T cell response, or a B cell response (such as antibody production) against the antigen from which the immunogenic peptide is derived.

In one embodiment, immunogenic peptides are identified using sequence motifs or other methods, such as neural net or polynomial determinations, known in the art. Typically, algorithms are used to determine the "binding threshold" of peptides to select those with scores that give them a high probability of binding at a certain affinity and will be immunogenic. The algorithms are based either on the effects on MHC binding of a particular amino acid at a particular position, the effects on antibody binding of a particular amino acid at a particular position, or the effects on binding of a particular substitution in a motif-containing peptide. Within the context of an immunogenic peptide, a "conserved residue" is one which appears in a significantly higher frequency than would be expected by random distribution at a particular position in a peptide. In one embodiment, a conserved residue is one where the MHC structure may provide a contact point with the immunogenic peptide.

Immunogenic peptides can also be identified by measuring their binding to a specific MHC protein and by their ability to stimulate CD4 and/or CD8 when presented in the context of the MHC protein. In one example, an immunogenic "Mtb peptide" is a series of contiguous amino acid residues from the Mtb protein generally between 9 and 20 amino acids in length, such as about 8 to 11 residues in length.

Generally, immunogenic Mtb polypeptides can be used to induce an immune response in a subject, such as a B cell response or a T cell response. In one example, an immunogenic Mtb polypeptide, when bound to a MHC Class I molecule, activates CD8⁺ T cells, such as cytotoxic T lymphocytes (CTLs) against Mtb. Induction of CTLs using synthetic peptides and CTL cytotoxicity assays are known in the art (see U.S. Patent 5,662,907). In one example, an immunogenic peptide includes an allele-specific motif or other sequence such that the peptide will bind an MHC molecule and induce a CD8⁺ response against the antigen from which the immunogenic peptide is derived. A CD8⁺ T cell that specifically recognizes an Mtb polypeptide is activated, proliferates, and/or secretes cytokines in response to that specific polypeptide, and not to other, non-related polypeptides.

**Inhibiting or treating a disease:** Inhibiting a disease, such as tuberculosis, refers to inhibiting the full development of a disease. In several examples, inhibiting a disease refers to lessening symptoms of a tuberculosis. "Treatment" refers to a therapeutic intervention that ameliorates a sign or symptom of a disease or pathological condition related to the disease, such as tuberculosis.

**Interferon gamma (IFN-γ):** IFN-γ is a dimeric protein with subunits of 146 amino acids. The protein is glycosylated at two sites, and the pI is 8.3-8.5. IFN-γ is synthesized as a precursor protein of 166 amino acids including a secretory signal sequence of 23 amino acids. Two molecular forms of the biologically active protein of 20 and 25 kDa have been described. Both of them are glycosylated at position 25. The 25 kDa form is also glycosylated at position 97. The observed differences of natural IFN-γ with respect to molecular mass and charge are due to variable glycosylation patterns. 40-60 kDa forms observed under non-denaturing conditions are dimers and tetramers of IFN-γ. The human gene has a length of approximately 6 kb. It contains four exons and maps to chromosome 12q24.1.

IFN-γ can be detected by sensitive immunoassays, such as an ELSA test that allows detection of individual cells producing IFN-γ. Minute amounts of IFN-γ can be detected indirectly by measuring IFN-induced proteins such as Mx protein. The induction of the synthesis of IP-10 has also been used to measure IFN-γ concentrations. In addition, bioassays can be used to detect IFN-γ, such as an assay that employs induction of indoleamine 2,3-dioxygenase activity in 2D9 cells. The production of IFN-γ can be used to assess T cell activation, such as activation of a T cell by a *Mycobacterium* antigen.

**Isolated:** An "isolated" biological component (such as a nucleic acid molecule, protein, or organelle) has been substantially separated or purified away from other biological components in the cell of the organism in which the component naturally occurs, such as other chromosomal and extra-chromosomal DNA and RNA, proteins, and organelles. Nucleic acids and proteins that have been "isolated" include nucleic acids and proteins purified by standard purification methods. The term also embraces nucleic acids and proteins prepared by recombinant expression in a host cell, as well as chemically synthesized nucleic acids or proteins, or fragments thereof.

**Linker sequence:** A linker sequence is an amino acid sequence that covalently links two polypeptide domains. Linker sequences can be included in the between the Mtb epitopes disclosed herein to provide rotational freedom to the linked polypeptide domains and thereby to promote proper domain folding and presentation to the MHC. By way of example, in a recombinant polypeptide comprising two Mtb domains, linker sequences can be provided between them, such as a polypeptide comprising Mtb polypeptide-linker-Mtb polypeptide. Linker sequences, which are generally between 2 and 25 amino acids in length, are well known in the art and include, but are not limited to, the glycine(4)-serine spacer (GGGGS x3) described by Chaudhary et al., Nature 339:394-397, 1989.

**Lymphocytes:** A type of white blood cell that is involved in the immune defenses of the body. There are two main types of lymphocytes: B cells and T cells.

**Mycobacteria:** A genus of aerobic intracellular bacterial organisms. Upon invasion of a host, these organisms survive within endosomal compartments of monocytes and macrophages. Human mycobacterial diseases include tuberculosis (cause by *M. tuberculosis*), Leprosy (caused by *M. leprae*)*,* Bairnsdale ulcers (caused by *M. ulcerans*)*,* and other infections that can be caused by *M. marinum, M. kansasii, M. scrofulaceum, M. szulgai, M. xenopi, M. fortuitum, M. haemophilum, M. chelonei,* and *M. intracelluare. Mycobacterium* strains that were previously considered to be nonpathogenic (such as *M. avium*) are also now known to be major killers of immunosuppressed AIDS patients.

The major response to mycobacteria involves cell mediated hypersensitivity (DTH) reactions with T cells and macrophages playing major roles in the intracellular killing and walling off (or containing) of the organism (granuloma formation). A major T cell response involves CD4⁺ lymphocytes that recognize mycobacterial heat shock proteins and immunodominant antigens.

**Operably linked:** A first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, the open reading frames are aligned.

**ORF (open reading frame):** A series of nucleotide triplets (codons) coding for amino acids without any termination codons. These sequences are usually translatable into a polypeptide.

**Peptide Modifications:** *Mycobacterium* polypeptides include synthetic embodiments of peptides described herein. In addition, analogues (non-peptide organic molecules), derivatives (chemically functionalized peptide molecules obtained starting with the disclosed peptide sequences) and variants (homologs) of these proteins can be utilized in the methods described herein. Each polypeptide of the invention is comprised of a sequence of amino acids, which may be either Land/or D- amino acids, naturally occurring and otherwise.

Peptides may be modified by a variety of chemical techniques to produce derivatives having essentially the same activity as the unmodified peptides, and optionally having other desirable properties. For example, carboxylic acid groups of the protein, whether carboxyl-terminal or side chain, may be provided in the form of a salt of a pharmaceutically-acceptable cation or esterified to form a C₁-C₁₆ ester, or converted to an amide of formula NR₁R₂ wherein R₁ and R₂ are each independently H or C₁-C₁₆ alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C₁-C₁₆ alkyl or dialkyl amino or further converted to an amide.

Hydroxyl groups of the peptide side chains may be converted to C₁-C₁₆ alkoxy or to a C₁-C₁₆ ester using well-recognized techniques. Phenyl and phenolic rings of the peptide side chains may be substituted with one or more halogen atoms, such as fluorine, chlorine, bromine or iodine, or with C₁-C₁₆ alkyl, C₁-C₁₆ alkoxy, carboxylic acids and esters thereof, or amides of such carboxylic acids. Methylene groups of the peptide side chains can be extended to homologous C₂-C₄ alkylenes. Thiols can be protected with any one of a number of well-recognized protecting groups, such as acetamide groups. Those skilled in the art will also recognize methods for introducing cyclic structures into the peptides of this invention to select and provide conformational constraints to the structure that result in enhanced stability.

Peptidomimetic and organomimetic embodiments are envisioned, whereby the three-dimensional arrangement of the chemical constituents of such peptido- and organomimetics mimic the three-dimensional arrangement of the peptide backbone and component amino acid side chains, resulting in such peptido- and organomimetics of a *Mycobacterium* polypeptide having measurable or enhanced ability to generate an immune response. For computer modeling applications, a pharmacophore is an idealized, three-dimensional definition of the structural requirements for biological activity. Peptido- and organomimetics can be designed to fit each pharmacophore with current computer modeling software (using computer assisted drug design or CADD). See Walters, "Computer-Assisted Modeling of Drugs," in Klegerman & Groves, eds., 1993, Pharmaceutical Biotechnology, Interpharm Press, Buffalo Grove, IL, pp. 165-174 and Principles of Pharmacology Munson (ed.) 1995, Ch. 102, for descriptions of techniques used in CADD. Also included are mimetics prepared using such techniques.

**Pharmaceutical agent or drug:** A chemical compound or composition capable of inducing a desired therapeutic or prophylactic effect when properly administered to a subject.

**Pharmaceutically acceptable carriers:** The pharmaceutically acceptable carriers useful with the polypeptides and nucleic acids described herein are conventional. Remington's Pharmaceutical Sciences, by E. W. Martin, Mack Publishing Co., Easton, PA, 19th Edition (1995), describes compositions and formulations suitable for pharmaceutical delivery of the polypeptides or polynucleotides herein disclosed.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (*e.g*., powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Polynucleotide:** A linear nucleotide sequence, including sequences of greater than 100 nucleotide bases in length.

**Polypeptide:** Any chain of amino acids, regardless of length or post-translational modification (e.g., glycosylation or phosphorylation). A "peptide" is a chain of amino acids that is less than 100 amino acids in length. In one embodiment, a "peptide" is a portion of a polypeptide, such as about 8-11, 9-12, or about 10, 20, 30, 40, 50, or 100 contiguous amino acids of a polypeptide that is greater than 100 amino acids in length.

**Probes and primers:** Nucleic acid probes and primers may readily be prepared based on the nucleic acids provided by this invention. A probe comprises an isolated nucleic acid attached to a detectable label or reporter molecule. Typical labels include radioactive isotopes, ligands, chemiluminescent agents, and enzymes. Methods for labeling and guidance in the choice of labels appropriate for various purposes are discussed, e.g., in Sambrook *et al.* (1989) and Ausubel *et al.* (1987).

Primers are short nucleic acids, preferably DNA oligonucleotides 15 nucleotides or more in length. Primers may be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, *e.g*., by the polymerase chain reaction (PCR) or other nucleic-acid amplification methods known in the art.

Methods for preparing and using probes and primers are described, for example, in Molecular Cloning: A Laboratory Manual, 2nd ed., vol. 1-3, ed. Sambrook et al., Cold Spring Harbor Laboratory Press, Cold Spring Harbor, NY, 1989, and Current Protocols in Molecular Biology, ed. Ausubel et al., Greene Publishing and Wiley-Interscience, New York, 1987 (with periodic updates). PCR primer pairs can be derived from a known sequence, for example, by using computer programs intended for that purpose such as Primer3 (Version 0.4.0, Whitehead Institute for Biomedical Research, Cambridge, MA).

**Preventing or treating a disease:** "Preventing" a disease refers to inhibiting the full development of a disease, for example in a person who is known to be at risk of infection with *M. tuberculosis* or *M. leprae.* An example of a person with a known predisposition is someone living with a person diagnosed with tuberculosis, health care professionals, or someone who has been exposed to *M. tuberculosis.* "Treatment" refers to a therapeutic intervention that ameliorates a sign or symptom of a disease or pathological condition, such as tuberculosis, after it has begun to develop.

**Promoter:** A promoter is an array of nucleic acid control sequences which direct transcription of a nucleic acid. A promoter includes necessary nucleic acid sequences near the start site of transcription, such as, in the case of a polymerase II type promoter, a TATA element. A promoter also optionally includes distal enhancer or repressor elements which can be located as much as several thousand base pairs from the start site of transcription. The promoter can be a constitutive or an inducible promoter. A specific, non-limiting example of a promoter is the HCMV IE promoter.

**Purified:** The term purified does not require absolute purity; rather, it is intended as a relative term. Thus, for example, a purified antigen preparation is one in which the antigen is more pure than the protein in its originating environment within a cell. A preparation of an antigen is typically purified such that the antigen represents at least 50% of the total protein content of the preparation. However, more highly purified preparations may be required for certain applications. For example, for such applications, preparations in which the antigen comprises at least 75% or at least 90% of the total protein content may be employed.

**Recombinant:** A recombinant nucleic acid or polypeptide is one that has a sequence that is not naturally occurring or has a sequence that is made by an artificial combination of two or more otherwise separated segments of sequence. This artificial combination is often accomplished by chemical synthesis or, more commonly, by the artificial manipulation of isolated segments of nucleic acids, *e.g.,* by genetic engineering techniques.

**Sequence identity:** The similarity between amino acid sequences is expressed in terms of the similarity between the sequences, otherwise referred to as sequence identity. Sequence identity is frequently measured in terms of percentage identity (or similarity or homology); the higher the percentage, the more similar the two sequences are. Variants of antigen polypeptides will possess a relatively high degree of sequence identity when aligned using standard methods.

Methods of alignment of sequences for comparison are well known in the art. Altschul *et al.* (1994) presents a detailed consideration of sequence alignment methods and homology calculations. The NCBI Basic Local Alignment Search Tool (BLAST) (Altschul *et al.,* 1990) is available from several sources, including the National Center for Biotechnology Information (NCBI, Bethesda, MD) and on the Internet, for use in connection with the sequence analysis programs blastp, blastn, blastx, tblastn and tblastx. It can be accessed at the NCBI website. A description of how to determine sequence identity using this program is available at the NCBI website, as are the default parameters.

Variants of antigenic polypeptides, such as a *Mycobacterium* polypeptide, are typically characterized by possession of at least 50% sequence identity counted over the full length alignment with the amino acid sequence of a native antigen sequence using the NCBI Blast 2.0, gapped blastp set to default parameters. Proteins with even greater similarity to the reference sequences will show increasing percentage identities when assessed by this method, such as at least 60%, at least 65%, at least 70%, at least 75%, at least 80%, at least 90% or at least 95% sequence identity. When less than the entire sequence is being compared for sequence identity, variants will typically possess at least 75% sequence identity over short windows of 10-20 amino acids, and may possess sequence identities of at least 85% or at least 90% or 95% depending on their similarity to the reference sequence. Methods for determining sequence identity over such short windows are described at the NCBI website.

**Therapeutically active polypeptide:** An agent, such as an epitope of Mtb that causes induction of an immune response, as measured by clinical response (for example increase in a population of immune cells, increased cytolytic activity against Mtb, or measurable reduction of a symptom of an infection). Therapeutically active molecules can also be made from nucleic acids. Examples of a nucleic acid based therapeutically active molecule is a nucleic acid sequence that encodes an Mtb epitope, wherein the nucleic acid sequence is operably linked to a control element such as a promoter.

In one embodiment, a therapeutically effective amount of an Mtb polypeptide is an amount used to generate an immune response. In several examples, "treatment" refers to a therapeutic intervention that ameliorates a sign or symptom of tuberculosis.

**Therapeutically effective amount:** A dose sufficient to inhibit advancement, or to cause regression of the disease, or which is capable of relieving symptoms caused by the disease. In one embodiment, a therapeutically effective dose is a dose sufficient to inhibit or prevent advancement or relieve symptoms of tuberculosis.

**Transduced and Transformed:** A virus or vector "transduces" a cell when it transfers nucleic acid into the cell. A cell is "transformed" by a nucleic acid transduced into the cell when the DNA becomes stably replicated by the cell, either by incorporation of the nucleic acid into the cellular genome, or by episomal replication. As used herein, the term transformation encompasses all techniques by which a nucleic acid molecule might be introduced into such a cell, including transfection with viral vectors, transformation with plasmid vectors, and introduction of naked DNA by electroporation, lipofection, and particle gun acceleration.

**Tuberculosis (TB):** A disease that is generally caused by *Mycobacterium tuberculosis* that usually infects the lungs. However, other "atypical" mycobacteria such as *M. kansasii* may produce a similar clinical and pathologic appearance of disease.

Transmission of *M. tuberculosis* occurs by the airborne route in confined areas with poor ventilation. In more than 90% of cases, following infection with *M. tuberculosis,* the immune system prevents development of disease from *M. tuberculosis,* often called, active tuberculosis. However, not all of the *M. tuberculosis* is killed, and thus tiny, hard capsules are formed. "Primary tuberculosis" is seen as disease that develops following an initial infection, usually in children. The initial focus of infection is a small subpleural granuloma accompanied by granulomatous hilar lymph node infection. Together, these make up the Ghon complex. In nearly all cases, these granulomas resolve and there is no further spread of the infection. "Secondary tuberculosis" is seen mostly in adults as a reactivation of previous infection (or reinfection), particularly when health status declines. The granulomatous inflammation is much more florid and widespread. Typically, the upper lung lobes are most affected, and cavitation can occur. Dissemination of tuberculosis outside of the lungs can lead to the appearance of a number of uncommon findings with characteristic patterns that include skeletal tuberculosis, genital tract tuberculosis, urinary tract tuberculosis, central nervous system (CNS) tuberculosis, gastrointestinal tuberculosis, adrenal tuberculosis, scrofula, and cardiac tuberculosis. "Latent" tuberculosis is an Mtb infection in an individual that can be detected by a diagnostic assay, such as, but not limited to a tuberculin skin test (TST) wherein the infection does not produce symptoms in that individual. "Active" tuberculosis is a symptomatic Mtb infection in a subject.

Microscopically, the inflammation produced with TB infection is granulomatous, with epithelioid macrophages and Langhans giant cells along with lymphocytes, plasma cells, maybe a few polymorphonuclear cells, fibroblasts with collagen, and characteristic caseous necrosis in the center. The inflammatory response is mediated by a type IV hypersensitivity reaction, and skin testing is based on this reaction. In some examples, tuberculosis can be diagnosed by a skin test, an acid fast stain, an auramine stain, or a combination thereof. The most common specimen screened is sputum, but the histologic stains can also be performed on tissues or other body fluids.

TB is a frequent complication of HIV infection. TB infection in subjects infected with a human immunodeficiency virus (HIV) can spread readily and progress rapidly to active disease. Specific symptoms of lung disease due to Mtb infection include chronic cough and spitting blood. Other symptoms of TB disease include fatigue, loss of appetite, weight loss, fever and drenching night sweats.

**Vector:** A nucleic acid molecule as introduced into a host cell, thereby producing a transformed host cell. A vector may include nucleic acid sequences that permit it to replicate in a host cell, such as an origin of replication. A vector may also include one or more selectable marker gene and other genetic elements known in the art. Vectors include plasmid vectors, including plasmids for expression in gram negative and gram positive bacterial cell. Exemplary vectors include those for expression in *E. coli* and *Salmonella.* Vectors also include viral vectors, such as, but not limited to, retrovirus, orthopox, avipox, fowlpox, capripox, suipox, adenovirus, herpes virus, alpha virus, baculovirus, Sindbis virus, vaccinia virus, and poliovirus vectors. Vectors also include vectors for expression in yeast cells

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, suitable methods and materials are described below. The term "comprises" means "includes." In case of conflict, the present specification, including explanations of terms, will control. In addition, the materials, methods, and examples are illustrative only and not intended to be limiting.

### III. Mycobacterium Polypeptides

It is disclosed herein that several Mycobacterium polypeptides can be used to induce an immune response to Mtb, such as a T cell response. In several embodiments, the polypeptide comprises or consists of the amino acid sequence set forth as:
VPHPWDTGDHERNWQGYFIPAMSVLRNRVGARTHAELRDAENDLVEARVI ELREDPNLLGDRTDLAYLRAIHRQLFQDIYVWAGDLRTVGIEKEDESFCAP GGISRPMEHVAAEIYQLDRLRAVGEGDLAGQVAYRYDYVNYAHPFREGNG RSTREFFDLLLSERGSGLDWGKTDLEELHGACHVARANSDLTGLVAMFKGI LDAEPTYDF (SEQ ID NO: 1; see also TUBERCULIST No. Rv3641c, as available on June 8, 2009, known as fic).
MDFALLPPEVNSARMYTGPGAGSLLAAAGGWDSLAAELATTAEAYGSVLS GLAALHWRGPAAESMAVTAAPYIGWLYTTAEKTQQTAIQARAAALAFEQA YAMTLPPPVVAANRIQLLALIATNFFGQNTAAIAATEAQYAEMWAQDAAA MYGYATASAAAALLTPFSPPRQTTNPAGLTAQAAAVSQATDPLSLLIETVT QALQALTIPSFIPEDFTFLDAIFAGYATVGVTQDVESFVAGTIGAESNLGLLN VGDENPAEVTPGDFGIGELVSATSPGGGVSASGAGGAASVGNTVLASVGR ANSIGQLSVPPSWAAPSTRPVSALSPAGLTTLPGTDVAEHGMPGVPGVPVA AGRASGVLPRYGVRLTVMAHPPAAG (SEQ ID NO: 2; see also TUBERCULIST No. Rv3136, as available on June 8, 2009, known as PPE51 or PPE).
MTEPRPVFAVVISAGLSAIPMVGGPLQTVFDAIEERTRHRAETTTREICESVG GADTVLSRIDKNPELEPLLSQAIEAATRTSMEAKRRLLAQAAAAALEDDQK VEPASLIVATLSQLEPVHIHALVRLAKAAKSSPDQDEIQRREVMRAASKVEP VPVLAALIQTGVAIATTTVWHGNGTGTPAEESGHILIHDVSDFGHRLLAYLR AADAGAELLILPSGGSAPTGDHPTPHPSTSR (SEQ ID NO: 3; see also TUBERCULIST No. Rv0394c, as available on June 8, 2009).
MADFLTLSPEVNSARMYAGGGPGSLSAAAAAWDELAAELWLAAASFESV CSGLADRWWQGPSSRMMAAQAARHTGWLAAAATQAEGAASQAQTMAL AYEAAFAATVHPALVAANRALVAWLAGSNVFGQNTPAIAAAEAIYEQMW AQDVVAMLNYHAVASAVGARLRPWQQLLHELPRRLGGEHSDSTNTELAN PSSTTTRITVPGASPVHAATLLPFIGRLLAARYAELNTAIGTNWFPGTTPEVV SYPATIGVLSGSLGAVDANQSIAIGQQMLHNEILAATASGQPVTVAGLSMG SMVIDRELAYLAIDPNAPPSSALTFVELAGPERGLAQTYLPVGTTIPIAGYTV GNAPESQYNTSVVYSQYDIWADPPDRPWNLLAGANALMGAAYFHDLTAY AAPQQGIEIAAVTSSLGGTTTTYMIPSPTLPLLLPLKQIGVPDWIVGGLNNVL KPLVDAGYSQYAPTAGPYFSHGNLVW (SEQ ID NO: 4; see also TUBERCULIST No. Rv3539, as available on June 8, 2009 known as PPE63 or PPE).
MTLDVPVNQGHVPPGSVACCLVGVTAVADGIAGHSLSNFGALPPEINSGRM YSGPGSGPLMAAAAAWDGLAAELSSAATGYGAAISELTNMRWWSGPASD SMVAAVLPFVGWLSTTATLAEQAAMQARAAAAAFEAAFAMTVPPPAIAA NRTLLMTLVDTNWFGQNTPAIATTESQYAEMWAQDAAAMYGYASAAAPA TVLTPFAPPPQTTNATGLVGHATAVAALRGQHSWAAAIPWSDIQKYWMMF LGALATAEGFIYDSGGLTLNALQFVGGMLWSTALAEAGAAEAAAGAGGA AGWSAWSQLGAGPVAASATLAAKIGPMSVPPGWSAPPATPQAQTVARSIP GIRSAAEAAETSVLLRGAPTPGRSRAAHMGRRYGRRLTVMADRPNVG (SEQ ID NO: 5; see also TUBERCULIST No. Rv1706c, as available on June 8, 2009, known as PPE23 or PPE).
MDFGALPPEINSARMYAGAGAGPMMAAGAAWNGLAAELGTTAASYESVI TRLTTESWMGPASMAMVAAAQPYLAWLTYTAEAAAHAGSQAMASAAAY EAAYAMTVPPEVVAANRALLAALVATNVLGINTPAIMATEALYAEMWAQ DALAMYGYAAASGAAGMLQPLSPPSQTTNPGGLAAQSAAVGSAAATAAV NQVSVADLISSLPNAVSGLASPVTSVLDSTGLSGIIADIDALLATPFVANIINS AVNTAAWYVNAAIPTAIFLANALNSGAPVAIAEGAIEAAEGAASAAAAGLA DSVTPAGLGASLGEATLVGRLSVPAAWSTAAPATTAGATALEGSGWTVAA EEAGPVTGMMPGMASAAKGTGAYAGPRYGFKPTVMPKQVVV (SEQ ID NO: 6; see also TUBERCULIST No. Rv1039c, as available on June 8, 2009, known as PPE 15 or PPE).
MAHFSVLPPEINSLRMYLGAGSAPMLQAAAAWDGLAAELGTAASSFSSVT TGLTGQAWQGPASAAMAAAAAPYAGFLTTASAQAQLAAGQAKAVASVFE AAKAAIVPPAAVAANREAFLALIRSNWLGLNAPWIAAVESLYEEYWAADV AAMTGYHAGASQAAAQLPLPAGLQQFLNTLPNLGIGNQGNANLGGGNTGS GNIGNGNKGSSNLGGGNIGNNNIGSGNRGSDNFGAGNVGTGNIGFGNQGPI DVNLLATPGQNNVGLGNIGNNNMGFGNTGDANTGGGNTGNGNIGGGNTG NNNFGFGNTGNNNIGIGLTGNNQMGINLAGLLNSGSGNIGIGNSGTNNIGLF NSGSGNIGVFNTGANTLVPGDLNNLGVGNSGNANIGFGNAGVLNTGFGNA SILNTGLGNAGELNTGFGNAGFVNTGFDNSGNVNTGNGNSGNINTGSWNA GNVNTGFGIITDSGLTNSGFGNTGTDVSGFFNTPTGPLAVDVSGFFNTASGG TVINGQTSGIGNIGVPGTLFGSVRSGLNTGLFNMGTAISGLFNLRQLLG (SEQ ID NO: 7; see also TUBERCULIST No. Rv3558, as available on June 8, 2009, known as PPE64 or PPE).
MEYLIAAQDVLVAAAADLEGIGSALAAANRAAEAPTTGLLAAGADEVSAA IASLFSGNAQAYQALSAQAAAFHQQFVRALSSAAGSYAAAEAANASPMQA VLDVVNGPTQLLLGRPLIGDGANGGPGQNGGDGGLLYGNGGNGGSSSTPG QPGGRGGAAGLIGNGGAGGAGGPGANGGAGGNGGWLYGNGGLGGNGGA ATQIGGNGGNGGHGGNAGLWGNGGAGGAGAAGAAGANGQNPVSHQVTH ATDGADGTTGPDGNGTDAGSGSNAVNPGVGGGAGGIGGDGTNLGQTDVS GGAGGDGGDGANFASGGAGGNGGAAQSGFGDAVGGNGGAGGNGGAGG GGGLGGAGGSANVANAGNSIGGNGGAGGNGGIGAPGGAGGAGGNANQD NPPGGNSTGGNGGAGGDGGVGASADVGGAGGFGGSGGRGGLLLGTGGAG GDGGVGGDGGIGAQGGSGGNGGNGGIGADGMANQDGDGGDGGNGGDG GAGGAGGVGGNGGTGGAGGLFGQSGSPGSGAAGGLGGAGGNGGAGGGG GTGFNPGAPGDPGTQGATGANGQHGLN (SEQ ID NO: 8; see also TUBERCULIST No. Rv1243c, as available on October 6, 2009; known as PE_PGRS23). MVMSLMVAPELVAAAAADLTGIGQAISAANAAAAGPTTQVLAAAGDEVS AAIAALFGTHAQEYQALSARVATFHEQFVRSLTAAGSAYATAEAANASPLQ ALEQQVLGAINAPTQLWLGRPLIGDGVHGAPGTGQPGGAGGLLWGNGGN GGSGAAGQVGGPGGAAGLFGNGGSGGSGGAGAAGGVGGSGGWLNGNGG AGGAGGTGANGGAGGNAWLFGAGGSGGAGTNGGVGGSGGFVYGNGGA GGIGGIGGIGGNGGDAGLFGNGGAGGAGAAGLPGAAGLNGGDGSDGGNG GTGGNGGRGGLLVGNGGAGGAGGVGGDGGKGGAGDPSFAVNNGAGGNG GHGGNPGVGGAGGAGGLLAGAHGAAGATPTSGGNGGDGGIGATANSPLQ AGGAGGNGGHGGLVGNGGTGGAGGAGHAGSTGATGTALQPTGGNGTNG GAGGHGGNGGNGGAQHGDGGVGGKGGAGGSGGAGGNGFDAATLGSPGA DGGMGGNGGKGGDGGKAGDGGAGAAGDVTLAVNQGAGGDGGNGGEVG VGGKGGAGGVSANPALNGSAGANGTAPTSGGNGGNGGAGATPTVAGENG GAGGNGGHGGSVGNGGAGGAGGNGVAGTGLALNGGNGGNGGIGGNGGS AAGTGGDGGKGGNGGAGANGQDFSASANGANGGQGGNGGNGGIGGKGG DAFATFAKAGNGGAGGNGGNVGVAGQGGAGGKGAIPAMKGATGADGTA PTSGGDGGNGGNGASPTVAGGNGGDGGKGGSGGNVGNGGNGGAGGNGA AGQAGTPGPTSGDSGTSGTDGGAGGNGGAGGAGGTLAGHGGNGGKGGN GGQGGIGGAGERGADGAGPNANGANGENGGSGGNGGDGGAGGNGGAGG KAQAAGYTDGATGTGGDGGNGGDGGKAGDGGAGENGLNSGAMLPGGGT VGNPGTGGNGGNGGNAGVGGTGGKAGTGSLTGLDGTDGITPNGGNGGNG GNGGKGGTAGNGSGAAGGNGGNGGSGLNGGDAGNGGNGGGALNQAGFF GTGGKGGNGGNGGAGMINGGLGGFGGAGGGGAVDVAATTGGAGGNGGA GGFASTGLGGPGGAGGPGGAGDFASGVGGVGGAGGDGGAGGVGGFGGQ GGIGGEGRTGGNGGSGGDGGGGISLGGNGGLGGNGGVSETGFGGAGGNG GYGGPGGPEGNGGLGGNGGAGGNGGVSTTGGDGGAGGKGGNGGDGGNV GLGGDAGSGGAGGNGGIGTDAGGAGGAGGAGGNGGSSKSTTTGNAGSGG AGGNGGTGLNGAGGAGGAGGNAGVAGVSFGNAVGGDGGNGGNGGHGG DGTTGGAGGKGGNGSSGAASGSGVVNVTAGHGGNGGNGGNGGNGSAGA GGQGGAGGSAGNGGHGGGATGGDGGNGGNGGNSGNSTGVAGLAGGAA GAGGNGGGTSSAAGHGGSGGSGGSGTTGGAGAAGGNGGAGAGGGSLSTG QSGGPRRQRWCRWQRRRWLGRQRRRRWCRWQRRCRRQRWRWRCRQRR LRRQWRQGRRRCRPWLHRRRGRQGRRWRQRRFQQRQRSRWQRR (SEQ ID NO: 9; see also TUBERCULIST No. Rv3345c, as available on October 6, 2009; known as PE_PGRS50).
VIQTCEVELRWRASQLTLAIATCAGVALAAAVVAGRWQLIAFAAPLLGVLC SISWQRPVPVIQVHGDPDSQRCFENEHVRVTVWVTTESVDAAVELTVSALA GMQFEALESVSRRTTTVSAVAQRWGRYPIRARVAVVARGGLLMGAGTVD AAEIVVFPLTPPQSTPLPQTELLDRLGAHLTRHVGPGVEYADIRPYVPGDQL RAVNWVVSARRGRLHVTRRLTDRAADVVVLIDMYRQPAGPATEATERVV RGAAQVVQTALRNGDRAGIVALGGNRPRWLGADIGQRQFYRVLDTVLGA GEGFENTTGTLAPRAAVPAGAVVIAFSTLLDTEFALALIDLRKRGHVVVAV DVLDSCPLQDQLDPLVVRMWALQRSAMYRDMATIGVDVLSWPADHSLQQ SMGALPNRRRRGRGRASRARLP (SEQ ID NO: 10; see also TUBERCULIST No. Rv3163c, as available on October 6, 2009;).
VNRRILTLMVALVPIVVFGVLLAVVTVPFVALGPGPTFDTLGEIDGKQVVQI VGTQTYPTSGHLNMTTVSQRDGLTLGEALALWLSGQEQLMPRDLVYPPGK SREEIENDNAADFKRSEAAAEYAALGYLKYPKAVTVASVMDPGPSVDKLQ AGDAIDAVDGTPVGNLDQFTALLKNTKPGQEVTIDFRRKNEPPGIAQITLGK NKDRDQGVLGIEVVDAPWAPFAVDFHLANVGGPSAGLMFSLAVVDKLTSG HLVGSTFVAGTGTIAVDGKVGQIGGITHKMAAARAAGATVFLVPAKNCYE ASSDSPPGLKLVKVETLSQAVDALHAMTSGSPTPSC (SEQ ID NO: 11; see also TUBERCULIST No. Rv3194c, as available on October 6, 2009;).
MSFVVTAPPVLASAASDLGGLASMISEANAMAAVRTTALAPAAADEVSAAI AALFSSYARDYQTLSVQVTAFHVQFAQTLTNAGQLYAVVDVGNGVLLKTE QQVLGVINAPTQTLVGRPLIGDGTHGAPGTGQNGGAGGILWGNGGNGGSG APGQPGGRGGDAGLFGHGGHGGVGGPGIAGAAGTAGLPGGNGANGGSGG IGGAGGAGGNGGLLFGNGGAGGQGGSGGLGGSGGTGGAGMAAGPAGGT GGIGGIGGIGGAGGVGGHGSALFGHGGINGDGGTGGMGGQGGAGGNGWA AEGITVGIGEQGGQGGDGGAGGAGGIGGSAGGIGGSQGAGGHGGDGGQG GAGGSGGVGGGGAGAGGDGGAGGIGGTGGNGSIGGAAGNGGNGGRGGA GGMATAGSDGGNGGGGGNGGVGVGSAGGAGGTGGDGGAAGAGGAPGH GYFQQPAPQGLPIGTGGTGGEGGAGGAGGDGGQGDIGFDGGRGGDGGPG GGGGAGGDGSGTFNAQANNGGDGGAGGVGGAGGTGGTGGVGADGGRG GDSGRGGDGGNAGHGGAAQFSGRGAYGGEGGSGGAGGNAGGAGTGGTA GSGGAGGFGGNGADGGNGGNGGNGGFGGINGTFGTNGAGGTGGLGTLLG GHNGNIGLNGATGGIGSTTLTNATVPLQLVNTTEPVVFISLNGGQMVPVLL DTGSTGLVMDSQFLTQNFGPVIGTGTAGYAGGLTYNYNTYSTTVDFGNGL LTLPTSVNVVTSSSPGTLGNFLSRSGAVGVLGIGPNNGFPGTSSIVTAMPGLL NNGVLIDESAGILQFGPNTLTGGITISGAPISTVAVQIDNGPLQQAPVMFDSG GINGTIPSALASLPSGGFVPAGTTISVYTSDGQTLLYSYTTTATNTPFVTSGG VMNTGHVPFAQQPIYVSYSPTAIGTTTFN (SEQ ID NO: 12; see also TUBERCULIST No. Rv0977, as available on October 6, 2009;).
MTHDHAHSRGVPAMIKEIFAPHSHDAADSVDDTLESTAAGIRTVKISLLVLG LTALIQIVIVVMSGSVALAADTIHNFADALTAVPLWIAFALGAKPATRRYTY GFGRVEDLAGSFVVAMITMSAIIAGYEAIARLIHPQQIEHVGWVALAGLVGF IGNEWVALYRIRVGHRIGSAALIADGLHARTDGFTSLAVLCSAGGVALGFP LADPIVGLLITAAILAVLRTAARDVFRRLLDGVDPAMVDAAEQALAARPGV QAVRSVRMRWIGHRLHADAELDVDPALDLAQAHRIAHDAEHELTHTVPKL TTALIHAYPAEHGSSIPDRGRTVE (SEQ ID NO: 13; see also TUBERCULIST No. Rv2025c, as available on October 6, 2009;).
VVNFSVLPPEINSGRMFFGAGSGPMLAAAAAWDGLAAELGLAAESFGLVT SGLAGGSGQAWQGAAAAAMVVAAAPYAGWLAAAAARAGGAAVQAKAV AGAFEAARAAMVDPVVVAANRSAFVQLVLSNVFGQNAPAIAAAEATYEQ MWAADVAAMVGYHGGASAAAAALAPWQQAVPGLSGLLGGAANAPAAA AQGAAQGLAELTLNLGVGNIGSLNLGSGNIGGTNVGSGNVGGTNLGSGNY GSLNWGSGNTGTGNAGSGNTGDYNPGSGNFGSGNFGSGNIGSLNVGSGNF GTLNLANGNNGDVNFGGGNTGDFNFGGGNNGTLNFGFGNTGSGNFGFGN TGNNNIGIGLTGDGQIGIGGLNSGTGNIGFGNSGNNNIGFFNSGDGNIGFFNS GDGNTGFGNAGNINTGFWNAGNLNTGFGSAGNGNVGIFDGGNSNSGSFNV GFQNTGFGNSGAGNTGFFNAGDSNTGFANAGNVNTGFFNGGDINTGGFNG GNVNTGFGSALTQAGANSGFGNLGTGNSGWGNSDPSGTGNSGFFNTGNGN SGFSNAGPAMLPGFNSGFANIGSFNAGIANSGNNLAGISNSGDDSSGAVNSG SQNSGAFNAGVGLSGFFR (SEQ ID NO: 14; see also TUBERCULIST No. Rv2356c, as available on October 6, 2009; known as PPE40).
MNYSVLPPEINSLRMFTGAGSAPMLAASVAWDRLAAELAVAASSFGSVTS GLAGQSWQGAAAAAMAAAAAPYAGWLAAAAARAAGASAQAKAVASAF EAARAATVHPMLVAANRNAFVQLVLSNLFGQNAPAIAAAEAMYEQMWA ADVAAMVGYHGGASAAAAQLSSWSIGLQQALPAAPSALAAAIGLGNIGVG NLGGGNTGDYNLGSGNSGNANVGSGNSGNANVGSGNDGATNLGSGNIGN TNLGSGNVGNVNLGSGNRGFGNLGNGNFGSGNLGSGNTGSTNFGGGNLGS FNLGSGNIGSSNIGFGNNGDNNLGLGNNGNNNIGFGLTGDNLVGIGALNSGI GNLGFGNSGNNNIGFFNSGNNNVGFFNSGNNNFGFGNAGDINTGFGNAGD TNTGFGNAGFFNMGIGNAGNEDMGVGNGGSFNVGVGNAGNQSVGFGNA GTLNVGFANAGSINTGFANSGSINTGGFDSGDRNTGFGSSVDQSVSSSGFGN TGMNSSGFFNTGNVSAGYGNNGDVQSGINNTNSGGFNVGFYNSGAGTVGI ANSGLQTTGIANSGTLNTGVANTGDHSSGGFNQGSDQSGFFGQP (SEQ ID NO: 15; see also TUBERCULIST No. Rv3159c, as available on October 6, 2009; known as PPE53).
MSFVFAAPEALAAAAADMAGIGSTLNAANVVAAVPTTGVLAAAADEVST QVAALLSAHAQGYQQLSRQMMTAFHDQFVQALRASADAYATAEASAAQT MVNAVNAPARALLGHPLISADASTGGGSNALSRVQSMFLGTGGSSALGGS AAANAAASGALQLQPTGGASGLSAVGALLPRAGAAAAAALPALAAESIGN AIKNLYNAVEPWVQYGFNLTAWAVGWLPYIGILAPQINFFYYLGEPIVQAV LFNAIDFVDGTVTFSQALTNIETATAASINQFINTEINWIRGFLPPLPPISPPGF PSLP (SEQ ID NO: 16; see also TUBERCULIST No. Rv1172c, as available on October 6, 2009; known as PE12).
MDYAFLPPEINSARMYSGPGPNSMLVAAASWDALAAELASAAENYGSVIA RLTGMHWWGPASTSMLAMSAPYVEWLERTAAQTKQTATQARAAAAAFE QAHAMTVPPALVTGIRGAIVVETASASNTAGTPP (SEQ ID NO: 17; see also TUBERCULIST No. Rv3135, as available on June 8, 2009, known as PPE50 or PPE).
LSASVSATTAHHGLPAHEVVLLLESDPYHGLSDGEAAQRLERFGPNTLAVV TRASLLARILRQFHHPLIYVLLVAGTITAGLKEFVDAAVIFGVVVINAIVGFI QESKAEAALQGLRSMVHTHAKVVREGHEHTMPSEELVPGDLVLLAAGDK VPADLRLVRQTGLSVNESALTGESTPVHKDEVALPEGTPVADRRNIAYSGT LVTAGHGAGIVVATGAETELGEIHRLVGAAEVVATPLTAKLAWFSKFLTIAI LGLAALTFGVGLLRRQDAVETFTAAIALAVGAIPEGLPTAVTITLAIGMARM AKRRAVIRRLPAVETLGSTTVICADKTGTLTENQMTVQSIWTPHGEIRATGT GYAPDVLLCDTDDAPVPVNANAALRWSLLAGACSNDAALVRDGTRWQIV GDPTEGAMLVVAAKAGFNPERLATTLPQVAAIPFSSERQYMATLHRDGTD HVVLAKGAVERMLDLCGTEMGADGALRPLDRATVLRATEMLTSRGLRVL ATGMGAGAGTPDDFDENVIPGSLALTGLQAMSDPPRAAAASAVAACHSAG IAVKMITGDHAGTATAIATEVGLLDNTEPAAGSVLTGAELAALSADQYPEA VDTASVFARVSPEQKLRLVQALQARGHVVAMTGDGVNDAPALRQANIGV AMGRGGTEVAKDAADMVLTDDDFATIEAAVEEGRGVFDNLTKFITWTLPT NLGEGLVILAAIAVGVALPILPTQILWINMTTAIALGLMLAFEPKEAGIMTRP PRDPDQPLLTGWLVRRTLLVSTLLVASAWWLFAWELDNGAGLHEARTAA LNLFVVVEAFYLFSCRSLTRSAWRLGMFANRWIILGVSAQAIAQFAITYLPA MNMVFDTAPIDIGVWVRIFAVATAITIVVATDTLLPRIRAQPP (SEQ ID NO: 18; see also TUBERCULIST No. Rv1997, as available on June 8, 2009, known as ctpF).

In a second embodiment, an Mtb polypeptide of use in the methods disclosed herein has a sequence at least 75%, 85%, 90%, 95%, 96%, 97%, 98% or 99% homologous to the amino acid sequence set forth in one of SEQ ID NOs: 1-18. For example, the polypeptide can have an amino acid sequence at least 85%, 90%, 95%, 96%, 97%, 98% or 99% homologous to one of the amino acid sequences set forth in SEQ ID NOs: 1-18. Exemplary sequences can be obtained using computer programs that are readily available on the internet and the amino acid sequences set forth herein. In one example, the polypeptide retains a function of the Mtb protein, such as binding to an antibody that specifically binds the Mtb epitope.

Minor modifications of an Mtb polypeptides primary amino acid sequences may result in peptides which have substantially equivalent activity as compared to the unmodified counterpart polypeptide described herein. Such modifications may be deliberate, as by site-directed mutagenesis, or may be spontaneous. All of the polypeptides produced by these modifications are included herein. Thus, a specific, non-limiting example of an Mtb polypeptide is a conservative variant of the Mtb polypeptide (such as a single conservative amino acid substitution, for example, one or more conservative amino acid substitutions, for example 1-10 conservative substitutions, 2-5 conservative substitutions, 4-9 conservative substitutions, such as 1, 2, 5 or 10 conservative substitutions). A table of conservative substitutions is provided herein. Substitutions of the amino acids sequence shown in SEQ ID NOs: 1-18 can be made based on this table.

Mtb polypeptides are disclosed herein that can be used to induce an immune response to Mtb. These peptides include or consist of at least nine amino acids, such as nine to twenty amino acids consecutive amino acids of an Mtb polypeptide set forth above. In particular non-limiting examples, the Mtb polypeptide includes or consists of MDFALLPPEVNSARM (amino acids 1-15 of SEQ ID NO: 2) or AEMWAQDAA (amino acids 141-149 of SEQ ID NO: 2). Specific, non-limiting examples are fifteen, fourteen, thirteen, twelve, eleven, ten, or nine consecutive amino acids of one of the Mtb polypeptides set forth above. In these examples, the Mtb polypeptide does not include the full-length amino acid sequences set forth as SEQ ID NOs: 1-18.

In several embodiments, the isolated Mtb polypeptide is included in a fusion protein. Thus, the fusion protein can include the Mtb polypeptide (see above) and a second heterologous moiety, such as a myc protein, an enzyme or a carrier (such as a hepatitis carrier protein or bovine serum albumin) covalently linked to the Mtb polypeptide. In several examples, a polypeptide consisting of nine to twelve amino acids of one of the amino acid sequences set forth as SEQ ID NOs: 1-18 that bind MHC class I is covalently linked to a carrier. In an additional example, a polypeptide consisting of one of the amino acid sequences set forth as one of SEQ ID NOs: 1-18 is covalently linked to a carrier.

In additional examples, the polypeptide can be a fusion protein and can also include heterologous sequences to Mtb. Thus, in several specific non-limiting examples, the immunogenic peptide is a fusion polypeptide, for example the polypeptide includes six sequential histidine residues, a β-galactosidase amino acid sequence, or an immunoglobulin amino acid sequence. The polypeptide can also be covalently linked to a carrier. In additional embodiments, the protein consists of the Mtb polypeptide.

The polypeptide can optionally include repetitions of one or more of the Mtb polypeptides disclosed herein. In one specific, non-limiting example, the polypeptide includes two, three, four, five, or up to ten repetitions of one of the Mtb polypeptides described above. Alternatively, more than one polypeptide can be included in a fusion polypeptide. Thus, in several examples, the polypeptide can include at least two, at least three, at least four, at least five or at least six of the amino acid sequences set forth as SEQ ID NOs: 1-18 or nine to twenty amino acids of one of the amino acid sequences set forth as SEQ ID NOs: 1-18 and repetitions of these sequences. A linker sequence can optionally be included between the Mtb polypeptides.

The Mtb polypeptides disclosed herein can be chemically synthesized by standard methods, or can be produced recombinantly. An exemplary process for polypeptide production is described in Lu et al., FEBS Lett. 429:31-35, 1998. They can also be isolated by methods including preparative chromatography and immunological separations. Polypeptides can also be produced using molecular genetic techniques, such as by inserting a nucleic acid encoding Mtb or an epitope thereof into an expression vector, introducing the expression vector into a host cell, and isolating the polypeptide (see below).

In particular embodiments provided herein, one or more of the disclosed Mtb polypeptides (or fragments thereof) can be conjugated to a substrate or solid support, such as a plate or array. In one example, the plate or array includes, consists essentially of, or consists of one (such as 2, 3, 4, 5, 6, 7, 8, 9, 10, 11, 12, 13, 14, 15, 16, 17, or all) of SEQ ID NOs: 1-18 or fragments thereof. In some examples, the plate or array also includes one or more control polypeptides. Methods for selecting an appropriate substrate and constructing a plate or array are well known to one of skill in the art (see, *e.g*., U.S. Pat. Nos. 5,143,854; 5,405,783; 5,445,934; and 5,744,305;).

Polynucleotides encoding the Mtb polypeptides disclosed herein are also provided. Exemplary nucleic acid sequences are set forth as SEQ ID NOs: 19-36. These polynucleotides include DNA, cDNA and RNA sequences which encode the polypeptide of interest. Silent mutations in the coding sequence result from the degeneracy (i.e., redundancy) of the genetic code, whereby more than one codon can encode the same amino acid residue. Tables showing the standard genetic code can be found in various sources *(e.g.,* L. Stryer, 1988, Biochemistry, 3rd Edition, W.H. Freeman and Co., NY).

A nucleic acid encoding an Mtb polypeptide can be cloned or amplified by *in vitro* methods, such as the polymerase chain reaction (PCR), the ligase chain reaction (LCR), the transcription-based amplification system (TAS), the self-sustained sequence replication system (3SR) and the Qβ replicase amplification system (QB). For example, a polynucleotide encoding the protein can be isolated by polymerase chain reaction of cDNA using primers based on the DNA sequence of the molecule. A wide variety of cloning and *in vitro* amplification methodologies are well known to persons skilled in the art. PCR methods are described in, for example, U.S. Patent No. 4,683,195; Mullis et al., Cold Spring Harbor Symp. Quant. Biol. 51:263, 1987; and Erlich, ed., PCR Technology, (Stockton Press, NY, 1989). Polynucleotides also can be isolated by screening genomic or cDNA libraries with probes selected from the sequences of the desired polynucleotide under stringent hybridization conditions.

The polynucleotides encoding an Mtb polypeptide include a recombinant DNA which is incorporated into a vector into an autonomously replicating plasmid or virus or into the genomic DNA of a prokaryote or eukaryote, or which exists as a separate molecule (such as a cDNA) independent of other sequences. The nucleotides of the invention can be ribonucleotides, deoxyribonucleotides, or modified forms of either nucleotide. The term includes single and double forms of DNA.

In one embodiment, vectors are used for expression in yeast such as S. *cerevisiae* or *Kluyveromyces lactis.* Several promoters are known to be of use in yeast expression systems such as the constitutive promoters plasma membrane H⁺-ATPase (*PMA1*), glyceraldehyde-3-phosphate dehydrogenase (*GPD*), phosphoglycerate kinase-1 (*PGK1*), alcohol dehydrogenase-1 (*ADH1*), and pleiotropic drug-resistant pump (*PDR5*)*.* In addition, many inducible promoters are of use, such as *GAL1-10* (induced by galactose), *PHO5* (induced by low extracellular inorganic phosphate), and tandem heat shock *HSE* elements (induced by temperature elevation to 37°C). Promoters that direct variable expression in response to a titratable inducer include the methionine-responsive *MET3* and *MET25* promoters and copper-dependent *CUP1* promoters. Any of these promoters may be cloned into multicopy (2µ) or single copy (*CEN*) plasmids to give an additional level of control in expression level. The plasmids can include nutritional markers (such as *URA3, ADE3, HIS1,* and others) for selection in yeast and antibiotic resistance (such as *AMP*) for propagation in bacteria. Plasmids for expression on *K. lactis* are known, such as pKLAC1. Thus, in one example, after amplification in bacteria, plasmids can be introduced into the corresponding yeast auxotrophs by methods similar to bacterial transformation.

The Mtb polypeptides can be expressed in a variety of yeast strains. For example, seven pleiotropic drug-resistant transporters, *YOR1, SNQ2, PDR5, YCF1, PDR10, PDR11,* and *PDR15,* together with their activating transcription factors, *PDR1* and *PDR3,* have been simultaneously deleted in yeast host cells, rendering the resultant strain sensitive to drugs. Yeast strains with altered lipid composition of the plasma membrane, such as the *erg6* mutant defective in ergosterol biosynthesis, can also be utilized. Proteins that are highly sensitive to proteolysis can be expressed in a yeast strain lacking the master vacuolar endopeptidase Pep4, which controls the activation of other vacuolar hydrolases. Heterologous expression in strains carrying temperature-sensitive (*ts*) alleles of genes can be employed if the corresponding null mutant is inviable.

Viral vectors encoding the Mtb polypeptides disclosed herein can also be prepared. A number of viral vectors have been constructed, including polyoma, SV40 (Madzak et al., 1992, J. Gen. Virol. 73:15331536), adenovirus (Berkner, 1992, Curr. Top. Microbiol. Immunol. 158:39-6; Berliner et al., 1988, BioTechniques 6:616-629; Gorziglia et al., 1992, J. Virol. 66:4407-4412; Quantin et al., 1992, Proc. Natl. Acad. Sci. USA 89:2581-2584; Rosenfeld et al., 1992, Cell 68:143-155; Wilkinson et al., 1992, Nucl. Acids Res. 20:2233-2239; Stratford-Perricaudet et al., 1990, Hum. Gene Ther. 1:241-256), vaccinia virus (Mackett et al., 1992, Biotechnology 24:495-499), adeno-associated virus (Muzyczka, 1992, Curr. Top. Microbiol. Immunol. 158:91-123; On et al., 1990, Gene 89:279-282), herpes viruses including HSV and EBV (Margolskee, 1992, Curr. Top. Microbiol. Immunol. 158:67-90; Johnson et al., 1992, J. Virol. 66:2952-2965; Fink et al., 1992, Hum. Gene Ther. 3:11-19; Breakfield et al., 1987, Mol. Neurobiol. 1:337-371; Fresse et al., 1990, Biochem. Pharmacol. 40:2189-2199), Sindbis viruses (Herweijer et al., 1995, Hum. Gene Ther. 6:1161-1167; U.S. Patent Nos. 5,091,309 and 5,2217,879), alphaviruses (Schlesinger, 1993, Trends Biotechnol. 11:18-22; Frolov et al., 1996, Proc. Natl. Acad. Sci. USA 93:11371-11377) and retroviruses of avian (Brandyopadhyay et al., 1984, Mol. Cell Biol. 4:749-754; Petropouplos et al., 1992, J. Virol. 66:3391-3397), murine (Miller, 1992, Curr. Top. Microbiol. Immunol. 158:1-24; Miller et al., 1985, Mol. Cell Biol. 5:431-437; Sorge et al., 1984, Mol. Cell Biol. 4:1730-1737; Mann et al., 1985, J. Virol. 54:401-407), and human origin (Page et al., 1990, J. Virol. 64:5370-5276; Buchschalcher et al., 1992, J. Virol. 66:2731-2739). Baculovirus (Autographa californica multinuclear polyhedrosis virus; AcMNPV) vectors are also known in the art, and may be obtained from commercial sources (such as PharMingen, San Diego, Calif.; Protein Sciences Corp., Meriden, Conn.; Stratagene, La Jolla, Calif.).

Thus, in one embodiment, the polynucleotide encoding an Mtb polypeptide is included in a viral vector. Suitable vectors include retrovirus vectors, orthopox vectors, avipox vectors, fowlpox vectors, capripox vectors, suipox vectors, adenoviral vectors, herpes virus vectors, alpha virus vectors, baculovirus vectors, Sindbis virus vectors, vaccinia virus vectors and poliovirus vectors. Specific exemplary vectors are poxvirus vectors such as vaccinia virus, fowlpox virus and a highly attenuated vaccinia virus (MVA), adenovirus, baculovirus and the like.

Pox viruses useful in practicing the present methods include orthopox, suipox, avipox, and capripox virus. Orthopox include vaccinia, ectromelia, and raccoon pox. One example of an orthopox of use is vaccinia. Avipox includes fowlpox, canary pox and pigeon pox. Capripox include goatpox and sheep pox. In one example, the suipox is swinepox. Examples of pox viral vectors for expression as described for example, in U.S. Patent No. 6,165,460, Other viral vectors that can be used include other DNA viruses such as herpes virus and adenoviruses, and RNA viruses such as retroviruses and poliovirus.

In some cases, vaccinia viral vectors may elicit a strong antibody response. Thus, while numerous boosts with vaccinia vectors are possible, its repeated use may not be useful in certain instances. However, this sensitivity problem can be minimized by using pox from different genera for boosts. In one example, when the first or initial pox virus vector is vaccinia, the second and subsequent pox virus vectors are selected from the pox viruses from a different genus such as suipox, avipox, capripox or an orthopox immunogenically distinct from vaccinia.

The vaccinia virus genome is known in the art. It is composed of a HIND F13L region, TK region, and an HA region. Recombinant vaccinia virus has been used to incorporate an exogenous gene for expression of the exogenous gene product (see, for example, Perkus et al. Science 229:981-984, 1985; Kaufman et al. Int. J. Cancer 48:900-907, 1991; Moss, Science 252:1662, 1991). A gene encoding an antigen of interest, such as an immunogenic Mtb polypeptide, can be incorporated into the HIND F13L region or alternatively incorporated into the TK region of recombinant vaccinia virus vector (or other nonessential regions of the vaccinia virus genome). Baxby and Paoletti (*Vaccine* 10:8-9, 1992) disclose the construction and use as a vector, of the non-replicating poxvirus, including canarypox virus, fowlpox virus and other avian species. Sutter and Moss (Proc. Natl. Acad. Sci. U.S.A. 89:10847-10851, 1992) and Sutter *et al.* (Vaccine 12:1032-1040, 1994) disclose the construction and use as a vector of the non-replicating recombinant Ankara virus (MVA, modified vaccinia Ankara).

Suitable vectors are disclosed, for example, in U.S. Patent No. 6,998,252,. In one example, a recombinant poxvirus, such as a recombinant vaccinia virus is synthetically modified by insertion of a chimeric gene containing vaccinia regulatory sequences or DNA sequences functionally equivalent thereto flanking DNA sequences which in nature are not contiguous with the flanking vaccinia regulatory DNA sequences that encode a Mtb polypeptide. The recombinant virus containing such a chimeric gene is effective at expressing the Mtb polypeptide. In one example, the vaccine viral vector comprises (A) a segment comprised of (i) a first DNA sequence encoding a Mtb polypeptide and (ii) a poxvirus promoter, wherein the poxvirus promoter is adjacent to and exerts transcriptional control over the DNA sequence encoding an Mtb polypeptide; and, flanking said segment, (B) DNA from a nonessential region of a poxvirus genome. The viral vector can encode a selectable marker. In one example, the poxvirus includes, for example, a thymidine kinase gene (see U.S. Patent No. 6,998,252,).

Viral vectors, such as poxviral vectors, that encode an Mtb polypeptide include at least one expression control element operationally linked to the nucleic
acid sequence encoding the Mtb polypeptide. The expression control elements are inserted in the viral vector to control and regulate the expression of the nucleic acid sequence. Examples of expression control elements of use in these vectors includes, but is not limited to, lac system, operator and promoter regions of phage lambda, yeast promoters and promoters derived from polyoma, adenovirus, retrovirus or SV40. Additional operational elements include, but are not limited to, leader sequence, termination codons, polyadenylation signals and any other sequences necessary for the appropriate transcription and subsequent translation of the nucleic acid sequence encoding the Mtb polypeptide in the host system. The expression vector can contain additional elements necessary for the transfer and subsequent replication of the expression vector containing the nucleic acid sequence in the host system. Examples of such elements include, but are not limited to, origins of replication and selectable markers. It will further be understood by one skilled in the art that such vectors are easily constructed using conventional methods (Ausubel et al., (1987) in "Current Protocols in Molecular Biology," John Wiley and Sons, New York, N.Y.) and are commercially available.

Basic techniques for preparing recombinant DNA viruses containing a heterologous DNA sequence encoding the one or more Mtb polypeptides are known in the art. Such techniques involve, for example, homologous recombination between the viral DNA sequences flanking the DNA sequence in a donor plasmid and homologous sequences present in the parental virus (Mackett et al., 1982, Proc. Natl. Acad. Sci. USA 79:7415-7419). In particular, recombinant viral vectors such as a poxviral vector can be used in delivering the gene. The vector can be constructed for example by steps known in the art, such as steps analogous to the methods for creating synthetic recombinants of the fowlpox virus described in U.S. Pat. No. 5,093,258, Other techniques include using a unique restriction endonuclease site that is naturally present or artificially inserted in the parental viral vector to insert the heterologous DNA.

Generally, a DNA donor vector contains the following elements: (i) a prokaryotic origin of replication, so that the vector may be amplified in a prokaryotic host; (ii) a gene encoding a marker which allows selection of prokaryotic host cells that contain the vector *(e.g.,* a gene encoding antibiotic resistance); (iii) at least one DNA sequence encoding the one or more Mtb polypeptide located adjacent to a transcriptional promoter capable of directing the expression of the sequence; and (iv) DNA sequences homologous to the region of the parent virus genome where the foreign gene(s) will be inserted, flanking the construct of element (iii). Methods for constructing donor plasmids for the introduction of multiple foreign genes into pox virus are described in PCT Publication No. WO 91/19803,

Generally, DNA fragments for construction of the donor vector, including fragments containing transcriptional promoters and fragments containing sequences homologous to the region of the parent virus genome into which foreign DNA sequences are to be inserted, can be obtained from genomic DNA or cloned DNA fragments. The donor plasmids can be mono-, di-, or multivalent (*e.g*., can contain one or more inserted foreign DNA sequences). The donor vector can contain an additional gene that encodes a marker that will allow identification of recombinant viruses containing inserted foreign DNA. Several types of marker genes can be used to permit the identification and isolation of recombinant viruses. These include genes that encode antibiotic or chemical resistance (e.g., see Spyropoulos et al., 1988, J. Virol. 62:1046; Falkner and Moss, 1988, J. Virol. 62:1849; Franke et al., 1985, Mol. Cell. Biol. 5:1918), as well as genes such as the *E*. *coli* lacZ gene, that permit identification of recombinant viral plaques by colorimetric assay (Panicali et al., 1986, Gene 47:193-199).

The DNA gene sequence to be inserted into the virus can be placed into a donor plasmid, such as an *E*. *coli* or a *salmonella* plasmid construct, into which DNA homologous to a section of DNA such as that of the insertion site of the poxvirus where the DNA is to be inserted has been inserted. Separately the DNA gene sequence to be inserted is ligated to a promoter. The promoter-gene linkage is positioned in the plasmid construct so that the promoter-gene linkage is flanked on both ends by DNA homologous to a DNA sequence flanking a region of pox DNA that is the desired insertion region. With a parental pox viral vector, a pox promoter is used. The resulting plasmid construct is then amplified by growth within *E*. *coli* bacteria and isolated. Next, the isolated plasmid containing the DNA gene sequence to be inserted is transfected into a cell culture, for example chick embryo fibroblasts, along with the parental virus, for example poxvirus. Recombination between homologous pox DNA in the plasmid and the viral genome respectively results in a recombinant poxvirus modified by the presence of the promoter-gene construct in its genome, at a site that does not affect virus viability.

As noted above, the DNA sequence is inserted into a region (insertion region) in the virus that does not affect virus viability of the resultant recombinant virus. One of skill in the art can readily identify such regions in a virus by, for example, randomly testing segments of virus DNA for regions that allow recombinant formation without seriously affecting virus viability of the recombinant. One region that can readily be used and is present in many viruses is the thymidine kinase (TK) gene. The TK gene has been found in all pox virus genomes examined, including leporipoxvirus (Upton et al., 1986, J. Virol. 60:920); shope fibroma virus; capripoxvirus (Gershon et al., 1989, J. Gen. Virol. 70:525); Kenya sheep-1; orthopoxvirus (Weir et al., 1983, J. Virol. 46:530); vaccinia (Esposito et al., 1984, Virology 135:561); monkeypox and variola virus (Hruby et al., 1983, Proc. Natl. Acad. Sci. USA 80:3411); vaccinia (Kilpatrick et al., 1985, Virology 143:399); Yaba monkey tumor virus; avipoxvirus (Binns et al., 1988, J. Gen. Virol. 69:1275); fowlpox; (Boyle et al., 1987, Virology 156:355; Schnitzlein et al., 1988, J. Virol. Meth. 20:341); and entomopox (Lytvyn et al., 1992, J. Gen. Virol. 73:3235-3240). In vaccinia, in addition to the TK region, other insertion regions include, for example, the HindIII M fragment. In fowlpox, in addition to the TK region, other insertion regions include, for example, the BamHI J fragment (Jenkins et al., 1991, AIDS Res. Hum. Retroviruses 7:991-998), the EcoRI-HindIII fragment, EcoRV-HindIII fragment, BamHI fragment and the HindIII fragment set forth in EPO Application No. 0 308220 A1 (see also Calvert et al., 1993, J. Virol. 67:3069-3076; Taylor et al., 1988, Vaccine 6:497-503; Spehner et al., 1990, J. Virol. 64:527-533; Boursnell et al., 1990, J. Gen. Virol. 71:621-628).

In swinepox, insertion sites include the thymidine kinase gene region. In addition to the requirement that the gene be inserted into an insertion region, successful expression of the inserted gene by the modified poxvirus requires the presence of a promoter operably linked to the desired gene. Generally, the promoter is placed so that it is located upstream from the gene to be expressed. Promoters are well known in the art and can readily be selected depending on the host and the cell type to be targeted. In one example, in poxviruses, pox viral promoters are used, such as the vaccinia 7.5K, 40K or fowlpox promoters such as FPV C1A. Enhancer elements can also be used in combination to increase the level of expression. Furthermore, inducible promoters can be utilized.

Homologous recombination between donor plasmid DNA and viral DNA in an infected cell can result in the formation of recombinant viruses that incorporate the desired elements. Appropriate host cells for *in vivo* recombination are generally eukaryotic cells that can be infected by the virus and transfected by the plasmid vector. Examples of such cells suitable for use with a pox virus are chick embryo fibroblasts, HuTK143 (human) cells, and CV-1 and BSC-40 (both monkey kidney) cells. Infection of cells with pox virus and transfection of these cells with plasmid vectors is accomplished by techniques standard in the art (see U.S. Pat. No. 4,603,112 and PCT Publication No. WO 89/03429).

Following *in vivo* recombination, recombinant viral progeny can be identified by one of several techniques. For example, if the DNA donor vector is designed to insert foreign genes into the parent virus thymidine kinase (TK) gene, viruses containing integrated DNA will be TK- and can be selected on this basis (Mackett et al., 1982, Proc. Natl. Acad. Sci. USA 79:7415). Alternatively, co-integration of a gene encoding a marker or indicator gene with the foreign gene(s) of interest, as described above, can be used to identify recombinant progeny. One specific non-limiting example of an indicator gene is the *E. coli* lacZ gene. Recombinant viruses expressing beta-galactosidase can be selected using a chromogenic substrate for the enzyme (Panicali et al., 1986, Gene 47:193). Once a recombinant virus has been identified, a variety of well-known methods can be used to assay the expression of the Mtb sequence encoded by the inserted DNA fragment. These methods include black plaque assay (an *in situ* enzyme immunoassay performed on viral plaques), Western blot analysis, radioimmunoprecipitation (RIPA), and enzyme immunoassay (EIA).

This disclosure encompasses a recombinant virus comprising more than one antigen of interest for the purpose of having a multivalent vaccine. For example, the recombinant virus may comprise the virus genome or portions thereof, the nucleic acid sequence encoding the Mtb polypeptide and a nucleic acid sequence encoding a hepatitis B surface antigen or any other carrier protein.

In one embodiment, a composition is provided that includes a recombinant virus comprising a vaccinia virus genome or portions thereof, the nucleic acid sequence encoding an Mtb polypeptide and a recombinant virus comprising the nucleic acid sequence encoding the immunostimulatory molecule, B7-1 alone or in combination with the nucleic acid sequence encoding the immunostimulatory molecule, B7-2, or a recombinant virus containing both the genes for an Mtb polypeptide and an immunostimulatory molecule. This disclosure also encompasses a recombinant virus comprising the Mtb polypeptide that is administered with a second recombinant virus comprising the virus genome or portion thereof, and one or more nucleic acid sequences encoding one or more B7 molecules, such as a recombinant vaccinia virus expressing B7-1 and/or B7-2.

Thus, in one example, recombinant virus is disclosed that is a recombinant vaccinia virus containing B7-1 and a recombinant vaccinia virus containing B7-2 (designated rV-B7-1 and rV-B7-2, respectively); the composition can include rV-B7-1 and/or rV-B7-2 in combination with an immunogenic Mtb polypeptide.

The B7 molecule includes but is not limited to B7-1, B7-2 and analogs thereof. The B7 gene may be cloned from mammalian sources, including but not limited to mammalian tissues, genomic libraries or cDNA libraries, such as from murine or human sources. Without being bound by theory, costimulatory molecules of the B7 family (namely B7-1, B7-2, and possibly B7-3) are believed to be members of the immunoglobulin gene superfamily. These molecules are present on macrophages, dendritic cells, monocytes (antigen presenting cells (APCs)). Significant amplification of the immune response against a given antigen generally does not occur without co-stimulation (June et al.. Immunology Today 15:321-331, 1994; Chen et al., Immunology Today 14:483-486; Townsend et al., Science 259:368-370). Freeman *et al.* (J. Immunol. 143:2714-2722, 1989) report cloning and sequencing of a B7-1 gene. Azuma *et al.* (Nature 366:76-79, 1993) report cloning and sequencing of a B7-2 gene. Thus, in one embodiment the B7-1 gene or the B7-2 genes are administered in conjunction with the Mtb polypeptide. The insertion of nucleic acids encoding B7-1 and B7-2 into vaccinia virus has been disclosed (see for example, U.S. Patent No. 6,893,869, : this U.S. Patent also discloses the use of a nucleic acid encoding IL-2 in a vaccinia virus). Several vectors including IL-2, B7-1 and B7-2 have been deposited with the American Type Culture Collection (ATCC) on Oct. 3, 1994 under the terms of the Budapest Treaty (for example, rV-CEA/ₙIL-2 (ATCC Designation VR 2480), rV-ₘB7-2 (ATCC Designation VR 2482); and rV-ₘB7-1 (ATCC Designation VR 2483)).

DNA sequences encoding an Mtb polypeptide can be expressed *in vitro* by DNA transfer into a suitable host cell. The cell may be prokaryotic or eukaryotic. The term also includes any progeny of the subject host cell. It is understood that all progeny may not be identical to the parental cell since there may be mutations that occur during replication. Methods of stable transfer, meaning that the foreign DNA is continuously maintained in the host, are known in the art.

As noted above, a polynucleotide sequence encoding an Mtb polypeptide can be operatively linked to expression control sequences. An expression control sequence operatively linked to a coding sequence is ligated such that expression of the coding sequence is achieved under conditions compatible with the expression control sequences. The expression control sequences include, but are not limited to, appropriate promoters, enhancers, transcription terminators, a start codon (i.e., ATG) in front of a protein-encoding gene, splicing signal for introns, maintenance of the correct reading frame of that gene to permit proper translation of mRNA, and stop codons.

Host cells can include microbial, yeast, insect and mammalian host cells. Methods of expressing DNA sequences having eukaryotic or viral sequences in prokaryotes are well known in the art. Non-limiting examples of suitable host cells include bacteria, archea, insect, fungi (for example, yeast), mycobacterium (such as *M. smegmatis*), plant, and animal cells (for example, mammalian cells, such as human). Exemplary cells of use include *E. coli, Bacillus subtilis, Saccharomyces cerevisiae, Salmonella typhimurium,* SF9 cells, C129 cells, 293 cells, *Neurospora,* and immortalized mammalian myeloid and lymphoid cell lines. Techniques for the propagation of mammalian cells in culture are well-known (see, Jakoby and Pastan (eds), 1979, Cell Culture. Meth. Enzymol., volume 58, Academic Press, Inc., Harcourt Brace Jovanovich, N.Y.). Examples of commonly used mammalian host cell lines are VERO and HeLa cells, CHO cells, and WI38, BHK, and COS cell lines, although other cell lines may be used, such as cells designed to provide higher expression, desirable glycosylation patterns, or other features. As discussed above, techniques for the transformation of yeast cells, such as polyethylene glycol transformation, protoplast transformation and gene guns are also known in the art (see Gietz and Woods, Meth. Enzymol. 350: 87-96, 2002).

Transformation of a host cell with recombinant DNA can be carried out by conventional techniques as are well known to those skilled in the art. Where the host is prokaryotic, such as, but not limited to, *E. coli,* competent cells which are capable of DNA uptake can be prepared from cells harvested after exponential growth phase and subsequently treated by the CaCl₂ method using procedures well known in the art. Alternatively, MgCl₂ or RbCl can be used. Transformation can also be performed after forming a protoplast of the host cell if desired, or by electroporation.

When the host is a eukaryote, such methods of transfection of DNA as calcium phosphate coprecipitates, conventional mechanical procedures such as microinjection, electroporation, insertion of a plasmid encased in liposomes, or virus vectors can be used. Eukaryotic cells can also be co-transformed with polynucleotide sequences encoding an Mtb polypeptide, and a second foreign DNA molecule encoding a selectable phenotype, such as the herpes simplex thymidine kinase gene. Another method is to use a eukaryotic viral vector, such as simian virus 40 (SV40) or bovine papilloma virus, to transiently infect or transform eukaryotic cells and express the protein (see for example, Eukaryotic Viral Vectors, Cold Spring Harbor Laboratory, Gluzman ed., 1982).

In particular embodiments provided herein, one or more of the disclosed Mtb polynucleotides (or fragments thereof) can be conjugated to a substrate or solid support, such as a plate or array. In one example, the plate or array includes, consists essentially of, or consists of one (such as 2, 3, 4, 5, 6, 7, 8, 9, 10, 11, 12, 13, 14, 15, 16, 17, or all) of SEQ ID NOs: 19-36 or fragments thereof. In some examples, the plate or array also includes one or more control polynucleotides. Methods for selecting an appropriate substrate and constructing a plate or array are well known to one of skill in the art (see, *e.g.,* U.S. Pat. Nos. 5,554,501; 5,985,567; 5,981,185; and 6,013,789; and PCT Publications WO 85/01051 and WO 89/10977;).

### IV. Therapeutic Methods and Pharmaceutical Compositions

The Mtb polypeptides disclosed herein, or nucleic acids encoding the Mtb polypeptides, can be used to generate an immune response in a subject. In several examples, the subject is infected with Mtb or is at risk of being infected with Mtb. Thus, in several embodiments, the methods include administering to a subject a therapeutically effective amount of one or more of the Mtb polypeptides disclosed herein (or polynucleotides encoding these polypeptides) in order to generate an immune response, such as, but not limited to, a protective immune response.

In exemplary applications, compositions are administered to a subject in an amount sufficient to produce an immune response to Mtb. These Mtb polypeptides, or polynucleotides encoding these polypeptides, are of use to inhibit or prevent an infection with Mtb, inhibit or prevent progression to disease in a subject having a latent Mtb infection, or to treat tuberculosis in a subject infected with Mtb. In several examples, administration of a therapeutically effective amount of a composition including one or more of the Mtb polypeptides disclosed herein (or polynucleotides encoding these polypeptides) induces a sufficient immune response to decrease a symptom of a disease due to Mtb infection, to inhibit the development of one or more symptoms of tuberculosis, or to inhibit infection with Mtb.

In some examples, the compositions are of use in inhibiting or preventing a future infection with Mtb. Thus, a therapeutically effective amount of the composition is administered to a subject at risk of becoming infected with Mtb. The composition inhibits or prevents the development of tuberculosis, such as latent or active tuberculosis, in the subject upon subsequent exposure to Mtb.

In additional examples, the compositions are administered to a subject with a latent Mtb infection, and inhibit or prevent the development of symptoms of tuberculosis. In some examples, the compositions are of use in treating a subject with latent tuberculosis, such that the subject does not develop active tuberculosis.

Amounts effective for these uses will depend upon the severity of the disease, the general state of the patient's health, and the robustness of the patient's immune system. In one example, a therapeutically effective amount of the compound is that which provides either subjective relief of a symptom(s) or an objectively identifiable improvement as noted by the clinician or other qualified observer. In other examples, a therapeutically effective amount is an amount sufficient to inhibit an infection with Mtb in a subject upon subsequent exposure of the subject to Mtb. In additional examples, a therapeutically effective amount is an amount sufficient to inhibit development of one or more symptoms in a subject infected with Mtb.

In some examples, one or more Mtb polypeptide described herein may be covalently linked to at least one other immunogenic protein, wherein the conjugate elicits an immune response to the Mtb polypeptide in a subject. The other immunogenic protein (sometimes referred to as a "carrier" protein) ideally has the properties of being immunogenic by itself, usable in a subject, and of a size that can be easily purified and conjugated to at least one other protein or peptide. Suitable carrier proteins are known to one of skill in the art. In particular examples, the other immunogenic protein (carrier protein) is bovine serum albumin (BSA), ovalbumin, tetanus toxoid, diphtheria toxoid, cholera toxin, *Clostridium difficile* toxin A, *C*. *difficile* toxin B, Shiga toxin, or *Pseudomonas aeruginosa* recombinant exoprotein A.

An Mtb polypeptide can be administered by any means known to one of skill in the art (see Banga, A., "Parenteral Controlled Delivery of Therapeutic Peptides and Proteins," in Therapeutic Peptides and Proteins, Technomic Publishing Co., Inc., Lancaster, PA, 1995) either locally or systemically, such as by intramuscular injection, subcutaneous injection, intraperitoneal injection, intravenous injection, oral administration, nasal administration, transdermal administration, or even anal administration. In one embodiment, administration is by oral administration, subcutaneous injection, or intramuscular injection. To extend the time during which the peptide or protein is available to stimulate a response, the peptide or protein can be provided as an implant, an oily injection, or as a particulate system. The particulate system can be a microparticle, a microcapsule, a microsphere, a nanocapsule, or similar particle. (*see,* e.g., Banga, *supra*)*.* A particulate carrier based on a synthetic polymer has been shown to act as an adjuvant to enhance the immune response, in addition to providing a controlled release. Aluminum salts can also be used as adjuvants to produce an immune response.

In one specific, non-limiting example, the Mtb polypeptide is administered in a manner to direct the immune response to a cellular response (that is, a cytotoxic T lymphocyte (CTL) response), rather than a humoral (antibody) response.

Optionally, one or more cytokines, such as IL-2, IL-6, IL-12, RANTES, GM-CSF, TNF-α, or IFN-γ, one or more growth factors, such as GM-CSF or G-CSF; one or more molecules such as OX-40L or 4-1 BBL, or combinations of these molecules, can be used as biological adjuvants (see, for example, Salgaller et al., 1998, J. Surg. Oncol. 68(2):122-38; Lotze et al., 2000, Cancer J. Sci. Am. 6(Suppl 1):S61-6; Cao et al., 1998, Stem Cells 16(Suppl 1):251-60; Kuiper et al., 2000, Adv. Exp. Med. Biol. 465:381-90). These molecules can be administered systemically (or locally) to the host. In several examples, IL-2, RANTES, GM-CSF, TNF-α, IFN-γ, G-CSF, LFA-3, CD72, B7-1, B7-2, B7-1 B7-2, OX-40L, 4-1 BBL, and/or ICAM-1 are administered.

A number of means for inducing cellular responses, both *in vitro* and *in vivo,* are known. Lipids have been identified as agents capable of assisting in priming CTL *in vivo* against various antigens. For example, as described in U.S. Patent No. 5,662,907, palmitic acid residues can be attached to the alpha and epsilon amino groups of a lysine residue and then linked (for example, via one or more linking residues, such as glycine, glycine-glycine, serine, serine-serine, or the like) to an immunogenic peptide. The lipidated peptide can then be injected directly in a micellar form, incorporated in a liposome, or emulsified in an adjuvant. As another example, *E. coli* lipoproteins, such as tripalmitoyl-S-glycerylcysteinlyseryl-serine can be used to prime tumor specific CTL when covalently attached to an appropriate peptide (see, Deres et al., Nature 342:561, 1989). Further, as the induction of neutralizing antibodies can also be primed with the same molecule conjugated to a peptide which displays an appropriate epitope, two compositions can be combined to elicit both humoral and cell-mediated responses where that is deemed desirable.

A pharmaceutical composition including an Mtb polypeptide is thus provided. These compositions are of use to promote an immune response to Mtb. In one embodiment, the Mtb polypeptide is mixed with an adjuvant containing two or more of a stabilizing detergent, a micelle-forming agent, and an oil. Suitable stabilizing detergents, micelle-forming agents, and oils are detailed in U.S. Patent No. 5,585,103; U.S. Patent No. 5,709,860; U.S. Patent No. 5,270,202; and U.S. Patent No. 5,695,770,. A stabilizing detergent is any detergent that allows the components of the emulsion to remain as a stable emulsion. Such detergents include polysorbate, 80 (TWEEN) (Sorbitan-mono-9-octadecenoate-poly(oxy-1,2-ethanediyl; manufactured by ICI Americas, Wilmington, DE), TWEEN 40™, TWEEN 20™, TWEEN 60™, ZWITTERGENT™ 3-12, TEEPOL HB7™, and SPAN 85™. These detergents are usually provided in an amount of approximately 0.05 to 0.5%, such as at about 0.2%. A micelle forming agent is an agent which is able to stabilize the emulsion formed with the other components such that a micelle-like structure is formed. Such agents generally cause some irritation at the site of injection in order to recruit macrophages to enhance the cellular response. Examples of such agents include polymer surfactants described by BASF Wyandotte publications, *e.g.,* Schmolka, J. Am. Oil. Chem. Soc. 54:110, 1977; and Hunter et al., J. Immunol. 127:1244-1250, 1981; for example, PLURONIC™ L62 LF, L101, and L64, PEG1000, and TETRONIC™ 1501, 150R1, 701, 901, 1301, and 130R1. The 701, 901, 1301, and 130R1. The chemical structures of such agents are well known in the art. In one embodiment, the agent is chosen to have a hydrophile-lipophile balance (HLB) of between 0 and 2, as defined by Hunter and Bennett, J. Immun. 133:3167, 1984. The agent can be provided in an effective amount, for example between 0.5 and 10%, or in an amount between 1.25 and 5%.

The oil included in the composition is chosen to promote the retention of the antigen in oil-in-water emulsion, such as to provide a vehicle for the desired antigen, and preferably has a melting temperature of less than 65°C such that emulsion is formed either at room temperature (about 20°C to 25°C), or once the temperature of the emulsion is brought down to room temperature. Examples of such oils include squalene, squalane, EICOSANE™, tetratetracontane, glycerol, and peanut oil or other vegetable oils. In one specific, non-limiting example, the oil is provided in an amount between 1 and 10%, or between 2.5 and 5%. The oil should be both biodegradable and biocompatible so that the body can break down the oil over time, and so that no adverse affects, such as granulomas, are evident upon use of the oil.

In one embodiment, the adjuvant is a mixture of stabilizing detergents, micelle-forming agent, and oil available under the name PROVAX® (IDEC Pharmaceuticals, San Diego, CA). An adjuvant can also be an immunostimulatory nucleic acid, such as a nucleic acid including a CpG motif, or a biological adjuvant (see above).

Controlled release parenteral formulations can be made as implants, oily injections, or as particulate systems. For a broad overview of protein delivery systems, see Banga, Therapeutic Peptides and Proteins: Formulation, Processing, and Delivery Systems, Technomic Publishing Company, Inc., Lancaster, PA, 1995. Particulate systems include microspheres, microparticles, microcapsules, nanocapsules, nanospheres, and nanoparticles. Microcapsules contain the therapeutic protein as a central core. In microspheres, the therapeutic agent is dispersed throughout the particle. Particles, microspheres, and microcapsules smaller than about 1 µm are generally referred to as nanoparticles, nanospheres, and nanocapsules, respectively. Capillaries have a diameter of approximately 5 µm so that only nanoparticles are administered intravenously. Microparticles are typically around 100 µm in diameter and are administered subcutaneously or intramuscularly (see Kreuter, Colloidal Drug Delivery Systems, J. Kreuter, ed., Marcel Dekker, Inc., New York, NY, pp. 219-342, 1994; Tice & Tabibi, Treatise on Controlled Drug Delivery, A. Kydonieus, ed., Marcel Dekker, Inc. New York, NY, pp. 315-339, 1992).

Polymers can be used for controlled release. Various degradable and nondegradable polymeric matrices for use in controlled drug delivery are known in the art (Langer, Accounts Chem. Res. 26:537, 1993). For example, the block copolymer, polaxamer 407 exists as a viscous yet mobile liquid at low temperatures but forms a semisolid gel at body temperature. It has shown to be an effective vehicle for formulation and sustained delivery of recombinant interleukin-2 and urease (Johnston et al., Pharm. Res. 9:425, 1992; and Pec, J. Parent. Sci. Tech. 44(2):58, 1990). Alternatively, hydroxyapatite has been used as a microcarrier for controlled release of proteins (Ijntema et al., Int. J. Pharm. 112:215, 1994). In yet another aspect, liposomes are used for controlled release as well as drug targeting of the lipid-capsulated drug (Betageri et al., Liposome Drug Delivery Systems, Technomic Publishing Co., Inc., Lancaster, PA, 1993). Numerous additional systems for controlled delivery of therapeutic proteins are known (e.g., U.S. Patent Nos. 5,055,303; 5,188,837; 4,235,871; 4,501,728; 4,837,028; 4,957,735; 5,019,369; 5,055,303; 5,514,670; 5,413,797; 5,268,164; 5,004,697; 4,902,505; 5,506,206; 5,271,961; 5,254,342; and 5,534,496).

In another embodiment, a pharmaceutical composition includes a nucleic acid encoding an Mtb polypeptide. A therapeutically effective amount of the Mtb polynucleotide can be administered to a subject in order to generate an immune response.

Optionally, one or more cytokines, such as IL-2, IL-6, IL-12, RANTES, GM-CSF, TNF-α, or IFN-γ, one or more growth factors, such as GM-CSF or G-CSF, one or more costimulatory molecules, such as ICAM-1, LFA-3, CD72, B7-1, B7-2, or other B7 related molecules; one or more molecules such as OX-40L or 4-1 BBL, or combinations of these molecules, can be used as biological adjuvants (see, for example, Salgaller et al., 1998, J. Surg. Oncol. 68(2):122-38; Lotze et al., 2000, Cancer J. Sci. Am. 6(Suppl 1):561-6; Cao et al., 1998, Stem Cells 16(Suppl 1):251-60; Kuiper et al., 2000, Adv. Exp. Med. Biol. 465:381-90). These molecules can be administered systemically to the host. It should be noted that these molecules can be co-administered via insertion of a nucleic acid encoding the molecules into a vector, for example, a recombinant pox vector (see, for example, U.S. Pat. No. 6,045,802). In various embodiments, the nucleic acid encoding the biological adjuvant can be cloned into same vector as the Mtb polypeptide coding sequence, or the nucleic acid can be cloned into one or more separate vectors for co-administration. In addition, nonspecific immunomodulating factors such as BCG and levamisole can be co-administered.

One approach to administration of nucleic acids is direct immunization with plasmid DNA, such as with a mammalian expression plasmid. As described above, the nucleotide sequence encoding an Mtb polypeptide can be placed under the control of a promoter to increase expression of the molecule.

Immunization by nucleic acid constructs is well known in the art and taught, for example, in U.S. Patent No. 5,643,578 (which describes methods of immunizing vertebrates by introducing DNA encoding a desired antigen to elicit a cell-mediated or a humoral response), and U.S. Patent Nos. 5,593,972 and 5,817,637 (which describe operably linking a nucleic acid sequence encoding an antigen to regulatory sequences enabling expression). U.S. Patent No. 5,880,103 describes several methods of delivery of nucleic acids encoding immunogenic peptides or other antigens to an organism. The methods include liposomal delivery of the nucleic acids (or of the synthetic peptides themselves), and immune-stimulating constructs, or ISCOMS™ (negatively charged cage-like structures of 30-40 nm in size formed spontaneously on mixing cholesterol and saponin). Protective immunity has been generated in a variety of experimental models of infection, including toxoplasmosis and Epstein-Barr virus-induced tumors, using ISCOMS™ as the delivery vehicle for antigens (Mowat and Donachie, Immunol. Today 12:383, 1991). Doses of antigen as low as 1 µg encapsulated in ISCOMS™ have been found to produce Class I mediated CTL responses (Takahashi et al., Nature 344:873, 1990).

In another approach to using nucleic acids for immunization, an Mtb polypeptide can also be expressed by attenuated viral hosts or vectors or bacterial vectors. Recombinant vaccinia virus, adeno-associated virus (AAV), herpes virus, retrovirus, or other viral vectors can be used to express the peptide or protein, thereby eliciting a CTL response. For example, vaccinia vectors and methods useful in immunization protocols are described in U.S. Patent No. 4,722,848. BCG provides another vector for expression of the peptides (see Stover, Nature 351:456-460, 1991).

When a viral vector is utilized, it is desirable to provide the recipient with a dosage of each recombinant virus in the composition in the range of from about 10⁵ to about 10¹⁰ plaque forming units, although a lower or higher dose can be administered. The composition of recombinant viral vectors can be introduced into a mammal (1) prior to any evidence of an infection with Mtb; (2) to inhibit development of tuberculosis in an individual infected with Mtb; or (3) to decrease a symptom of tuberculosis in a mammal infected with Mtb. Examples of methods for administering the composition into mammals include, but are not limited to, exposure of cells to the recombinant virus *ex vivo*, or injection of the composition into the affected tissue or intravenous, subcutaneous, intradermal or intramuscular administration of the virus. Alternatively the recombinant viral vector or combination of recombinant viral vectors may be administered locally in a pharmaceutically acceptable carrier. Generally, the quantity of recombinant viral vector, carrying the nucleic acid sequence of one or more Mtb polypeptides to be administered is based on the titer of virus particles. An exemplary range of the immunogen to be administered is 10⁵ to 10¹⁰ virus particles per mammal, such as a human.

In the embodiment where a combination of a first recombinant viral vector carrying a nucleic acid sequence of one or more Mtb polypeptide and a second recombinant viral vector carrying the nucleic acid sequence of one or more immunostimulatory molecules is used, the mammal can be immunized with different ratios of the first and second recombinant viral vector. In one embodiment the ratio of the first vector to the second vector is about 1:1, or about 1:3, or about 1:5. Optimal ratios of the first vector to the second vector may easily be titered using the methods known in the art (see, for example, U.S. Patent No. 6,893,869,).

In one embodiment the recombinant viruses have been constructed to express cytokines (such as TNF-α, IL-6, GM-CSF, and IL-2), and costimulatory and accessory molecules (B7-1, B7-2) alone and in a variety of combinations. Simultaneous production of an immunostimulatory molecule and the Mtb polypeptide enhances the generation of specific effectors. Without being bound by theory, dependent upon the specific immunostimulatory molecules, different mechanisms might be responsible for the enhanced immunogenicity: augmentation of help signal (IL-2), recruitment of professional APC (GM-CSF), increase in CTL frequency (IL-2), effect on antigen processing pathway and MHC expression (IFNγ and TNFα) and the like. For example, IL-2, IL-6, interferon, tumor necrosis factor, or a nucleic acid encoding these molecules, can be administered in conjunction with an Mtb immunogenic polypeptide, or a nucleic acid encoding an Mtb polypeptide. The co-expression of an Mtb polypeptide together with at least one immunostimulatory molecule can be effective in an animal model to show therapeutic effects.

In one embodiment, a nucleic acid encoding an Mtb polypeptide is introduced directly into cells. For example, the nucleic acid can be loaded onto gold microspheres by standard methods and introduced into the skin by a device such as the Helios™ Gene Gun (Bio-Rad, Hercules, CA). The nucleic acids can be "naked," consisting of plasmids under control of a strong promoter. Typically, the DNA is injected into muscle, although it can also be injected directly into other sites. Dosages for injection are usually around 0.5 µg/kg to about 50 mg/kg, and typically are about 0.005 mg/kg to about 5 mg/kg (see, for example, U.S. Patent No. 5,589,466).

In one specific, non-limiting example, a pharmaceutical composition for intravenous administration would include about 0.1 µg to 10 mg of immunogenic Mtb polypeptide per patient per day. Dosages from 0.1 to about 100 mg per patient per day can be used, particularly if the agent is administered to a secluded site and not into the circulatory or lymph system, such as into a body cavity or into a lumen of an organ. Actual methods for preparing administrable compositions will be known or apparent to those skilled in the art and are described in more detail in such publications as Remington's Pharmaceutical Sciences, 19th Ed., Mack Publishing Company, Easton, Pennsylvania, 1995.

Single or multiple administrations of the compositions are administered, depending on the dosage and frequency as required and tolerated by the subject. In one embodiment, the dosage is administered once as a bolus, but in another embodiment can be applied periodically until a therapeutic result is achieved. In one embodiment, the dose is sufficient to treat or ameliorate symptoms or signs of tuberculosis without producing unacceptable toxicity to the subject. In another embodiment, the dose is sufficient to inhibit infection with Mtb upon subsequent exposure to Mtb. In a further embodiment, the dose is sufficient to inhibit a symptom of tuberculosis in a subject with a latent Mtb infection. Systemic or local administration can be utilized.

In another method, antigen presenting cells (APCs), such as dendritic cells (DCs), are isolated from a subject of interest and pulsed or co-incubated with peptides comprising an Mtb polypeptide *in vitro.* In one specific, non-limiting example, the APCs are autologous cells isolated from the subject of interest. A therapeutically effective amount of the antigen presenting cells is administered (reintroduced) to the subject of interest.

The Mtb polypeptide can be delivered to the DCs or to DC precursors via any method known in the art, including, but not limited to, pulsing DCs directly with antigen, or utilizing a broad variety of antigen delivery vehicles, such as, for example, liposomes, or other vectors known to deliver antigen to cells. In one specific, non-limiting example an antigenic formulation includes about 0.1 µg to about 1,000 µg, or about 1 to about 100 µg of a selected Mtb polypeptide. The Mtb polypeptide can also be administered with agents that promote DC maturation. Specific, non-limiting examples of agents of use are interleukin-4 (IL-4) and granulocyte/macrophage colony stimulating factor (GM-CSF), or flt-3 ligand (flt-3L). The preparation can also contain buffers, excipients, and preservatives, amongst other ingredients.

In one embodiment, mature APCs are generated to present the immunogenic Mtb polypeptide. These DCs are then administered alone to a subject infected with Mtb, or at risk for infection with Mtb. In another embodiment, the mature DCs are administered in conjunction with an antibacterial or antiviral agent.

Alternatively, the APCs are used to sensitize CD8 cells, such as peripheral blood lymphocytes (PBLs). The PBLs can be from the same subject (autologous) that is to be treated. Alternatively, the PBLs can be heterologous. However, they should at least be MHC Class-I restricted to the HLA types the subject possesses. An effective amount of the sensitized cells is then administered to the subject.

Peripheral blood mononuclear cells (PBMCs) can be used as the responder cell source of cytotoxic T lymphocyte (CTL) precursors. The appropriate antigen-presenting cells are incubated with peptide, after which the peptide-loaded antigen-presenting cells are then incubated with the responder cell population under optimized culture conditions. Positive CTL activation can be determined by assaying the culture for the presence of CTLs that kill radio-labeled target cells, both specific peptide-pulsed targets as well as target cells expressing endogenously processed forms of the antigen from which the peptide sequence was derived.

Alternatively, a CD8⁺ T cell clone that recognizes the Mtb polypeptide can be isolated from a subject of interest. This CD8⁺ T cell clone can be expanded *in vitro*, using methods known in the art. A therapeutically effective amount of the CD8⁺ T cells is then administered to the subject of interest.

Thus, cells can be administered to a subject to treat, inhibit, or even prevent an Mtb infection, such as to decrease a symptom of an Mtb infection. In these applications, a therapeutically effective amount of activated APCs, or activated lymphocytes, are administered to a subject in an amount sufficient to raise an immune response to Mtb.

In supplemental methods, any therapeutic regimen is augmented by administering a cytokine, such as interleukin (IL)-2, IL-3, IL-6, IL-10, IL-12, IL-15, GM-CSF, interferons. In further methods, an additional antibacterial or antiviral agent is administered to the subject.

The disclosure is illustrated by the following non-limiting Examples.

### EXAMPLES

### Example 1

### Selection of Antigens

A peptide library encompassing 39,499 Mtb peptides was screened for antigens and/or epitopes that were both strongly and commonly recognized in individuals with Mtb infection in Portland, Oregon. This peptide library represents 389 genes, representing roughly 10% of the Mtb genome. The peptides are 15mers overlapping by 11 amino acids for each gene product. 50 nmol of each peptide was synthesized individually and then pooled into 777 pools of 50 peptides in a 96 well format (nine plates). Five blank wells and one well of an irrelevant peptide pool, SIV gag, were included on each of the nine plates.

CD8⁺ T cells from donors were screened against the peptide library by IFN-γ ELISPOT. The IFN-γ ELISPOT assay was performed as described previously (Beckman et al., J. Immunol. 157:2795-2803, 1996). For determination of *ex vivo* frequencies of CD8⁺ T cells responding to Mtb infection or Mtb antigens, CD8⁺ T-cells were positively selected from peripheral blood mononuclear cells using magnetic beads (Miltenyi Biotec, Auburn CA) as a source of responder T cells and tested for their response to autologous DC. Each plate of the genomic peptide library was screened in duplicate, for a total of 18 ELISPOT plates per screen. CD8+ T cells were prepared from cryopreserved PBMC by CD8 selection using magnetic bead separations. Resulting cell populations contained >99% CD8+ T cells. CD8+ T cells (250,000 cells/ well), autologous DCs (20,000 cells/ well), and IL-2 (0.5ng/ml) were added to peptide (final 5ug/ml, individual peptides) in the ELISPOT plates. Five media control wells were included on each plate. Spots are enumerated using with the AID EliSpot Reader System. For each plate, the mean of these five wells was subtracted from each well of that plate to normalize between plates. Each technical replicate on each plate was then scored. A well was scored positive if the spot forming units (SFU), less the mean of the media wells, was greater than or equal to ten and the SFU was greater than or equal to twice the mean of the media. Twenty donors were tested, including fifteen LTBI (6 Caucasian, 4 African American, 5 SE Asian) and five donors with active TB.

Two criteria were used to select the peptide pools. First, peptide pools had to be in the top 5% of a donor's response. Second, the peptide pool had to be recognized by three or more donors. The peptide pools selected by this method were identical independent of the order these criteria were applied. A well was considered positive in the donor screen if only one technical replicate was statistically positive. However, since there is more confidence in a well where both technical replicates are positive, the selected wells were compared if the average spot forming units (SFU) for wells with two positive technical replicates was weighted by 200% to the selected wells if the average SFU was not weighted. 32 wells were selected if there was no weighting given to the technical replicates and 35 wells were selected if the weighting was applied. However, 19 wells were selected by both weighting and not weighting the average SFU and these were chosen for further analysis (Table 2).

**Table 2. Selected antigens and epitopes for clinical validation studies**

| **Antigen Number** | **Rv Numbers** | **Gene Names** |
|---|---|---|
| **1** | Rv3641c (33)¹ | fic |
| **2** | Rv3136 (46):Rv3135 (4) | PPE51 : PPE50 |
| **3** | Rv0383c (30):Rv0394c (20) | Rv0383c : Rv0394c |
| **4** | Rv1184c (20) | Rv1184c |
| **5** | Rv3514 (47):Rv3532 (3) | PE_PGRS57 : PPE61 |
| **6** | Rv3558 (44):Rv3539 (6) | PPE64: PPE63 |
| **7** | Rv1979c (50) | Rv1979c |
| **8** | Rv1980c (28):Rv1984c (22) | mpt64 : cfp21 |
| **9** | Rv3347c (50) | PPE55 |
| **10** | Rv0151c (50) | PE1 |
| **11** | Rv1997 (50) | ctpF |
| **12** | Rv1997 (50) | ctpF |
| **13** | Rv0159c (50) | PE3 |
| **14** | Rv1997 (50) | ctpF |
| **15** | Rv2711 (37):Rv1404 (13) | ideR : Rv1404 |
| **16** | Rv1706c (50) | PPE23 |
| **17** | Rv2041c (50) | Rv2041c |
| **18** | Rv2041c (43):Rv2093c (7) | Rv2041c : tatC |
| **19** | Rv1039c (50) | PPE15 |

| | | |
|---|---|---|
| ¹Number of peptides from each gene shown in parentheses | | |

### Example 2

### Screening of Selected Antigens

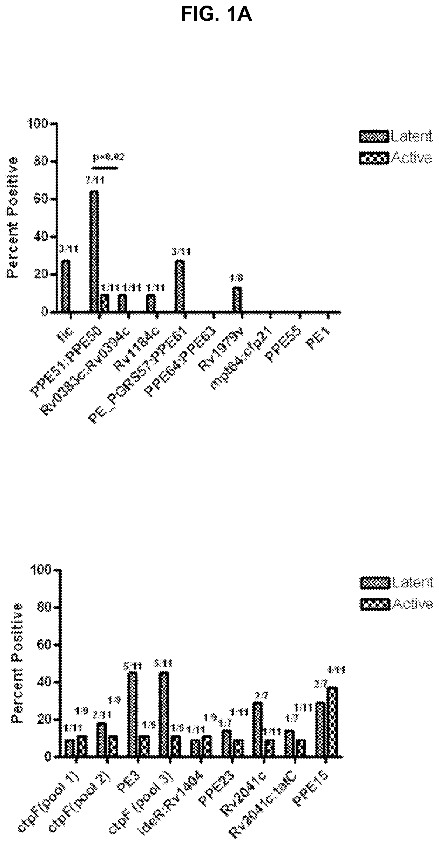

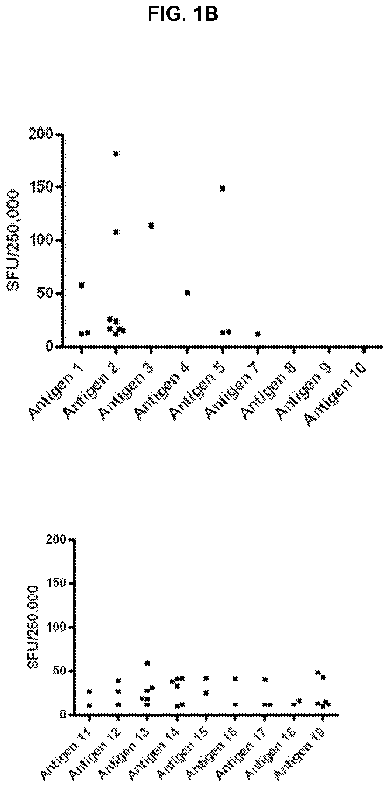

The antigens identified in Example 1 were screened in a CD8 ELISPOT assay against latent and active TB donors from Uganda. ELISPOT plates were read using the AID ELISPOT reader and output was exported into excel files. Data were imported into SAS® version 9.1 (SAS Institute, Inc., Cary, NC) and analyzed. A categorical variable for a positive ELISPOT was created in SAS®. For a positive response to the antigen, the mean of the antigen containing wells must be greater than the background wells by two standard deviations. If this was true, the background was subtracted and this difference must then be greater than 10 spots. Similarly, a continuous ELISPOT variable was created for each antigen detailing the spot forming units remaining if the antigen met the categorical criteria above. The results were graphed by proportion of positive responses stratified by active or latent TB along with the corresponding spot forming unit (FIG. 1A and B).

Five antigens were selected for the validation stage. Several factors were considered in the selection, including those antigens that had a suggestion of disease specificity, as well as antigens with a broad and strong response. These antigens included PPE50:51, PE3, CtpF, PPE15, and EsxJ. Fifty-six latent and 52 active TB individuals were studied in the validation phase. Twenty-one individuals (19.2%) responded to all five antigens at the predefined cut-off, whereas 10 individuals (9%) responded to four of the antigens. Forty individuals (36%) responded to up to three antigens and 35% did not respond to any of the five antigens selected. Although some disease specificity was noted in the screening stage, especially as it applied to PPE50:51, this was not apparent in the validation stage.

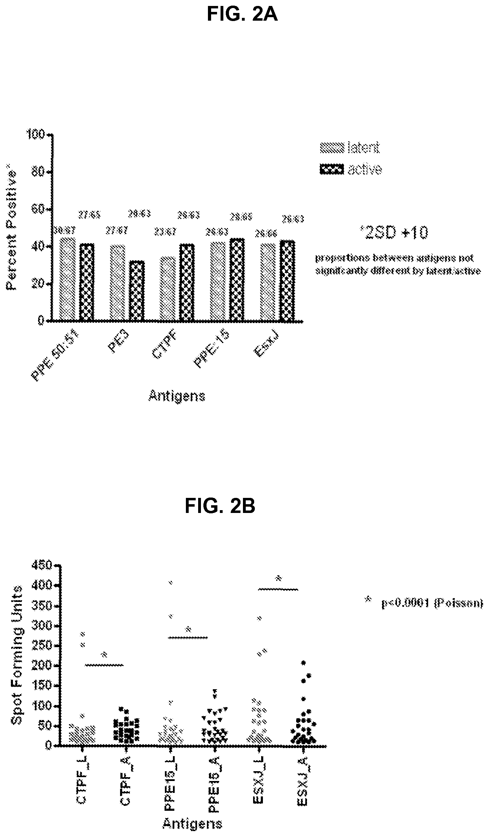

The magnitude of the response was studied as well. Using Poisson modeling, individuals with latent disease had a significantly greater spot count than those with active disease for 4 antigens (PPE50:51, cTPF, PPE15, EsXJ) however the difference was not clinically meaningful (FIG. 2).

### Example 3

### Additional Antigens

Additional antigens were selected using the methods described in Example 1. The additional antigens are provided in Table 3.

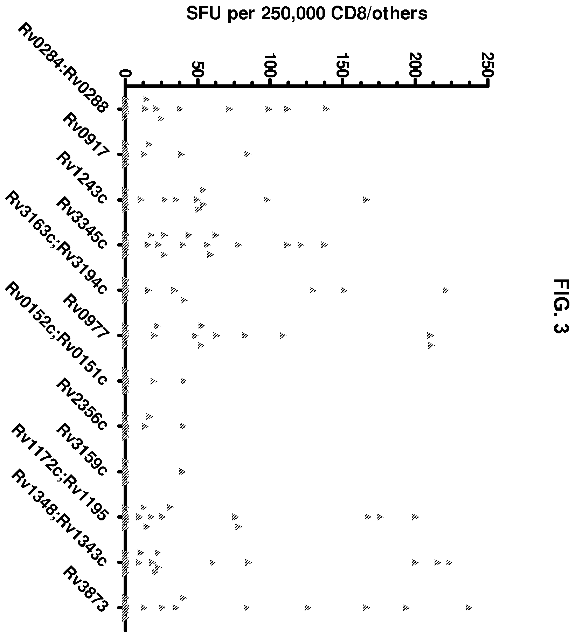

The additional identified antigens were screened in a CD8 ELISPOT assay (as described in Examples 1 and 2) against latent and active TB donors from Uganda. The results were graphed by the corresponding spot forming unit (FIG. 3).

**Table 3. Additional antigens and epitopes for clinical validation studies**

| **Rv_Numbers (# peptides in pool)** | **Gene**_**Names** |
|---|---|
| Rv0284(17) : Rv0288(11) | Rv0284 : esxH |
| Rv0917(31) | betP |
| Rv1243c(50) | PE_PGRS23 |
| Rv3345c(100) | PE_PGRS50 |
| Rv3163c(41) : Rv3194c(9) | Rv3163c : Rv3194c |
| Rv0977(50) | PE_PGRS16 |
| Rv0152c(40) : Rv0151c(10) | PE2 : PE1 |
| Rv1917c(50) | PPE34 |
| Rv2040c(37) : Rv2025c(13) | Rv2040c : Rv2025c |
| Rv2356c(50) | PPE40 |
| Rv3159c(50) | PPE53 |
| Rv1172c(32) : Rv1195(18) | PE12 : PE13 |
| Rv1348(35) : Rv1343c(15) | Rv1348 : lprD |
| Rv3873(50) | PPE68 |

### Example 4

### Identification of Peptide-Specific T Cell Clones

Peptide-specific T cell clones were isolated from individuals with LTBI or active TB, using peptide pulsed DCs as APCs and limiting dilution cloning methodology. Briefly, CD8+ T cells were isolated from PBMCs using positive selection using CD8 antibody-coated magnetic beads per the manufacturer's instructions (Miltenyi Biotec, Bergisch Gladbach, Germany). T cells were seeded at various concentrations in the presence of a 2 x 10⁴-irradiated autologous peptide pulsed DC, 1 x 10⁵ irradiated autologous PBMC, and rIL-2 (5 ng/ml) in cell culture media consisting of 200 µl of RPMI 1640 supplemented with 10% human sera. Wells exhibiting growth between 10-14 days were assessed for peptide specificity using ELISPOT and peptide pulsed DCs as a source of APCs. T cells retaining peptide specificity were further phenotyped for αβ T cell receptor expression and CD8 expression by FACS.

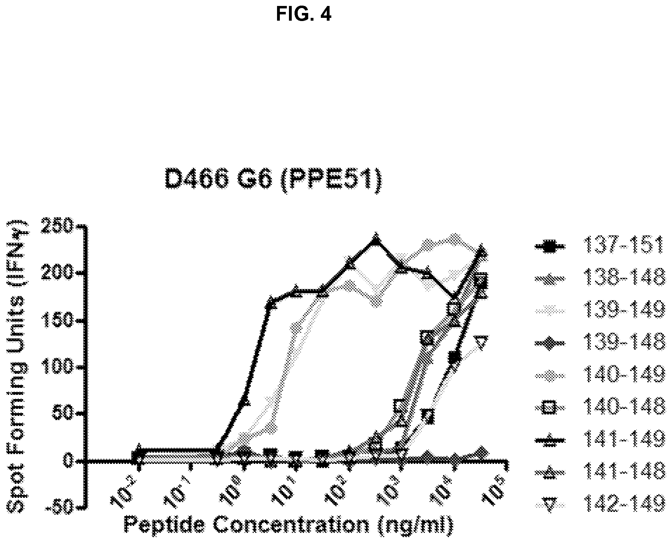

Using the 15mer Rv3136₁₃₇₋₁₅₁, T cell clones were generated to the peptide using the methods described. Having derived an antigen-specific CD8⁺ T cell clone, the minimal epitope was determined. The minimal epitope was defined as the epitope which allowed for T cell recognition at the lowest concentration of peptide. Each 9-mer, 10-mer, and 11-mer peptide within the 15-mer was tested over a broad range of peptide concentrations, and by definition, the peptide eliciting a response at the lowest peptide concentration is the minimal epitope. Peptides including amino acids 139-149 of Rv3136 (SEQ ID NO: 2) allowed for T cell recognition at the lowest concentrations (FIG. 4), with amino acids 141-49 eliciting a response at the lowest concentration of all tested peptides.

### Example 5

### Animal models

In tuberculosis research, mouse and guinea pig models have been used extensively to model various aspects of the disease.

### A. Mouse Model:

Mice can be infected by a variety of routes, including intravenous, intraperitoneal and tracheal. One route is aerosolization of the infectious organism for respiratory infection. The mice are exposed to the aerosol in a chamber (wither whole body or nose only infection). The dose of invention can be varied by manipulating the concentration of Mtb in the nebulizer or time of exposure. A low dose infection, such as about 50 colony forming units (CFU) via aerosol, results in a slow and steady increase in bacterial numbers in the lungs, generally reaching a peak in four weeks, which coincides with the peak number of T cells in the lungs. The initial period is considered the acute stage of infection. Following infection, there is a dissemination of bacteria to the mediastinal lymph nodes. T cell priming is generally detectable between two and three weeks. After about four weeks the bacterial numbers stabilize, and there is a slow progressive pathologic response. This system is of use for modeling active infection. Thus, the above-described polypeptides, or polynucleotides encoding these polypeptides, can be administered prior to infection. The ability of the Mtb polypeptides (or polynucleotides encoding these polypeptides) to inhibit or prevent infection is then assessed. Alternatively, the mice are administered Mtb, and the ability of the Mtb polypeptide (or polynucleotide encoding these polypeptides) to treat the Mtb infection is monitored. The effectiveness of the Mtb polypeptides (or polynucleotides) can be monitored by measuring the T cell response, such as the number of CD8⁺ or CD4⁺ T cells, and/or measuring the bacterial numbers, and/or evaluating the pathology.

Exemplary protocols are provided below (see also Repique et al., Infec. Immun. 70: 3318-3323, 2002, for an additional protocol).

### 1. Short Term Mouse Model:

C57BL/6 mice are vaccinated with a composition including one or more Mtb polypeptide, or a polynucleotide encoding these one or more polypeptides according to the appropriate protocol and then rested for 4 to 6 weeks. Immunized mice are infected with a low dose aerosol (50-100 CFU) of virulent *M. tuberculosis* and protection is evaluated by assessing the number of viable bacilli 30 days post challenge.

Viable counts are performed on the lung and spleen of mice by homogenizing the organs and plating serial 10-fold dilutions on 7H11 agar plates. Plates are incubated for up to 21 days and the number of colony forming units per organ determined.

BCG vaccinated mice have approximately 1Log₁₀ protection in their lung and spleen when compared to PBS-treated mice.

### B. Guinea Pig Models:

### 1. Short Term Guinea Pig Model

Out-bred Hartley guinea pigs are vaccinated with a composition including one or more Mtb polypeptide, or a polynucleotide encoding these one or more polypeptides, and then rested for 8 to 10 weeks. Immunized guinea pigs are infected with a low dose aerosol (10-30 CFU) of virulent *M. tuberculosis* and protection is evaluated by assessing the number of viable bacilli 30 days post challenge.

Viable counts are performed on the lung and spleen of guinea pigs by homogenizing the organs and plating serial 10-fold dilutions on 7H11 agar plates. Plates are incubated for up to 21 days and the number of colony forming units per organ determined. Lung and spleen segments are also taken for histological analyses.

BCG vaccinated guinea pigs have approximately 2-3Log₁₀ protection in their lung and spleen when compared to PBS-treated guinea pigs. In addition, BCG vaccinated guinea pigs have well defined granulomas when compared to unvaccinated animals.

### 2. Long Term Guinea Pig Model

The guinea pig model is similar to the mouse model, but the experiments are open-ended survival type and can last for as long as 2 years. Guinea pigs develop "classical" granulomas similar to humans with active TB, and as lung tissue necrosis progresses, they begin to lose weight and die of TB similar to humans. The number of colony forming units in the lungs and spleen can be assessed. Histological examination can also be performed to determine the degree of lung involvement and tissue destruction. After low-dose aerosol exposure in the guinea pig the number of organisms increases progressively during the first three weeks and then plateaus into a chronic state. During the later stages of infection there is increased bacterial load in the lung and this is associated with a worsening pathological condition. Without treatment, there is a concomitant rise in both CD4 and CD8 T cells in the lungs of infected guinea pigs.

Out-bred Hartley guinea pigs are vaccinated with the experimental vaccine (such as a composition including one or more Mtb polypeptide, or a polynucleotide encoding these one or more polypeptides) according to the appropriate protocol and then rested for 8 to 10 weeks. Immunized guinea pigs are then infected with a low dose aerosol (10-30 CFU) of virulent *M. tuberculosis.* Guinea pigs are weighed weekly and monitored daily for signs of disease (such as increased respiration and failure to thrive). Unvaccinated guinea pigs succumb to infection from 20 to 25 weeks post challenge, while BCG vaccinated guinea pigs survive for 50 to 55 weeks post challenge.

At necropsy, the lung and spleen are assessed for the number of CFU and the extent of pathology. The relative protection of the experimental composition is compared to BCG vaccinated animals.

### Example 6

### Methods of Treating or Inhibiting Tuberculosis in a Subject

This example describes methods that can be used to induce an immune response in a subject that has or is at risk of having tuberculosis. In particular examples, the method includes selecting a subject having, thought to have, or at risk of having tuberculosis. Subjects having or thought to have tuberculosis include those with symptoms such as persistent cough, blood-tinged sputum, fever, weight loss, Ghon complex, or a positive diagnostic test (such as a tuberculin skin test). Subjects at risk of tuberculosis include those with exposure to an infected individual, those in an area where tuberculosis is endemic, and immunocompromised individuals.

Subjects selected for treatment can be administered a therapeutic amount of a disclosed immunogenic Mtb polypeptide or immunogenic fragment thereof or a polynucleotide encoding the polypeptide or fragment thereof. In some examples, a Mtb polypeptide or immunogenic fragment thereof or a polynucleotide encoding the polypeptide or fragment thereof is administered to the subject at doses of about 0.1 µg to 10 mg of immunogenic Mtb polypeptide or polynucleotide encoding the polypeptide. Dosages from 0.1 mg to about 100 mg per subject can be used, particularly if the agent is administered to a secluded site and not into the circulatory or lymph system, such as into a body cavity or into a lumen of an organ. However, the particular dose can be determined by a skilled clinician. The disclosed Mtb polypeptide (or immunogenic fragment thereof) or polynucleotide encoding the polypeptide or fragment thereof can be administered in one or several doses, for example continuously, daily, weekly, or monthly. When administered sequentially, the time separating the administration of the disclosed Mtb polypeptide (or immunogenic fragment thereof) or a polynucleotide encoding the polypeptide or fragment thereof can be seconds, minutes, hours, days, or even weeks.

The mode of administration can be any used in the art. The amount of agent administered to the subject can be determined by a clinician, and may depend on the particular subject treated. Specific exemplary amounts are provided herein (but the disclosure is not limited to such doses).

## Claims

1. A composition comprising a therapeutically effective amount of a polypeptide, or a polynucleotide encoding the polypeptide, for use in producing an immune response to *Mycobacterium tuberculosis.* (Mtb) in a subject or for use in treating a subject infected with *Mycobacterium tuberculosis* or for use in inhibiting an infection with *Mycobacterium tuberculosis* in a subject, wherein the polypeptide consists of:
(a) the amino acid sequence set forth as SEQ ID NO: 6; or
(b) at least nine to twenty consecutive amino acids of the amino acid sequence set forth as SEQ ID NO: 6, wherein the nine to twenty consecutive amino acids specifically bind major histocompatibility complex (MHC) class I.

2. A composition for use according to claim 1, wherein the use comprises administering to the subject a therapeutically effective amount of the polypeptide.

3. A composition for use according to any one of claims 1 or 2, wherein the use further comprises administering a therapeutically effective amount of an adjuvant.

4. A composition for use according to any one of claims 1 to 3, wherein the immune response is a protective immune response.

5. A composition for use according to any of claims 1 to 4, wherein the subject is at risk of infection with Mtb.

6. A composition for use according to any of claims 1 to 4, wherein the subject is infected with Mtb, optionally wherein the subject has a latent infection with Mtb.

7. An isolated polypeptide consisting of nine to twenty consecutive amino acids of the amino acid sequence set forth as SEQ ID NO 6, wherein the nine to twenty consecutive amino acids specifically bind major histocompatibility complex (MHC) class I, wherein the isolated polypeptide does not comprise any of the amino acid sequences set forth as SEQ ID NOs: 1-16.

8. An isolated polynucleotide encoding the polypeptide of claim 7, optionally wherein the polynucleotide is operably linked to a promoter.

9. A vector comprising the polynucleotide of claim 8, optionally wherein the vector is a plasmid vector, optionally wherein the plasmid vector is expressed in yeast, or optionally wherein the vector is a viral vector, optionally wherein the viral vector is a retrovirus, orthopox, avipox, fowlpox, capripox, suipox, adenovirus, herpes virus, alpha virus, baculovirus, Sindbis virus, vaccinia virus, or poliovirus vector.

10. A vector comprising the polynucleotide of claim 8, wherein the vector is a poxvirus vector.

11. An isolated host cell comprising the vector of claims 9 or 10, optionally wherein the host cell is a mycobacterium.

12. A pharmaceutical composition comprising:
the polypeptide of claim 7 and a pharmaceutically acceptable carrier;
a therapeutically effective amount of the host cell of claim 11 in a pharmaceutically acceptable carrier; or
a therapeutically effective amount of the polynucleotide of claim 8 in a pharmaceutically acceptable carrier, optionally wherein the polynucleotide is carried by a recombinant viral vector, particularly a recombinant pox virus vector.

13. A composition according to claim 1 wherein the polypeptide consists of the amino acid sequence set forth as SEQ ID NO: 6.

## Patentansprüche

1. Zusammensetzung umfassend eine therapeutisch wirksame Menge eines Polypeptids oder eines Polynukleotids, das für das Polypeptid codiert, zur Verwendung beim Hervorrufen einer Immunantwort auf *Mycobacterium tuberculosis* (Mtb) in einem Subjekt oder zur Verwendung beim Behandeln eines mit *Mycobacterium tuberculosis* infizierten Subjekts oder zur Verwendung beim Inhibieren einer Infektion mit *Mycobacterium tuberculosis* in einem Subjekt, worin das Polypeptid aus Folgendem besteht:
(a) der als SEQ ID Nr. 6 dargelegten Aminosäuresequenz; oder
(b) mindestens neun bis zwanzig konsekutiven Aminosäuren der als SEQ ID Nr. 6 dargelegten Aminosäuresequenz, worin die neun bis zwanzig konsekutiven Aminosäuren den Haupthistokompatibilitätskomplex (MHC) der Klasse I spezifisch binden.

2. Zusammensetzung zur Verwendung nach Anspruch 1, worin die Verwendung das Verabreichen, an das Subjekt, einer therapeutisch wirksamen Menge des Polypeptids umfasst.

3. Zusammensetzung zur Verwendung nach einem der Ansprüche 1 oder 2, worin die Verwendung ferner das Verabreichen einer therapeutisch wirksamen Menge eines Adjuvans umfasst.

4. Zusammensetzung zur Verwendung nach einem der Ansprüche 1 bis 3, worin die Immunantwort eine schützende Immunantwort ist.

5. Zusammensetzung zur Verwendung nach einem der Ansprüche 1 bis 4, worin bei dem Subjekt die Gefahr einer Infektion mit Mtb besteht.

6. Zusammensetzung zur Verwendung nach einem der Ansprüche 1 bis 4, worin das Subjekt mit Mtb infiziert ist, wahlweise, worin das Subjekt eine latente Infektion mit Mtb hat.

7. Isoliertes Polypeptid bestehend aus neun bis zwanzig konsekutiven Aminosäuren der als SEQ ID Nr. 6 dargelegten Aminosäuresequenz, worin die neun bis zwanzig konsekutiven Aminosäuren den Haupthistokompatibilitätskomplex (MHC) 2-P der Klasse I spezifisch binden.

8. Isoliertes Polynukleotid, das für das Polypeptid nach Anspruch 7 codiert, wahlweise, worin das Polynukleotid operabel an einen Promoter gebunden ist.

9. Vektor umfassend das Polynukleotid nach Anspruch 8, wahlweise, worin der Vektor ein Plasmid-Vektor ist, wahlweise, worin der Plasmid-Vektor in Hefe exprimiert ist, oder wahlweise, worin der Vektor ein viraler Vektor ist, wahlweise, worin der virale Vektor ein Retrovirus-, Orthopox-, Avipox-, Fowlpox-, Capripox-, Suipox-, Adenovirus-, Herpesvirus-, Alphavirus-, Baculovirus-, Sindbisvirus-, Vacciniavirus- oder Poliovirus-Vektor ist.

10. Vektor umfassend das Polynukleotid nach Anspruch 8, worin der Vektor ein Poxvirus-Vektor ist.

11. Isolierte Wirtszelle umfassend den Vektor nach Anspruch 9 oder 10, wahlweise, worin die Wirtszelle ein Mycobacterium ist.

12. Pharmazeutische Zusammensetzung umfassend:
das Polypeptid nach Anspruch 7 und einen pharmazeutisch verträglichen Träger; eine therapeutisch wirksame Menge der Wirtszelle nach Anspruch 11 in einem pharmazeutisch verträglichen Träger; oder
eine therapeutisch wirksame Menge des Polynukleotids nach Anspruch 8 in einem pharmazeutisch verträglichen Träger, wahlweise, worin das Polynukleotid durch einen rekombinanten viralen Vektor, insbesondere einen rekombinanten Poxvirus-Vektor, getragen wird.

13. Zusammensetzung zur Verwendung nach Anspruch 1, worin das Polypeptid aus der als SEQ ID Nr. 6 dargelegten Aminosäuresequenz besteht.

## Revendications

1. Une composition comprenant une quantité thérapeutiquement efficace d'un polypeptide ou d'un polynucléotide codant le polypeptide, pour une utilisation dans la production d'une réponse immunitaire au *Mycobacterium tuberculosis* (Mtb) chez un sujet ou pour une utilisation dans le traitement d'un sujet infecté par *Mycobacterium tuberculosis* ou pour une utilisation dans l'inhibition d'une infection par *Mycobacterium tuberculosis* chez un sujet, dans laquelle le polypeptide est constitué par :
(a) la séquence d'acides aminés indiquée dans SEQ ID NO: 6 ; ou
(b) au moins neuf à vingt acides aminés consécutifs de la séquence d'acides aminés indiquée dans SEQ ID NO: 6, dans laquelle les neuf à vingt acides aminés consécutifs se lient spécifiquement au complexe majeur d'histocompatibilité (MHC) de classe I.

2. Une composition pour une utilisation selon la revendication 1, dans laquelle l'utilisation consiste à administrer au sujet une quantité thérapeutiquement efficace du polypeptide.

3. Une composition pour une utilisation selon l'une quelconque des revendications 1 ou 2, dans laquelle l'utilisation consiste en outre à administrer une quantité thérapeutiquement efficace d'un adjuvant.

4. Une composition pour une utilisation selon l'une quelconque des revendications 1 à 3, dans laquelle la réponse immunitaire est une réponse immunitaire protectrice.

5. Une composition pour une utilisation selon l'une quelconque des revendications 1 à 4, dans laquelle le sujet risque d'être infecté par Mtb.

6. Une composition pour une utilisation selon l'une quelconque des revendications 1 à 4, dans laquelle le sujet est infecté par Mtb, en option dans laquelle le sujet a une infection latente par Mtb.

7. Un polypeptide isolé constitué par neuf à vingt acides aminés consécutifs de la séquence d'acides aminés indiquée dans SEQ ID NO 6, dans laquelle les neuf à vingt acides aminés consécutifs se lient spécifiquement au complexe majeur d'histocompatibilité (MHC) 2-P de classe I.

8. Un polynucléotide isolé codant le polypeptide selon la revendication 7, en option dans laquelle le polynucléotide est lié de manière fonctionnelle à un promoteur.

9. Un vecteur comprenant le polynucléotide selon la revendication 8, en option dans laquelle le vecteur est un vecteur plasmide, en option dans laquelle le vecteur plasmide est exprimé dans la levure, ou en option dans laquelle le vecteur est un vecteur viral, en option dans laquelle le vecteur viral est un rétrovirus, un orthopox, un avipox, un fowlpox, un capripox, un suipox, un adénovirus, un herpèsvirus, un alphavirus, un baculovirus, un virus Sindbis, un virus de la vaccine ou un vecteur poliovirus.

10. Un vecteur comprenant le polynucléotide selon la revendication 8, dans laquelle le vecteur est un vecteur poxvirus.

11. Une cellule hôte isolée comprenant le vecteur selon la revendication 9 ou 10, en option dans laquelle la cellule hôte est un mycobacterium.

12. Une composition pharmaceutique comprenant :
le polypeptide selon la revendication 7 et un excipient pharmaceutiquement acceptable ;
une quantité thérapeutiquement efficace de la cellule hôte selon la revendication 11 dans un excipient pharmaceutiquement acceptable ; ou
une quantité thérapeutiquement efficace du polynucléotide selon la revendication 8 dans un excipient pharmaceutiquement acceptable, en option dans laquelle le polynucléotide est porté par un vecteur viral recombinant, particulièrement un vecteur viral pox recombinant.

13. Une composition pour une utilisation selon la revendication 1 dans laquelle le polypeptide est constitué par la séquence d'acides aminés indiquée dans SEQ ID NO: 6.
